# EUROPEAN PATENT APPLICATION

(11) **EP 2 322 222 A1**
(43) Date of publication of application: **18.05.2011**
(21) Application number: 09805035.4
(22) Date of filing: 06.08.2009
(51) Int. Cl.: A61K 45/00, A61K 31/498, A61K 31/553, A61P 1/00, A61P 1/12, C07D 401/12, C07D 498/04

(54) **THERAPEUTIC AGENT FOR IRRITABLE BOWEL SYNDROME**

(30) Priority: 07.08.2008 JP 2008204843
(71) Applicant: Takeda Pharmaceutical Company Limited, Osaka-shi, Osaka 541-0045 (JP)
(72) Inventor: MARUYAMA, Minoru, Osaka-shi Osaka 532-8686 (JP)
(74) Representative: Held, Stephan
(86) International application number: PCT/JP2009/063960
(87) International publication number: WO 2010/016552

(57) **Abstract**

[Problem]The present invention aims to provide an agent for the prophylaxis or treatment of irritable bowel syndrome.

[Solving Means]An agent for the prophylaxis or treatment of irritable bowel syndrome, containing a compound having a TGR5 receptor agonist activity, a fused ring compound represented by the formula 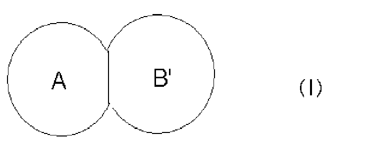 wherein ring A is an optionally substituted aromatic ring, and ring B' is a 5- to 9-membered ring having one or more substituents, or a salt thereof as an active ingredient.

## Description

### Technical Field

The present invention relates to an agent for the prophylaxis or treatment of irritable bowel syndrome. More particularly, the present invention relates to an agent for the prophylaxis or treatment of irritable bowel syndrome, which comprises a fused ring compound.

### (Background of the Invention)

Irritable bowel syndrome (IBS) is a disease group showing symptoms such as abdominal pain, diarrhea, constipation and the like due to functional hypersensitivity of the intestine, even though physical disease such as cancer, inflammatory disease and the like is not present. The causes thereof are considered to include irregular hours, mental strain and anxiety, stress and the like, of which stress is considered to be the most common cause of IBS. The disease type is divided into 1) diarrhea type, 2) constipation type, 3) alternating diarrhea and constipation type, and 4) gas symptom dominant type. For the treatment of IBS, improvement of lyfestyle, diet therapy, drug therapy, psychological therapy and the like are used. For symptomatic therapy, an agent for regulating gastrointestinal tract function (antiflatulent, anti-constipation agent, antidiarrheal agent etc.) may be used or an antianxiety drug or antidepressant may be formulated when stress is the cause.

Patent document 1 reports the following fused ring compound. A compound represented by

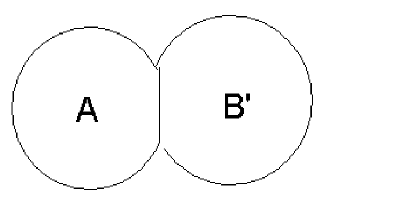

wherein ring A is an optionally substituted aromatic ring, and ring B' is a 5- to 9-membered ring having one or more substituents, or a salt thereof.
While the document discloses target diseases such as gastrointestinal diseases such as ulcerative colitis, gastrointestinal tract ulcer, enteritis, Crohn's disease and the like, it does not describe that the compound is useful for IBS.
In addition, patent document 1 reports that the aforementioned fused ring compound has a TGR5 agonistic activity.
TGR5 is a G-protein-coupled receptor protein, and an agonist or antagonist thereof is reported to be useful for the treatment of central nervous diseases, inflammatory diseases and the like (see patent documents 3 and 5). However, the relationship between TGR5 and IBS is not described.
Patent document 4 discloses human BG37 (TGR5), and a screening method of agonist/antagonist using the same. The document recites, as examples of target diseases of agonist/antagonist, gastric ulcer, bowel disease (inflammatory bowel disease etc.), ischemic cardiac disease, obesity, pain, allergic disease and autoimmune disease. However, the relationship with IBS is not described.
Patent document 5 discloses a screening method of a TGR5 agonist/antagonist using TGR5 and a substance relating to cholesterol metabolism. As a ligand, it discloses a cholesterol metabolism-related substance, an analogue thereof, and a substance activating TGR5. However, it does not describe the concept of low molecular synthetic ligand. While an agonist/antagonist is described to be useful as an agent for the prophylaxis or treatment of the central nervous system diseases (e.g., eating disorder and the like), inflammatory diseases (e.g., allergy and the like), immune diseases (Crohn's disease and the like), digestive tract diseases (ulcer, enteritis and the like) and the like, the relationship with IBS is not known.

Patent document 2 reports the following compound. A compound represented by

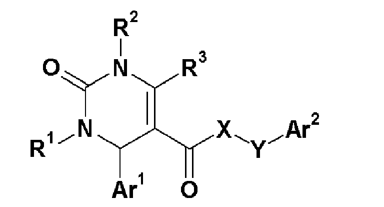

wherein R¹, R² and R³ are each a hydrogen atom or an optionally halogenated C₁₋₆ alkyl group; X is a bond, -O-, -NR- (R is a hydrogen atom or a lower alkyl group) or -S-; Y is an optionally substituted C₁₋₅ alkylene group; Ar¹ and Ar² are each an optionally substituted monocyclic aromatic group, or a salt thereof.
The document discloses that the compound has a TGR5 receptor agonistic action and recites gastric ulcer, ulcerative colitis, Crohn's disease, gastrointestinal tract ulcer, enteritis and the like as target diseases. However, it does not describe that the compound is useful for IBS.

Patent document 9 reports the following compound. A compound represented by

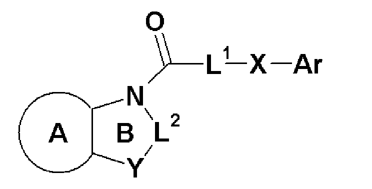

wherein ring A is an optionally substituted 5- or 6-membered aromatic ring,
ring B is a 6- to 8-membered nonaromatic nitrogen-containing heterocycle,
X and Y are independently a bond, -O-, -NR- (R is a hydrogen atom or an optionally substituted C₁₋₆ alkyl group) or -S(O)n- (n is an integer of 0 to 2),
L¹ is an optionally substituted chained C₁₋₅ alkylene group or an optionally substituted chained C₂₋₅ alkenylene group,
L² is an optionally substituted chained C₂₋₅ alkylene group or an optionally substituted chained C₂₋₅ alkenylene group, and
Ar is an optionally substituted cyclic group, or a salt thereof or a prodrug thereof.
The document discloses that the compound has a TGR5 receptor agonistic action, and gastric ulcer, ulcerative colitis, Crohn's disease, gastrointestinal tract ulcer, enteritis and the like are target diseases. However, the document does not describe that the compound is useful for IBS.

Patent document 10 reports the following compound. A compound represented by

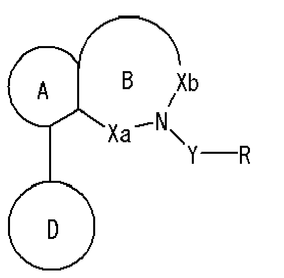

wherein ring A is an optionally further substituted aromatic heterocycle;
ring B is an optionally further substituted nitrogen-containing 6-to 9-membered ring;
Xa is an optionally substituted methylene group (excluding -C(=O)-);
Xb is an optionally substituted methylene group;
Y is an optionally substituted C₁₋₃ alkylene group, -CONH-, -SO₂NH- or -SO₂-;
R is an optionally substituted aromatic group; and
ring D is an optionally substituted aromatic ring or an optionally substituted nonaromatic heterocycle,
provided that when ring D is an optionally substituted benzene ring, the benzene ring has a substituent at the ortho-position relative to the bond with ring A, or Xb is a substituted methylene group, or a salt thereof.
The document discloses that the compound has a TGR5 receptor agonistic action, and the target diseases are gastric ulcer, ulcerative colitis, Crohn's disease, gastrointestinal tract ulcer, enteritis and the like. However, the document does not describe that the compound is useful for IBS.

On the other hand, patent document 6 reports the following benzoxazepine compounds. Compounds represented by

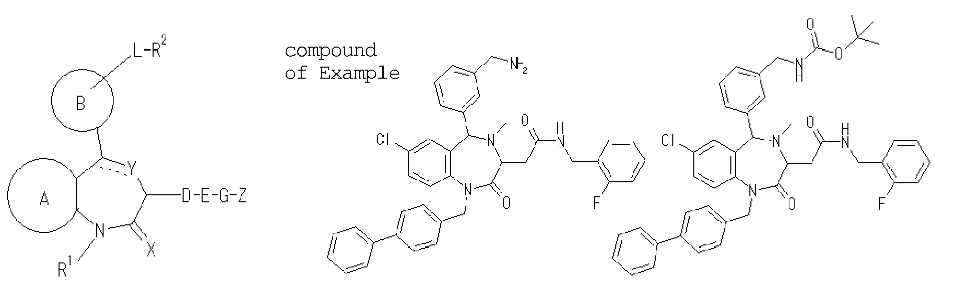

wherein ring A and ring B are each aromatic hydrocarbon optionally having substituent(s) or aromatic heterocycle optionally having substituent(s); Z is a cyclic group optionally having substituent(s) or a chain hydrocarbon group optionally having substituent(s); R¹ is a hydrogen atom, a hydrocarbon group optionally having substituent(s) or a heterocyclic group optionally having substituent(s); R² is an amino group optionally having substituent(s); D and G are each a bond or a divalent hydrocarbon group optionally having substituent(s); E is a bond, CON(R^{a}) (R^{a} is a hydrogen atom or a hydrocarbon group optionally having substituent(s)) and the like; L is a divalent group; ring B may be bonded to R² to form nonaromatic condensed nitrogen-containing heterocycle optionally having substituent(s); X is two hydrogen atoms, or an oxygen atom or a sulfur atom;

-̅ -̅ -̅ -̅ -̅

is a single bond or a double bond; and Y is a nitrogen atom when

-̅ -̅ -̅ -̅ -̅

is a double bond, and Y is an oxygen atom, -NR⁴- (R⁴ is a hydrogen atom, a hydrocarbon group optionally having substituent(s) or an acyl group) or S(O)n (n is 0, 1 or 2) when

-̅ -̅ -̅ -̅ -̅

is a single bond, or a salt thereof.
The document describes that the compound has a somatostatin receptor agonistic action, and is useful for the treatment of diabetes, Alzheimer's disease, inveterate diarrhea and the like. In addition, the specification describes irritable bowel syndrome as a target disease.

Furthermore, patent document 7 reports the following tricyclic tachykinin compound as a pyridooxazepine compound. A compound represented by

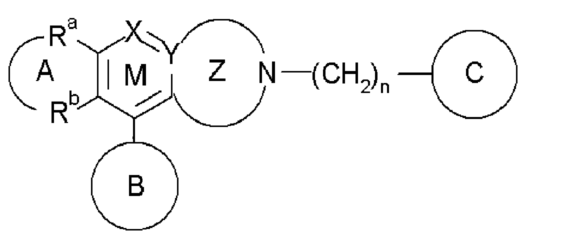

wherein ring M is heterocycle having -N=C<,-CO-N< or-CS-N< as a partial structure of -X=Y<; R^{a} and R^{b} are bonded to each other to form ring A, or the same or different and each is a hydrogen atom or a substituent of ring M; ring A and ring B are each a homo- or heterocycle optionally having substituent(s), at least one of which is a heterocycle optionally having substituent(s); ring C is a homo- or heterocycle optionally having substituent(s); ring Z is an optionally substituted nitrogen-containing heterocycle; and n is an integer of 1 to 6, or a salt thereof.
Furthermore, the specification of patent document 7 discloses the following compounds (Example compounds).

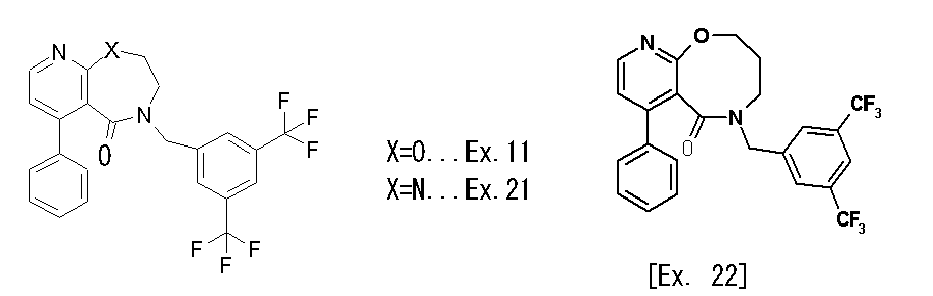

The document describes that the compound has a tachykinin antagonistic action, and is useful for inflammation or allergic diseases (e.g., asthma, rheumatoid arthritis, osteoarthritis), central nervous system diseases (schizophrenia, psychosomatic disorder etc.), digestive tract diseases (e.g., irritable bowel syndrome, ulcerative colitis), circulatory diseases (e.g., angina pectoris, cardiac failure), immune abnormality and the like.
Furthermore, patent document 8 describes that the tricyclic tachykinin compound disclosed in patent document 7 is useful for many diseases, symptoms and pathologies such as irritable bowel syndrome, depression, anxiety, diabetic neuropathy, contradictory immune reactions such as rejection of transplanted tissue and the like.

### [Document List]

### [Patent Documents]

patent document 1: WO2004/067008
patent document 2: WO2004/043468
patent document 3: WO01/77325
patent document 4: WO02/40669
patent document 5: WO02/084286
patent document 6: WO98/47882
patent document 7: EP-A-733632
patent document 8: WO99/47132
patent document 9: JP-A-2006-63064
patent document 10: JP-A-2006-56881

### Disclosure of the Invention

### Problems to be Solved by the Invention

The present invention aims to provide a novel agent for the prophylaxis or treatment of irritable bowel syndrome.

### Means of Solving the Problems

In view of the aforementioned problems, the present inventors have conducted intensive studies and found that a compound having a particular structure having a TGR5 receptor agonistic activity or a particular fused ring compound suppresses stress-induced bowel movement, and can improve abdominal symptoms of irritable bowel syndrome (IBS) in which stress is considered to be deeply involved in the pathology, particularly abdominal symptoms of diarrhea type irritable bowel syndrome (IBS), which resulted in the completion of the present invention.

Accordingly, the present invention provides the following.
[1] An agent for the prophylaxis or treatment of irritable bowel syndrome, comprising a compound having a TGR5 receptor agonistic activity.
[2] The agent of the above-mentioned [1], wherein the compound having a TGR5 receptor agonistic activity is a fused ring compound represented by the formula

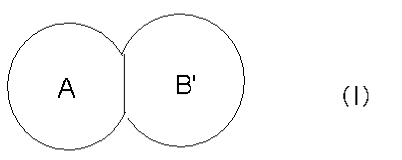

wherein ring A is an optionally substituted aromatic ring, and ring B' is a 5- to 9-membered ring having one or more substituents, or a salt thereof.
[3] An agent for the prophylaxis or treatment of irritable bowel syndrome, comprising a fused ring compound represented by the formula

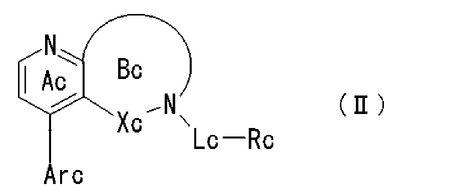

wherein ring Ac is a pyridine ring optionally further having substituent(s) other than -Arc,
ring Bc is a nitrogen-containing 6- to 9-membered ring optionally further having substituent(s) other than -Lc-Rc,
Xc is an optionally substituted methylene group,
Arc is an optionally substituted aromatic group,
Rc is an optionally substituted cyclic group,
Lc is an optionally substituted C₁₋₃ alkylene group, -CONH-, -SO₂NH- or-SO₂-, and
Xc group is not a methylene group substituted by an oxo group,
or a salt thereof.
[4] The agent of the above-mentioned [3], wherein the fused ring compound is represented by the formula

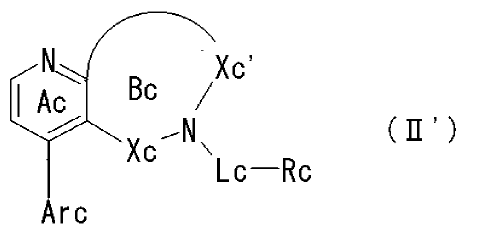

wherein Xc' is an optionally substituted methylene group, and other symbols are each as defined in the above-mentioned [3].
[5] The agent of the above-mentioned [3], wherein the fused ring compound is represented by the formula

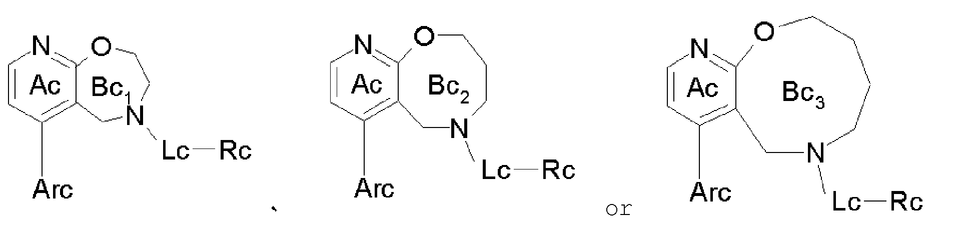

wherein ring Bc₁, ring Bc₂ and ring Bc₃ each optionally further have substituent(s) other than -Lc-Rc, and other symbols are each as defined in the above-mentioned [3].
[6] The agent of the above-mentioned [3] or [4], wherein Xc is a methylene group.
[7] The agent of any one of the above-mentioned [3] to [5], wherein the cyclic group for Rc is a phenyl group.
[8] The agent of any one of the above-mentioned [3] to [5], wherein Rc is a 3,5-bis(trifluoromethyl)phenyl group.
[9] The agent of any one of the above-mentioned [3] to [5], wherein Lc is a C₁₋₃ alkylene group or -SO₂-, which is optionally substituted by a C₁₋₃ alkyl group or an oxo group.
[10] The agent of any one of the above-mentioned [3] to [5], wherein Arc is an optionally substituted phenyl group.

[11] The agent of any one of the above-mentioned [3] to [5], wherein Arc is a phenyl group optionally having substituent(s) at the ortho-position relative to the bond with ring Ac.
[12] The agent of the above-mentioned [3], wherein the fused ring compound is represented by the formula

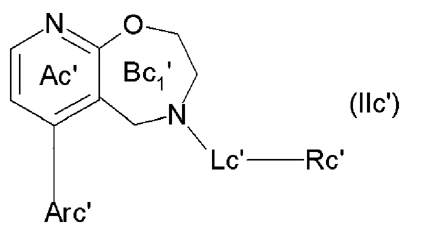

wherein ring Ac' is a pyridine ring optionally further having substituent(s) other than -Arc',
ring Bc₁' optionally further has substituent(s) other than -Lc'-Rc', Lc' is a C₁₋₃ alkylene group optionally substituted by a C₁₋₃ alkyl group or an oxo group,
Rc' is an optionally substituted phenyl group, and
Arc' is a phenyl group optionally having substituent(s) at the ortho-position relative to the bond with ring Ac'.
[13] An agent for the prophylaxis or treatment of irritable bowel syndrome, comprising a fused ring compound represented by the formula

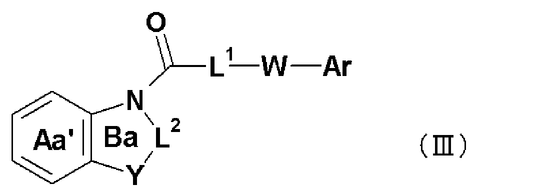

wherein ring Aa' is an optionally substituted benzene ring,
ring Ba is a 6- to 8-membered nonaromatic nitrogen-containing heterocycle,
W is -O- or -S(O)nₐ- (nₐ is an integer of 0 to 2),
Y is a bond, -O-, -NR- (R is a hydrogen atom or an optionally substituted C₁₋₆ alkyl group) or -S(O)n_{b}- (n_{b} is an integer of 0 to 2),
L¹ is (1) a chained C₁₋₅ alkylene group optionally substituted by an optionally halogenated C₁₋₆ alkyl group or (2) an optionally substituted chained C₂₋₅ alkenylene group,
L² is (1) a chained C₂₋₅ alkylene group optionally substituted by substituent(s) selected from a fluorine atom, an optionally halogenated C₁₋₆ alkyl group, an optionally substituted C₇₋₁₁ aralkyl group, an optionally halogenated C₁₋₆ alkoxy group, a hydroxy group and an oxo group or (2) an optionally substituted chained C₂₋₅ alkenylene group, and
Ar is an optionally substituted phenyl group or an optionally substituted bicyclic benzene fused ring group,
or a salt thereof.
[14] The agent of the above-mentioned [13], wherein W is -O-.
[15] The agent of the above-mentioned [13], wherein L¹ is a chained C₁₋₅ alkylene group.
[16] The agent of the above-mentioned [13], wherein ring Ba is a 6-membered nonaromatic nitrogen-containing heterocycle.
[17] The agent of the above-mentioned [13], wherein ring Aa' is a benzene ring optionally substituted by 1 to 3 substituents selected from a halogen atom, an optionally halogenated C₁₋₆ alkyl group, a carboxyl group and a C₁₋₆ alkoxy-carbonyl group.
[18] The agent of the above-mentioned [13], wherein Ar is a phenyl group, an indanyl group, a tetrahydronaphthyl group or a 5-isoquinolinyl group each optionally substituted by 1 to 3 substituents selected from a halogen atom, an optionally halogenated C₁₋₆ alkyl group, a heterocyclic group, an optionally halogenated C₁₋₆ alkoxy group, a C₇₋₁₄ aralkyloxy group, an optionally halogenated C₁₋₆ alkylthio group, a hydroxy group, a mercapto group, a cyano group, a carboxyl group, a formyl group, an optionally halogenated C₁₋₆ alkyl-carbonyl group, a C₆₋₁₄ aryl-carbonyl group, a heterocyclic group-carbonyl group, a C₁₋₆ alkoxy-carbonyl group, a mono- or di-C₁₋₆ alkylamino group, a formylamino group, an optionally halogenated C₁₋₆ alkyl-carbonylamino group, a carbamoyl group, a mono- or di-C₁₋₆ alkyl-carbamoyl group, a C₆₋₁₄ aryl group, an oxo group, a hydroxyimino group and a C₁₋₆ alkoxyimino group.
[19] The agent of the above-mentioned [13], wherein ring Aa' is a benzene ring optionally substituted by a halogen atom,
   ring Ba is a 6-membered nonaromatic nitrogen-containing heterocycle, W is -O-, Y is -NR- (R is a hydrogen atom or an optionally substituted C₁₋₆ alkyl group),
   L¹ is a methylene group optionally substituted by an optionally halogenated C₁₋₆ alkyl group,
   L² is a chained C₂₋₅ alkylene group optionally substituted by substituent(s) selected from a C₁₋₆ alkyl group and an oxo group, and
   Ar is an optionally substituted bicyclic benzene fused ring group.
[20] A method for the prophylaxis or treatment of irritable bowel syndrome, comprising administering an effective amount of a compound having a TGR5 receptor agonistic activity to a mammal.
[21] A method for the prophylaxis or treatment of irritable bowel syndrome, comprising administering an effective amount of the fused ring compound of the above-mentioned [3] or [13] to a mammal.
[22] Use of a compound having a TGR5 receptor agonistic activity for the production of an agent for the prophylaxis or treatment of irritable bowel syndrome.
[23] Use of the fused ring compound of the above-mentioned [3] or [13] for the production of an agent for the prophylaxis or treatment of irritable bowel syndrome.

### Effect of the Invention

A medicament having an action to suppress stress-induced bowel movement, and capable of improving abdominal symptoms of irritable bowel syndrome (IBS) in which stress is considered to be deeply involved in the pathology, particularly abdominal symptoms of diarrhea type irritable bowel syndrome (IBS) can be provided.
Examples of such medicament include a compound having a TGR5 receptor agonistic activity (e.g., a fused ring compound represented by the formula (I) or a salt thereof), a fused ring compound represented by the formula (II) or the formula (III) or a salt thereof and the like.
Here, for example, a fused ring compound represented by the formula (II) or the formula (III) or a salt thereof and the like may exert an IBS-improving effect via or not via a TGR5 receptor.
As the medicament, a compound having a TGR5 receptor agonistic activity is desirable.

### Brief Description of the Drawings

Fig. 1 is a drawing showing the cAMP production amount when CHO-mTGR5 is stimulated with each compound, wherein the vertical axis shows an increase rate relative to the cAMP amount when stimulated with an assay medium without the compound.

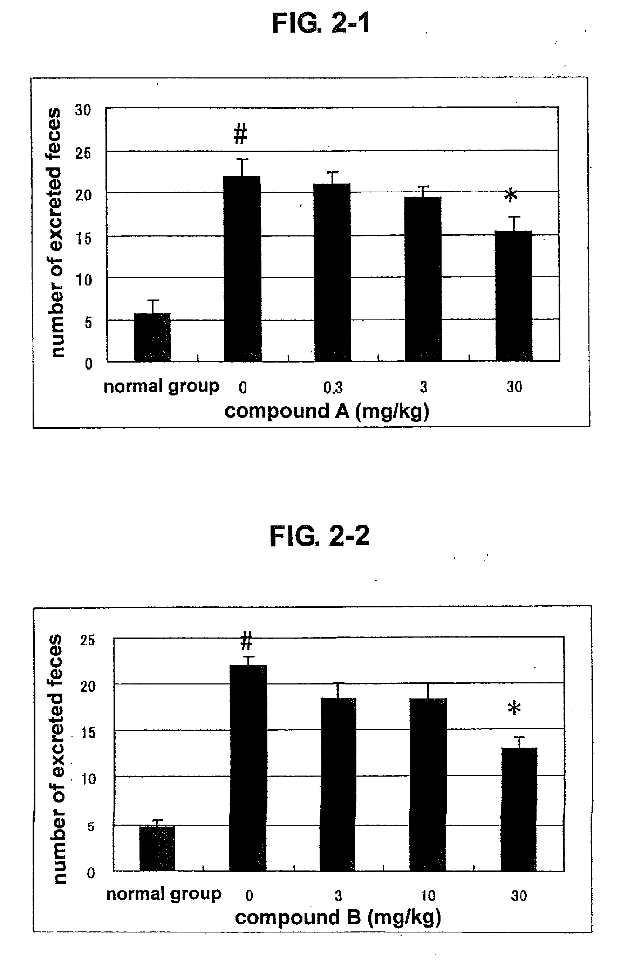

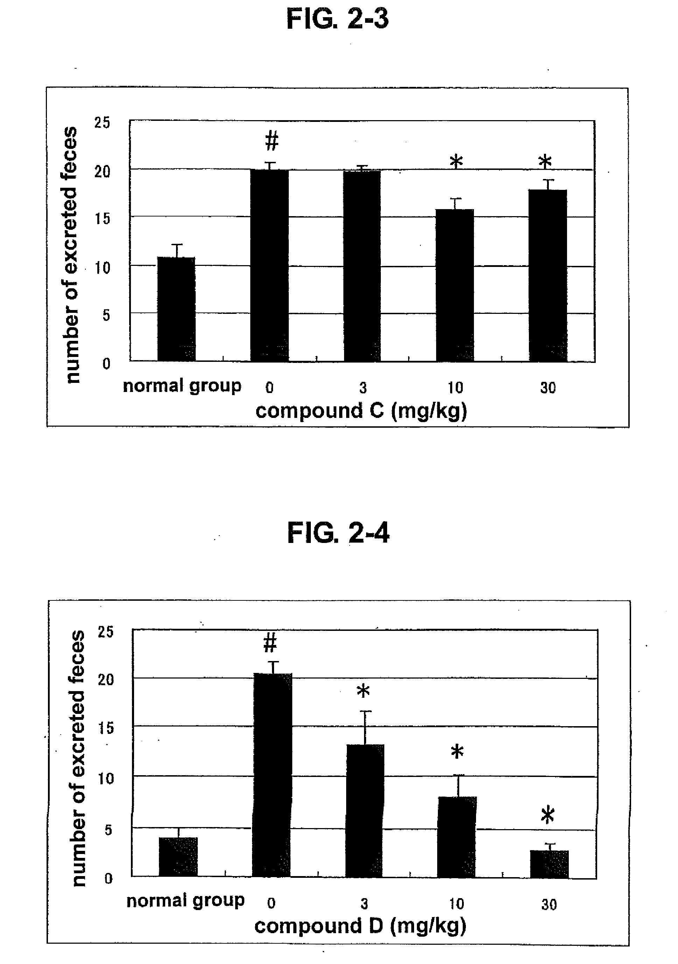

Fig. 2 is a drawing showing the effect of a compound relative to mouse restraint stress-induced bowel movement. Fig. 2-1 shows the results of compound A, Fig. 2-2 shows the results of compound B, Fig. 2-3 shows the results of compound C, and Fig. 2-4 shows the results of compound D. The vertical axis shows bowel movement numbers for 2 hr in a normal group or restraint stress treatment group (n=6, average value ± standard error). # in the drawing shows a significant increase in the bowel movement number relative to the normal group (p<0.01: Student t-test). * in the drawing shows a significant increase in the bowel movement number relative to a compound 0 mg/kg administration group (p<0.01:parametric Williams' test).
Fig. 3 shows the effect of each compound (compounds A, D and E) for rat exo-vivo ileum peristalsis. The horizontal axis shows time, and the vertical axis shows intraluminal pressure of the intestine. The arrow shows the timing when compounds of each concentration were added.

### (Detailed Description of the Invention)

The definitions of the substituents to be frequently used in the present specification are as follows.
Examples of the "halogen atom" include fluorine, chlorine, bromine and iodine.

Examples of the "C₁₋₆ alkyl group" include methyl, ethyl, propyl, isopropyl, butyl, isobutyl, sec-butyl, tert-butyl, pentyl, isopentyl, neopentyl, hexyl and the like.
Examples of the "optionally halogenated C₁₋₆ alkyl group" include a C₁₋₆ alkyl group optionally having 1 to 5, preferably 1 to 3, halogen atoms. Specific examples include methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

Examples of the "C₁₋₆ alkoxy group" include methoxy, ethoxy, propoxy, isopropoxy, butoxy, tert-butoxy, pentyloxy, hexyloxy and the like.
Examples of the "optionally halogenated C₁₋₆ alkoxy group" include a C₁₋₆ alkoxy group optionally having 1 to 5, preferably 1 to 3, halogen atoms. Specific examples include methoxy, fluoromethoxy, difluoromethoxy, trifluoromethoxy, ethoxy, 2,2,2-trichloroethoxy, 2,2,2-trifluoroethoxy, propoxy, isopropoxy, butoxy, 4,4,4-trifluorobutoxy, isobutoxy, sec-butoxy, tert-butoxy, pentyloxy, isopentyloxy, hexyloxy and the like.

Examples of the "C₁₋₆ alkylthio group" include methylthio, ethylthio, propylthio, isopropylthio, butylthio, isobutylthio, sec-butylthio, tert-butylthio, pentylthio, hexylthio and the like.
Examples of the "optionally halogenated C₁₋₆ alkylthio group" include a C₁₋₆ alkylthio group optionally having 1 to 5, preferably 1 to 3, halogen atoms. Specific examples include methylthio, difluoromethylthio, trifluoromethylthio, ethylthio, propylthio, isopropylthio, butylthio, isobutylthio, sec-butylthio, tert-butylthio, 4,4,4-trifluorobutylthio, pentylthio, hexylthio and the like.

Examples of the "C₂₋₆ alkenyl group" include vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like.

Examples of the "C₃₋₆ cycloalkyl group" include cyclopropyl, cyclobutyl, cyclopentyl, cyclohexyl and the like.

Examples of the "C₆₋₁₄ aryl group" include phenyl, naphthyl, anthryl, phenanthryl, acenaphthyl, indenyl and the like. The aryl group may be partially saturated, and examples of the partially saturated aryl group include indanyl, dihydronaphthyl, tetrahydronaphthyl and the like.

Examples of the "C₇₋₁₁ aralkyl group" include benzyl, 1-phenethyl, 2-phenethyl, 2-phenylpropyl, 3-phenylpropyl, 4-phenylbutyl, 1-naphthylmethyl, 2-naphthylmethyl and the like.

Examples of the "acyl group" include a formyl group, a carboxyl group, an optionally halogenated C₁₋₆ alkyl-carbonyl group (e.g., acetyl, propionyl, butyryl, tert-butylcarbonyl, trifluoroacetyl, pentanoyl), a C₃₋₈ cycloalkyl-carbonyl group (e.g., cyclopropylcarbonyl, cyclobutylcarbonyl, cyclopentylcarbonyl, cyclohexylcarbonyl), a C₆₋₁₄ aryl-carbonyl group (e.g., benzoyl), a heterocyclic group-carbonyl group (e.g., nicotinoyl, isonicotinoyl, pyrrolidinyl-carbonyl, piperidinyl-carbonyl, morpholinyl-carbonyl, piperazinyl-carbonyl, pyridyl-carbonyl, furyl-carbonyl, thienyl-carbonyl, pyrrolyl-carbonyl, oxazolyl-carbonyl, isoxazolyl-carbonyl, thiazolyl-carbonyl, isothiazolyl-carbonyl, pyrazinyl-carbonyl, quinolyl-carbonyl, isoquinolyl-carbonyl), a C₇₋₁₄ aralkyl-carbonyl group (e.g., benzylcarbonyl), a C₁₋₆ alkoxy-carbonyl group (e.g., methoxycarbonyl, ethoxycarbonyl, propoxycarbonyl, sec-propoxycarbonyl, butoxycarbonyl, tert-butoxycarbonyl), a C₇₋₁₄ aralkyloxy-carbonyl group (e.g., benzyloxycarbonyl), a C₆₋₁₄ aryloxy-carbonyl group (e.g., phenoxycarbonyl), a C₁₋₆ alkylthio-carbonyl group (e.g., methylthiocarbonyl, ethylthiocarbonyl), a sulfo group, an optionally halogenated C₁₋₆ alkylsulfonyl group (e.g., methylsulfonyl, ethylsulfonyl, propylsulfonyl, sec-propylsulfonyl, butylsulfonyl, tert-butylsulfonyl, trifluoromethanesulfonyl), a C₆₋₁₄ arylsulfonyl group (e.g., phenylsulfonyl, p-toluenesulfonyl), a heterocyclic group-sulfonyl group (e.g., pyridylsulfonyl, thienylsulfonyl, pyrrolidinosulfonyl, piperidinosulfonyl, morpholinosulfonyl, piperazinosulfonyl), a sulfamoyl group, a mono-or di-C₁₋₆ alkyl-sulfamoyl group (e.g., N-methylsulfamoyl, N-ethylsulfamoyl, N,N-dimethylsulfamoyl, N,N-diethylsulfamoyl), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl, N-propylcarbamoyl), a mono- or di-C₇₋₁₄ aralkyl-carbamoyl group (e.g., benzylcarbamoyl, phenethylcarbamoyl), a mono- or di-C₆₋₁₄ aryl-carbamoyl group (e.g., phenylcarbamoyl, naphthylcarbamoyl), a heterocyclic group-carbamoyl group (e.g., pyridylcarbamoyl, thiazolylcarbamoyl), a mono- or di-carbazoyl group and the like. These acyl groups may have, at substitutable positions, 1 to 3 substituents selected from a halogen atom (e.g., fluorine, chlorine, bromine, iodine), a nitro group, a cyano group, an optionally halogenated C₁₋₆ alkyl group (e.g., methyl, ethyl, trifluoromethyl), an optionally substituted C₃₋₈ cycloalkyl group (e.g., cyclopropyl, cyclohexyl, methoxycarbonyl-cyclohexyl, hydroxycarbonyl-cyclohexyl, cycloheptyl), an optionally halogenated C₁₋₆ alkylsulfonyl group (e.g., methylsulfonyl, ethylsulfonyl, propylsulfonyl, isopropylsulfonyl, butylsulfonyl, tert-butylsulfonyl, trifluoromethanesulfonyl), a mono- or di-C₁₋₆ alkylamino group (e.g., methylamino, dimethylamino, ethylamino), a C₁₋₆ alkylthio group (e.g., methylthio), a hydroxy group, a C₁₋₆ alkoxy group (e.g., methoxy, ethoxy, propoxy, isopropoxy), a C₁₋₆ alkoxy-carbonyl group (e.g., t-butoxycarbonyl), a hydroxycarbonyl group, a heterocyclic group (e.g., pyridyl, imidazolyl, tetrahydrofuryl, thienyl, furyl) and the like.

The definition of each symbol used in the present specification is explained in detail in the following.
The present invention provides an agent for the prophylaxis or treatment of irritable bowel syndrome (preferably, diarrhea type irritable bowel syndrome), comprising a fused ring compound represented by the formula

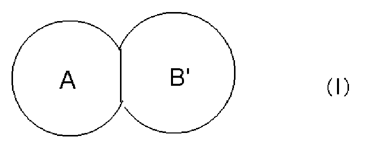

wherein ring A is an optionally substituted aromatic ring,
ring B' is a 5- to 9-membered ring having one or more substituents (hereinafter sometimes to be abbreviated as compound (I)), or a salt thereof.

As the aromatic ring for ring A, for example, an aromatic hydrocarbon and an aromatic heterocycle can be mentioned.
As the "aromatic hydrocarbon", for example, a C₆₋₁₄ aromatic hydrocarbon (e.g., benzene, naphthalene, anthracene, phenanthrene) can be mentioned. Among them, benzene is preferable.
As the "aromatic heterocycle", for example, a 5- or 6-membered monocyclic aromatic heterocycle having, as ring-constituting atom(s) besides a carbon atom, 1 to 4 hetero atoms selected from a nitrogen atom, an oxygen atom and a sulfur atom, a fused ring of the monocyclic aromatic heterocycle and a benzene ring or a 5- or 6-membered monocyclic aromatic heterocycle, and the like can be mentioned. As specific examples of the "aromatic heterocycle", furan, thiophene, pyrrole, oxazole, isoxazole, thiazole, isothiazole, imidazole, pyrazole, 1,2,3-oxadiazole, 1,2,3-thiadiazole, 1,2,3-triazole, pyridine, pyridazine, pyrimidine, pyrazine, 1,2,4-triazine, benzofuran, isobenzofuran, benzo[b]thiophene, indole, isoindole, 1H-indazole, benzimidazole, benzoxazole, 1,2-benzisoxazole, benzothiazole, 1,2-benzisothiazole, 1H-benzotriazole, quinoline, isoquinoline, cinnoline, quinazoline, quinoxaline, phthalazine, naphthyridine and the like can be mentioned. Among them, pyridine is preferable.
The aromatic ring for ring A is preferably a monocyclic aromatic ring, more preferably a benzene ring or a pyridine ring.

The aromatic ring for ring A optionally has 1 to 4 substituents at substitutable positions. Examples of such substituent include a halogen atom, a nitro group, a cyano group,
an optionally substituted heterocyclic group, an optionally substituted hydroxy group, an optionally substituted mercapto group, an optionally substituted amino group, an acyl group, an optionally substituted hydrocarbon group, an optionally substituted sulfinyl group and the like.

### (1) Optionally substituted heterocyclic group

Examples of the "heterocyclic group" of the "optionally substituted heterocyclic group" include a 5- to 7-membered monocyclic heterocyclic group containing, as a ring-constituting atom besides carbon atom, 1 to 4 hetero atoms selected from a nitrogen atom, an oxygen atom and a sulfur atom (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, oxiranyl, azetidinyl, oxetanyl, thietanyl, pyrrolidinyl, tetrahydrofuranyl, thioranyl, piperidinyl, tetrahydropyranyl, morpholinyl, thiomorpholinyl, piperazinyl, dioxolanyl, dioxanyl, dithianyl, perhydroazepinyl and the like, preferably a 5- or 6-membered monocyclic heterocyclic group), a bicyclic or tricyclic fused heterocyclic group (e.g., benzofuryl, isobenzofuryl, dihydrobenzofuryl, dihydroisobenzofuryl, benzo[b]thienyl, indolyl, isoindolyl, 1H-indazolyl, benzimidazolyl, benzoxazolyl, 1,2-benzoisooxazolyl, benzothiazolyl, 1,2-benzoisothiazolyl, 1H-benzotriazolyl, quinolyl, isoquinolyl, dihydroquinolyl, dihydroisoquinolyl, tetrahydroquinolyl, tetrahydroisoquinolyl, cinnolyl, quinazolinyl, quinoxalinyl, phthalazinyl, naphthyridinyl, purinyl, pteridinyl, carbazolyl, α-carbolinyl, β-carbolinyl, γ-carbolinyl, acrydinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-a]pyridazinyl, imidazo[1,2-a]pyrimidinyl, 1,2,4-triazolo[4,3-a]pyridyl, 1,2,4-triazolo[4,3-b]pyridazinyl, isochromanyl, chromanyl, isochromenyl, chromenyl, indolinyl, isoindolinyl, benzodioxolyl) and the like.

Preferred are an aromatic nitrogen-containing heterocyclic group (e.g., pyrrolyl, oxazolyl, isoxazolyl, thiazolyl, isothiazolyl, imidazolyl, pyrazolyl, oxadiazolyl, furazanyl, thiadiazolyl, triazolyl, tetrazolyl, pyridyl, pyridazinyl, pyrimidinyl, pyrazinyl), furyl, thienyl, pyrrolidinyl, morpholinyl, piperidinyl and perhydroazepinyl.

The "heterocyclic group" may have 1 to 5 substituents at substitutable positions, and as such substituent, a halogen atom (e.g., fluorine, chlorine, bromine, iodine), an optionally halogenated C₁₋₆ alkyl group (e.g., methyl, trifluoromethyl, neopentyl), a hydroxy-C₁₋₆ alkyl group (e.g., hydroxymethyl), an amino-C₁₋₆ alkyl group (e.g., aminomethyl), a C₁₋₆ alkoxy-carbonylamino-C₁₋₆ alkyl group (e.g., tert-butoxycarbonylaminomethyl), a C₂₋₆ alkenyl group (e.g., vinyl, propenyl), a C₂₋₆ alkynyl group (e.g., ethynyl, propargyl), a C₃₋₈ cycloalkyl group (e.g., cyclopropyl, cyclobutyl, cyclopentyl, cyclohexyl), a heterocyclic group, a C₇₋₁₄ aralkyl group (e.g., benzyl, phenethyl, phenylpropyl), an optionally halogenated C₁₋₆ alkoxy group (e.g., methoxy, ethoxy, trifluoromethoxy), a C₆₋₁₄ aryloxy group (e.g., phenoxy), a heterocyclic group-oxy group, a C₇₋₁₄ aralkyloxy group (e.g., benzyloxy, phenethyloxy, phenylpropyloxy), a formyloxy group, a C₁₋₆ alkyl-carbonyloxy group (e.g., acetyloxy), an optionally halogenated C₁₋₆ alkylthio group (e.g., methylthio), a C₁₋₆ alkylsulfinyl group (e.g., methylsulfinyl), a hydroxy group, a mercapto group, a cyano group, a nitro group, a carboxyl group, a formyl group, an optionally halogenated C₁₋₆ alkyl-carbonyl group (e.g., acetyl, propionyl, trifluoroacetyl), a C₆₋₁₄ aryl-carbonyl group (e.g., benzoyl), a heterocyclic group-carbonyl group, a C₁₋₆ alkoxy-carbonyl group (e.g., methoxycarbonyl, ethoxycarbonyl, propoxycarbonyl), a C₆₋₁₄ aryloxy-carbonyl group (e.g., phenoxycarbonyl), an amino group, a mono- or di-C₁₋₆ alkylamino group (e.g., methylamino, ethylamino, dimethylamino, diethylamino), a formylamino group, an amino group mono- or disubstituted by optionally halogenated C₁₋₆ alkyl-carbonyl (e.g., acetylamino, propionylamino, butyrylamino, trifluoroacetylamino, diacetylamino), a C₁₋₆ alkoxy-carbonylamino group (e.g., methoxycarbonylamino, ethoxycarbonylamino, propoxycarbonylamino, tert-butoxycarbonylamino), a ureido group, a mono- or di- or tri-C₁₋₆ alkyl-ureido group (e.g., 1-methylureido, 3-methylureido, 3,3-dimethylureido, 1,3-dimethylureido, 1,3,3-trimethylureido), an optionally halogenated C₁₋₆ alkyl-sulfonylamino group (e.g., methylsulfonylamino, trifluoromethanesulfonylamino), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), a sulfo group, an optionally halogenated C₁₋₆ alkylsulfonyl group (e.g., methylsulfonyl, ethylsulfonyl, propylsulfonyl, sec-propylsulfonyl, butylsulfonyl, tert-butylsulfonyl, trifluoromethanesulfonyl), a C₆₋₁₄ arylsulfonyl group (e.g., phenylsulfonyl, naphthylsulfonyl), a heterocyclic group-sulfonyl group, a sulfamoyl group, a mono- or di-C₁₋₆ alkyl-sulfamoyl group (e.g., N-methylsulfamoyl, N-ethylsulfamoyl, N,N-dimethylsulfamoyl, N,N-diethylsulfamoyl), a C₆₋₁₄ aryl-carbonyl-C₁₋₆ alkoxy group (e.g., benzoylmethoxy), a hydroxy-C₁₋₆ alkoxy group (e.g., hydroxyethoxy), a C₁₋₆ alkoxy-C₁₋₆ alkoxy group (e.g., methoxymethoxy), a carboxy-C₁₋₆ alkoxy group (e.g., carboxymethoxy), a C₁₋₆ alkoxy-carbonyl-C₁₋₆ alkoxy group (e.g., methoxycarbonylmethoxy), a C₃₋₁₄ cycloalkyl-C₁₋₆ alkoxy group (e.g., cyclohexylmethoxy), a heterocyclic group-C₁₋₆ alkoxy group, a C₇₋₁₄ aralkyloxy-carbonyl-C₁₋₆ alkoxy group (e.g., benzyloxycarbonylmethoxy), a hydroxyphenyl-C₁₋₆ alkoxy group (e.g., [3-(4-hydroxyphenyl)propyl]oxy), a C₇₋₁₄ aralkyloxy-carbonyl group (e.g., benzyloxycarbonyl), a mono- or di-C₁₋₆ alkylamino-C₁₋₆ alkoxy (e.g., methylaminoethoxy, ethylaminoethoxy, dimethylaminoethoxy), a mono- or di-C₁₋₆ alkylamino-carbonyloxy (e.g., methylaminocarbonyloxy, ethylaminocarbonyloxy, dimethylaminocarbonyloxy), a C₆₋₁₄ aryl group (e.g., phenyl), a carbamoyl-C₁₋₆ alkoxy group (e.g., carbamoylmethoxy), an amino-C₁₋₆ alkoxy group (e.g., aminomethoxy), a C₁₋₆ alkoxy-carbonylamino-C₁₋₆ alkoxy group (e.g., methoxycarbonylaminomethoxy, tert-butoxycarbonylaminomethoxy) can be used. Examples of the heterocycle of the "heterocyclic group", "heterocyclic group-oxy group", "heterocyclic group-carbonyl group", "heterocyclic group-sulfonyl group" and "heterocyclic group-C₁₋₆ alkoxy group" include those similar to the groups exemplified as the "heterocyclic group" of (1) "optionally substituted heterocyclic group" which is the substituent of ring A.
As the "heterocyclic group", furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, oxiranyl, azetidinyl, oxetanyl, thietanyl, pyrrolidinyl, tetrahydrofuranyl, thioranyl, piperidinyl, tetrahydropyranyl, morpholinyl, thiomorpholinyl, piperazinyl, dioxolanyl, dioxanyl, dithianyl, benzofuryl, isobenzofuryl, dihydrobenzofuryl, dihydroisobenzofuryl, benzo[b]thienyl, indolyl, isoindolyl, 1H-indazolyl, benzimidazolyl, benzoxazolyl, 1,2-benzoisooxazolyl, benzothiazolyl, 1,2-benzoisothiazolyl, 1H-benzotriazolyl, quinolyl, isoquinolyl, dihydroquinolyl, dihydroisoquinolyl, tetrahydroquinolyl, tetrahydroisoquinolyl, cinnolyl, quinazolinyl, quinoxalinyl, phthalazinyl, naphthyridinyl, purinyl, pteridinyl, carbazolyl, α-carbolinyl, β-carbolinyl, γ-carbolinyl, acrydinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-a]pyridazinyl, imidazo[1,2-a]pyrimidinyl, 1,2,4-triazolo[4,3-a]pyridyl, 1,2,4-triazolo[4,3-b]pyridazinyl, isochromanyl, chromanyl, isochromenyl, chromenyl, indolinyl, isoindolinyl, benzodioxolyl and the like are preferable.
As the "heterocyclic group-oxy group", pyridyloxy is preferable.
As the "heterocyclic group-carbonyl group", for example, nicotinoyl, isonicotinoyl, pyrrolidinyl-carbonyl, piperidinyl-carbonyl, morpholinyl-carbonyl, piperazinyl-carbonyl, pyridyl-carbonyl, furyl-carbonyl, thienyl-carbonyl, pyrrolyl-carbonyl, oxazolyl-carbonyl, isoxazolyl-carbonyl, thiazolyl-carbonyl, isothiazolyl-carbonyl, pyrazinyl-carbonyl, quinolyl-carbonyl and isoquinolyl-carbonyl are preferable.
As the "heterocyclic group-sulfonyl group", for example, pyridylsulfonyl, thienylsulfonyl, pyrrolidinosulfonyl, piperidinosulfonyl, morpholinosulfonyl and piperazinosulfonyl are preferable.
As the "heterocyclic group-C₁₋₆ alkoxy group", imidazol-1-ylpropyloxy is preferable.

### (2) Optionally substituted hydroxy group

Examples of the substituent of the "optionally substituted hydroxy group" include (i) an optionally substituted C₁₋₆ alkyl group, (ii) an optionally substituted C₆₋₁₀ aryl group, (iii) an optionally substituted C₇₋₁₄ aralkyl group, (iv) an acyl group, (v) an optionally substituted heterocyclic group and the like.

The "optionally substituted C₁₋₆ alkyl group" of (i) is a "C₁₋₆ alkyl group" optionally having 1 to 3 substituents at substitutable positions, and examples of the C₁₋₆ alkyl group include methyl, ethyl, propyl, isopropyl, butyl, pentyl, neopentyl and the like. Examples of the substituent include a halogen atom (e.g., fluorine, chlorine, bromine, iodine), a hydroxy group, a C₁₋₆ alkoxy group (e.g., methoxy, ethoxy, propoxy, isopropoxy), a formyl group, a C₁₋₆ alkyl-carbonyl group (e.g., acetyl, propionyl, butyryl), a C₆₋₁₄ aryl-carbonyl group (e.g., benzoyl), a C₇₋₁₄ aralkyloxy-carbonyl group (e.g., a benzyloxycarbonyl group), C₃₋₁₄ cycloalkyl (e.g., cyclohexyl), a carboxyl group, a C₁₋₆ alkoxy-carbonyl group (e.g., methoxycarbonyl, ethoxycarbonyl, propoxycarbonyl, sec-propoxycarbonyl, butoxycarbonyl, tert-butoxycarbonyl), an amino group, a mono- or di-C₁₋₆ alkylamino group (e.g., methylamino, ethylamino, dimethylamino, diethylamino), a heterocyclic group (e.g., a 5- or 6-membered nitrogen-containing heterocyclic group (e.g., imidazolyl, pyrrolidinyl, piperidinyl, morpholinyl, thiomorpholinyl, piperazinyl, triazolyl)), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), a mono- or di-(5- or 6-membered nitrogen-containing heterocyclic group (preferably a 5-membered nitrogen-containing heterocyclic group (e.g., imidazolyl))-C₁₋₆ alkyl)-carbamoyl group (e.g., imidazolylpropylcarbamoyl), a mono- or di-C₇₋₁₄ aralkyl-carbamoyl group (e.g., benzylcarbamoyl, phenethylcarbamoyl), a C₆₋₁₄ aryloxy group (e.g., phenoxy), a mono- or di-C₁₋₆ alkyl-carbamoyloxy group (e.g., N-methylcarbamoyloxy, N-ethylcarbamoyloxy, N,N-dimethylcarbamoyloxy, N,N-diethylcarbamoyloxy), a formylamino group, a C₁₋₆ alkyl-carbonylamino group (e.g., acetylamino, propionylamino, butyrylamino), a C₁₋₆ alkoxy-carbonylamino group (e.g., methoxycarbonylamino, ethoxycarbonylamino, tert-butoxycarbonylamino), a formyloxy group, a C₁₋₆ alkyl-carbonyloxy group (e.g., acetoxy), an optionally halogenated C₁₋₆ alkylsulfonyl group (e.g., methylsulfonyl, ethylsulfonyl, propylsulfonyl, isopropylsulfonyl, butylsulfonyl, tert-butylsulfonyl, trifluoromethanesulfonyl), a cyano group, an oxo group, a hydroxyphenyl group and the like.

Examples of the "C₆₋₁₀ aryl group" of the "optionally substituted C₆₋₁₀ aryl group" of (ii) include phenyl, naphthyl and the like.

Examples of the "C₇₋₁₄ aralkyl group" of the "optionally substituted C₇₋₁₄ aralkyl group" of (iii) include benzyl, phenethyl and the like.
The aforementioned (ii) "C₆₋₁₀ aryl group" and (iii) "C₇₋₁₄ aralkyl group" may have 1 to 5 substituents at substitutable positions. Examples of such substituent include the substituents exemplified for the aforementioned "optionally substituted C₁₋₆ alkyl group", a C₁₋₆ alkyl group optionally substituted by 1 to 5, preferably 1 to 3, halogen atoms (e.g., methyl, ethyl, propyl, isopropyl, trifluoromethyl) and the like.

Examples of the "acyl group" of (iv) include a formyl group, a carboxyl group, an optionally halogenated C₁₋₆ alkyl-carbonyl group (e.g., acetyl, propionyl, butyryl, tert-butylcarbonyl, trifluoroacetyl, pentanoyl), a C₃₋₈ cycloalkyl-carbonyl group (e.g., cyclopropylcarbonyl, cyclobutylcarbonyl, cyclopentylcarbonyl, cyclohexylcarbonyl), a C₆₋₁₄ aryl-carbonyl group (e.g., benzoyl), a heterocyclic group-carbonyl group, a C₇₋₁₄ aralkyl-carbonyl group (e.g., benzylcarbonyl), a C₁₋₆ alkoxy-carbonyl group (e.g., methoxycarbonyl, ethoxycarbonyl, propoxycarbonyl, sec-propoxycarbonyl, butoxycarbonyl, tert-butoxycarbonyl), a C₇₋₁₄ aralkyloxy-carbonyl group (e.g., benzyloxycarbonyl), a C₆₋₁₄ aryloxy-carbonyl group (e.g., phenoxycarbonyl), a C₁₋₆ alkylthio-carbonyl group (e.g., methylthiocarbonyl, ethylthiocarbonyl), a sulfo group, an optionally halogenated C₁₋₆ alkylsulfonyl group (e.g., methylsulfonyl, ethylsulfonyl, propylsulfonyl, sec-propylsulfonyl, butylsulfonyl, tert-butylsulfonyl, trifluoromethanesulfonyl), a C₆₋₁₄ arylsulfonyl group (e.g., phenylsulfonyl, p-toluenesulfonyl), a heterocyclic group-sulfonyl group, a sulfamoyl group, a mono- or di-C₁₋₆ alkyl-sulfamoyl group (e.g., N-methylsulfamoyl, N-ethylsulfamoyl, N,N-dimethylsulfamoyl, N,N-diethylsulfamoyl), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl, N-propylcarbamoyl), a mono- or di-C₇₋₁₄ aralkyl-carbamoyl group (e.g., benzylcarbamoyl, phenethylcarbamoyl), a mono- or di-C₆₋₁₄ aryl-carbamoyl group (e.g., phenylcarbamoyl, naphthylcarbamoyl), a heterocyclic group-carbamoyl group, a mono-or di-carbazoyl group and the like. These acyl groups may have, at substitutable positions, 1 to 3 substituents selected from (a) a halogen atom (e.g., fluorine, chlorine, bromine, iodine), (b) a nitro group, (c) a cyano group, (d) an optionally halogenated C₁₋₆ alkyl group (e.g., methyl, ethyl, trifluoromethyl), (e) a C₃₋₈ cycloalkyl group optionally substituted by a C₁₋₆ alkoxy-carbonyl group or a hydroxycarbonyl group (e.g., cyclopropyl, cyclohexyl, methoxycarbonyl-cyclohexyl, hydroxycarbonyl-cyclohexyl, cycloheptyl), (f) an optionally halogenated C₁₋₆ alkylsulfonyl group (e.g., methylsulfonyl, ethylsulfonyl, propylsulfonyl, isopropylsulfonyl, butylsulfonyl, tert-butylsulfonyl, trifluoromethanesulfonyl), (g) a mono- or di-C₁₋₆ alkylamino group (e.g., methylamino, dimethylamino, ethylamino), (h) a C₁₋₆ alkylthio group (e.g., methylthio), (i) a hydroxy group, (j) a C₁₋₆ alkoxy group (e.g., methoxy, ethoxy, propoxy, isopropoxy), (k) a C₁₋₆ alkoxy-carbonyl group (e.g., t-butoxycarbonyl), (1) a hydroxycarbonyl group, (m) a heterocyclic group and the like.
Examples of the "heterocyclic group" and "heterocyclic group" of the "heterocyclic group-carbonyl group", "heterocyclic group-sulfonyl group" and "heterocyclic group-carbamoyl group" include those similar to the groups exemplified as the "heterocyclic group" of (1) "optionally substituted heterocyclic group" which is the substituent of ring A.
As the "heterocyclic group-carbonyl group", preferred are nicotinoyl, isonicotinoyl, pyrrolidinyl-carbonyl, piperidinyl-carbonyl, morpholinyl-carbonyl, piperazinyl-carbonyl, pyridyl-carbonyl, furyl-carbonyl, thienyl-carbonyl, pyrrolyl-carbonyl, oxazolyl-carbonyl, isoxazolyl-carbonyl, thiazolyl-carbonyl, isothiazolyl-carbonyl, pyrazinyl-carbonyl, quinolyl-carbonyl and isoquinolyl-carbonyl.
As the "heterocyclic group-sulfonyl group", pyridylsulfonyl, thienylsulfonyl, pyrrolidinosulfonyl, piperidinosulfonyl, morpholinosulfonyl and piperazinosulfonyl are preferable.
As the "heterocyclic group-carbamoyl group", pyridylcarbamoyl and thiazolylcarbamoyl are preferable.
As the "heterocyclic group", pyridyl, imidazolyl, tetrahydrofuryl, thienyl and furyl are preferable.

The "optionally substituted heterocyclic group" of (v) is as defined for (1) "optionally substituted heterocyclic group" which is the substituent of ring A.

Preferable examples of the "optionally substituted hydroxy group" include a hydroxy group, an optionally halogenated C₁₋₆ alkoxy group (e.g., methoxy), a C₃₋₆ alkenyloxy group (e.g., allyloxy), a C₆₋₁₄ aryloxy group (e.g., phenoxy), a C₇₋₁₄ aralkyloxy group (e.g., benzyloxy, phenethyloxy, phenylpropyloxy), a formyloxy group, a C₁₋₆ alkyl-carbonyloxy group (e.g., acetyloxy), a hydroxy-C₁₋₆ alkoxy group (e.g., hydroxyethoxy), a C₁₋₆ alkoxy-C₁₋₆ alkoxy group (e.g., methoxyethoxy), a carboxy-C₁₋₆ alkoxy group (e.g., carboxymethoxy), a C₁₋₆ alkoxy-carbonyl-C₁₋₆ alkoxy group (e.g., methoxycarbonylmethoxy, tert-butoxycarbonylmethoxy), a mono- or di-C₁₋₆ alkyl-carbamoyloxy (e.g., methylcarbamoyloxy, ethylcarbamoyloxy, dimethylcarbamoyloxy), a trityloxy group, a heterocyclic group-oxy group (e.g., pyridyloxy) and the like.

### (3) Optionally substituted mercapto group

Examples of the substituent of the "optionally substituted mercapto group" include those similar to the substituents exemplified as the substituent of the aforementioned (2) "optionally substituted hydroxy group".
Preferable examples of the "optionally substituted mercapto group" include a mercapto group, an optionally halogenated C₁₋₆ alkylthio group (e.g., methylthio), a C₃₋₆ alkenylthio group (e.g., allylthio), a C₆₋₁₄ arylthio group (e.g., phenylthio), a C₇₋₁₄ aralkylthio group (e.g., benzylthio, phenethylthio, phenylpropylthio) and the like.

### (4) Optionally substituted amino group

Examples of the substituent of the "optionally substituted amino group" include a C₁₋₆ alkyl group (e.g., methyl, ethyl, propyl, isopropyl), a C₆₋₁₄ aryl group (e.g., phenyl) and a C₇₋₁₄ aralkyl group (e.g., benzyl), each of which is optionally substituted by 1 to 5 halogen atoms (e.g., fluorine, chlorine, bromine, iodine); an acyl group (e.g., acetyl, benzoyl, phenylsulfonyl) and the like. The number of substituents is 1 or 2.

### (5) Acyl group

The "acyl group" is as defined for (iv) "acyl group" exemplified as the substituent of (2) "optionally substituted hydroxy group" recited as the substituent of ring A.

### (6) Optionally substituted hydrocarbon group

Examples of the "hydrocarbon group" of the "optionally substituted hydrocarbon group" include an aliphatic hydrocarbon group, an alicyclic hydrocarbon group, an aryl group, an aralkyl group, and a group obtained by combining these groups.

Preferable examples of the "aliphatic hydrocarbon group" include a C₁₋₁₀ aliphatic hydrocarbon group (e.g., a C₁₋₁₀ alkyl group, a C₂₋₁₀ alkenyl group, a C₂₋₁₀ alkynyl group) and the like.
Examples of the "C₁₋₁₀ alkyl group" include methyl, ethyl, propyl, isopropyl, butyl, isobutyl, sec-butyl, tert-butyl, pentyl, isopentyl, neopentyl, 1-methylpropyl, hexyl, isohexyl, 1,1-dimethylbutyl, 2,2-dimethylbutyl, 3,3-dimethylbutyl, 3,3-dimethylpropyl, 2-ethylbutyl, heptyl and the like. Preferred is methyl.
Examples of the "C₂₋₁₀ alkenyl group" include vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like.
Examples of the "C₂₋₁₀ alkynyl group" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like.

Preferable examples of the "alicyclic hydrocarbon group" include a C₃₋₁₀ alicyclic hydrocarbon group (e.g., a C₃₋₁₀ cycloalkyl group, a C₃₋₁₀ cycloalkenyl group, a C₅₋₁₀ cycloalkadienyl group) and the like.
Examples of the "C₃₋₁₀ cycloalkyl group" include cyclopropyl, cyclobutyl, cyclopentyl, cyclohexyl, cycloheptyl, cyclooctyl, cyclononyl and the like.
Examples of the "C₃₋₁₀ cycloalkenyl group" include 1-cyclobuten-1-yl, 1-cyclopenten-1-yl, 2-cyclopenten-1-yl, 3-cyclopenten-1-yl, 2-cyclohexen-1-yl, 3-cyclohexen-1-yl and the like.
Examples of the "C₅₋₁₀ cycloalkadienyl group" include 2,4-cyclopentadien-1-yl, 2,5-cyclohexadien-1-yl and the like.

Examples of the "aryl group" include a C₆₋₁₄ aryl group (e.g., phenyl, naphthyl, anthryl, phenanthryl, acenaphthyl, indenyl) and the like. The aryl group may be partially saturated, and examples of the partially saturated aryl group include indanyl, dihydronaphthyl, tetrahydronaphthyl and the like. Among these, phenyl is preferable.

Examples of the "aralkyl group" include a C₇₋₁₄ aralkyl group (e.g., benzyl, 1-phenethyl, 2-phenethyl, 2-phenylpropyl, 3-phenylpropyl, 4-phenylbutyl, 2-naphthylmethyl, benzhydryl), a trityl group and the like.

As the hydrocarbon group besides the above-mentioned, moreover, a C₁₋₆ alkyl-C₆₋₁₄ aryl group (e.g., methylphenyl, ethylphenyl), a C₁₋₆ alkyl-C₃₋₁₀ cycloalkyl group (e.g., methylcyclohexyl, ethylcyclohexyl), a C₁₋₆ alkyl-C₇₋₁₄ aralkyl group (e.g., methylbenzyl, ethylbenzyl), a C₁₋₆ alkylidene group (e.g., methylidene, ethylidene, propylidene), a C₃₋₁₀ cycloalkyl-C₁₋₆ alkyl group (e.g., cyclopropylmethyl, cyclohexylmethyl, cyclohexylethyl, cycloheptylmethyl), a C₈₋₁₄ polycyclic group (e.g., tetralinyl, decalyl, 2-norbornyl, 3-noradamantyl, adamantyl) and the like can also be mentioned.

The aforementioned "hydrocarbon group" may have 1 to 5, preferably 1 to 3, substituents at substitutable positions. Examples of such substituent include a halogen atom (e.g., fluorine, chlorine, bromine, iodine), an optionally substituted C₁₋₆ alkyl group, a nitro group, a cyano group, an oxo group, an optionally substituted heterocyclic group, an optionally halogenated C₁₋₆ alkylthio group, an optionally substituted amino group, an optionally substituted hydroxy group, an optionally substituted mercapto group, an acyl group and the like.

The "optionally substituted C₁₋₆ alkyl group" is as defined for (i) "optionally substituted C₁₋₆ alkyl group" exemplified as the substituent of (2) "optionally substituted hydroxy group" recited as the substituent of ring A. Examples of the "optionally substituted heterocyclic group", "optionally substituted amino group", "optionally substituted hydroxy group", "optionally substituted mercapto group" and "acyl group" each include those similar to the groups exemplified as the substituent of ring A.

Examples of the "optionally halogenated C₁₋₆ alkylthio group" exemplified as the substituent of the aforementioned "hydrocarbon group" include a C₁₋₆ alkylthio group optionally having 1 to 5, preferably 1 to 3, halogen atoms. Specific examples include methylthio, difluoromethylthio, trifluoromethylthio, ethylthio, propylthio, isopropylthio, butylthio, isobutylthio, sec-butylthio, tert-butylthio, 4,4,4-trifluorobutylthio, pentylthio, hexylthio and the like.

### (7) Optionally substituted sulfinyl group

Examples of the substituent of the "optionally substituted sulfinyl group" include those similar to the substituents exemplified as the substituent of (2) "optionally substituted hydroxy group" recited as the substituent of ring A. Preferable examples of the "optionally substituted sulfinyl group" include a C₁₋₆ alkylsulfinyl group (e.g., methylsulfinyl, ethylsulfinyl) and the like.

Preferable examples of the substituent of ring A include a halogen atom (e.g., fluorine, chlorine, bromine, iodine), an optionally halogenated C₁₋₆ alkyl group (e.g., methyl, ethyl, tert-butyl, trifluoromethyl), a hydroxy-C₁₋₆ alkyl group (e.g., hydroxymethyl), an amino-C₁₋₆ alkyl group (e.g., aminomethyl), a C₁₋₆ alkoxy-carbonylamino-C₁₋₆ alkyl group (e.g., tert-butoxycarbonylaminomethyl), a C₂₋₆ alkenyl group (e.g., vinyl, propenyl), a C₂₋₆ alkynyl group (e.g., ethynyl, propargyl), a C₃₋₈ cycloalkyl group (e.g., cyclopropyl, cyclobutyl, cyclopentyl, cyclohexyl), an optionally substituted heterocyclic group, a C₇₋₁₄ aralkyl group (e.g., benzyl, phenethyl, phenylpropyl), an optionally halogenated C₁₋₆ alkoxy group (e.g., methoxy, ethoxy, trifluoromethoxy), a C₆₋₁₄ aryloxy group (e.g., phenoxy), a heterocyclic group-oxy group, a C₇₋₁₄ aralkyloxy group (e.g., benzyloxy, phenethyloxy, phenylpropyloxy), a formyloxy group, a C₁₋₆ alkyl-carbonyloxy group (e.g., acetyloxy), an optionally halogenated C₁₋₆ alkylthio group (e.g., methylthio, ethylthio, trifluoromethylthio), a C₁₋₆ alkylsulfinyl group (e.g., methylsulfinyl), a hydroxy group, a mercapto group, a cyano group, a nitro group, a carboxyl group, a formyl group, an optionally halogenated C₁₋₆ alkyl-carbonyl group (e.g., acetyl, propionyl, trifluoroacetyl), a C₆₋₁₄ aryl-carbonyl group (e.g., benzoyl), a heterocyclic group-carbonyl group, a C₁₋₆ alkoxy-carbonyl group (e.g., methoxycarbonyl, ethoxycarbonyl, propoxycarbonyl), a C₆₋₁₄ aryloxy-carbonyl group (e.g., phenoxycarbonyl), an amino group, a mono- or di-C₁₋₆ alkylamino group (e.g., methylamino, ethylamino, dimethylamino, diethylamino), a formylamino group, an amino group mono- or disubstituted by optionally halogenated C₁₋₆ alkyl-carbonyl (e.g., acetylamino, propionylamino, butyrylamino, trifluoroacetylamino, diacetylamino), a C₁₋₆ alkoxy-carbonylamino group (e.g., methoxycarbonylamino, ethoxycarbonylamino, propoxycarbonylamino, tert-butoxycarbonylamino), an ureido group, a mono- or di- or tri-C₁₋₆ alkyl-ureido group (e.g., 1-methylureido, 3-methylureido, 3,3-dimethylureido, 1,3-dimethylureido, 1,3,3-trimethylureido), an optionally halogenated C₁₋₆ alkyl-sulfonylamino group (e.g., methylsulfonylamino, trifluoromethanesulfonylamino), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), a sulfo group, an optionally halogenated C₁₋₆ alkylsulfonyl group (e.g., methylsulfonyl, ethylsulfonyl, propylsulfonyl, sec-propylsulfonyl, butylsulfonyl, tert-butylsulfonyl, trifluoromethanesulfonyl), a C₆₋₁₄ arylsulfonyl group (e.g., phenylsulfonyl, naphthylsulfonyl), a heterocyclic group-sulfonyl group, a sulfamoyl group, a mono- or di-C₁₋₆ alkyl-sulfamoyl group (e.g., N-methylsulfamoyl, N-ethylsulfamoyl, N,N-dimethylsulfamoyl, N,N-diethylsulfamoyl), a C₆₋₁₄ aryl-carbonyl-C₁₋₆ alkoxy group (e.g., benzoylmethoxy), a hydroxy-C₁₋₆ alkoxy group (e.g., hydroxyethoxy), a C₁₋₆ alkoxy-C₁₋₆ alkoxy group (e.g., methoxymethoxy), a carboxy-C₁₋₆ alkoxy group (e.g., carboxymethoxy), a C₁₋₆ alkoxy-carbonyl-C₁₋₆ alkoxy group (e.g., methoxycarbonylmethoxy), a C₃₋₁₄ cycloalkyl-C₁₋₆ alkoxy group (e.g., cyclohexylmethoxy), a heterocyclic group-C₁₋₆ alkoxy group, a C₇₋₁₄ aralkyloxy-carbonyl-C₁₋₆ alkoxy group (e.g., benzyloxycarbonylmethoxy), a hydroxyphenyl-C₁₋₆ alkoxy group (e.g., [3-(4-hydroxyphenyl)propyl]oxy), a C₇₋₁₄ aralkyloxy-carbonyl group (e.g., benzyloxycarbonyl), a mono- or di-C₁₋₆ alkylamino-C₁₋₆ alkoxy (e.g., methylaminoethoxy, ethylaminoethoxy, dimethylaminoethoxy), a mono- or di-C₁₋₆ alkylamino-carbonyloxy (e.g., methylaminocarbonyloxy, ethylaminocarbonyloxy, dimethylaminocarbonyloxy), an optionally substituted C₆₋₁₄ aryl group, a carbamoyl-C₁₋₆ alkoxy group (e.g., carbamoylmethoxy), an amino-C₁₋₆ alkoxy group (e.g., aminomethoxy), a C₁₋₆ alkoxy-carbonylamino-C₁₋₆ alkoxy group (e.g., methoxycarbonylaminomethoxy, tert-butoxycarbonylaminomethoxy) (hereinafter to be also referred to as substituent group A) and the like. In addition, when ring A is a pyridine ring, it may be N-oxidized.

The "optionally substituted heterocyclic group" in substituent group A is as defined for (1) "optionally substituted heterocyclic group" recited as the substituent of ring A. In addition, examples of the "heterocyclic group" of the "heterocyclic group-oxy group", "heterocyclic group-carbonyl group", "heterocyclic group-sulfonyl group" and "heterocyclic group-C₁₋₆ alkoxy group" in substituent group A include those similar to the groups exemplified as the "heterocyclic group" of (1) "optionally substituted heterocyclic group" recited as the substituent of ring A.
Examples of the "C₆₋₁₄ aryl group" of the "optionally substituted C₆₋₁₄ aryl group" in substituent group A include an "aryl group" having a carbon number of 6 to 14 which is from among the "aryl group" of (6) "optionally substituted hydrocarbon group" which is the substituent of ring A. Examples of the substituent of the "optionally substituted C₆₋₁₄ aryl group" include those similar to the substituents exemplified as the substituent of the aforementioned "optionally substituted hydrocarbon group".
As the "optionally substituted heterocyclic group" in substituent group A, thienyl, furyl, pyridyl, piperidyl, thiazolyl, isothiazolyl, pyrrolidiyl, perhydroazepinyl, tetrahydrofuryl, oxazolyl, isoxazolyl, pyrimidinyl and pyrazinyl are preferable.
As the "heterocyclic group-oxy group" in substituent group A, pyridyloxy is preferable.
As the "heterocyclic group-carbonyl group" in substituent group A, nicotinoyl, isonicotinoyl, pyrrolidinyl-carbonyl, piperidinyl-carbonyl, morpholinyl-carbonyl, piperazinyl-carbonyl, pyridyl-carbonyl, furyl-carbonyl, thienyl-carbonyl, pyrrolyl-carbonyl, oxazolyl-carbonyl, isoxazolyl-carbonyl, thiazolyl-carbonyl, isothiazolyl-carbonyl, pyrazinyl-carbonyl, quinolyl-carbonyl and isoquinolyl-carbonyl are preferable.
As the "heterocyclic group-sulfonyl group" in substituent group A, pyridylsulfonyl, thienylsulfonyl, pyrrolidinosulfonyl, piperidinosulfonyl, morpholinosulfonyl and piperazinosulfonyl are preferable.
As the "heterocyclic group-C₁₋₆ alkoxy group" in substituent group A, imidazol-1-ylpropyloxy is preferable.

Ring A is preferably an optionally substituted pyridine ring or an optionally substituted benzene ring. As ring A, more preferred includes (1) a pyridine ring optionally substituted by substituent(s) selected from an optionally halogenated C₁₋₆ alkyl group and an optionally substituted aromatic group, (2) a benzene ring optionally substituted by 1 to 3 substituents selected from (i) a halogen atom, (ii) an optionally halogenated C₁₋₆ alkyl group, (iii) a carboxyl group and (iv) a C₁₋₆ alkoxy-carbonyl group. Here, examples of the "optionally substituted aromatic group" include those similar to the groups exemplified as the aforementioned "optionally substituted C₆₋₁₄ aryl group", and those similar to the groups exemplified as (1) "optionally substituted heterocyclic group" which is the substituent of ring A and aromatic. The "optionally substituted aromatic group" is preferably an "optionally substituted C₆₋₁₄ aryl group", more preferably a phenyl group, a phenyl group substituted by 1 to 3, particularly preferably 1 or 2, halogen atoms, and the like.

As ring A, particularly preferred is (1) a pyridine ring substituted by (a) a phenyl group or (b) a phenyl group substituted by one halogen atom (preferably, a phenyl group substituted at the ortho-position relative to ring A), or (2) a benzene ring optionally substituted by one halogen atom.

As the "5- to 9- membered ring" for ring B', for example, a benzene ring, a 5- to 9- membered nonaromatic cyclic hydrocarbon, a 5- to 9-membered aromatic heterocycle, a 5- to 9-membered nonaromatic heterocycle and the like can be mentioned.
Here, as the "5- to 9- membered nonaromatic cyclic hydrocarbon", for example, a C₅₋₉ cycloalkane, a C₅₋₉ cycloalkene, a C₅₋₉ cycloalkadiene and the like can be mentioned.
As specific examples of the C₅₋₉ cycloalkane, cyclopentane, cyclohexane, cycloheptane, cyclooctane, cyclononane and the like can be mentioned.
As specific examples of the C₅₋₉ cycloalkene, cyclopentene, cyclohexene, cycloheptene, cyclooctene, cyclononene and the like can be mentioned.
As specific examples of the C₅₋₉ cycloalkadiene, cyclopenta-1,3-diene, cyclohexa-1,3-diene, cyclohexa-1,4-diene, cyclohepta-1,3-diene, cyclohepta-1,4-diene, cycloocta-1,3-diene, cycloocta-1,4-diene, cycloocta-1,5-diene and the like can be mentioned.
As the "5- to 9-membered aromatic heterocycle", for example, a 5- to 9-membered aromatic heterocycle having, as ring-constituting atom(s) besides a carbon atom, 1 to 4 hetero atoms selected from a nitrogen atom, an oxygen atom and a sulfur atom can be mentioned. As specific examples of the 5- to 9-membered aromatic heterocycle, furan, thiophene, pyrrole, oxazole, isoxazole, thiazole, isothiazole, imidazole, pyrazole, oxadiazole, thiadiazole, triazole, tetrazole, pyridine, pyrazine, pyrimidine, pyridazine, azepine, diazepine, oxazepine, thiazepine, azocine, diazocine, oxazocine, thiazocine, azonine, diazonine, oxazonine, thiazonine and the like can be mentioned.

As the "5- to 9-membered nonaromatic heterocycle", for example, a 5- to 9-membered nonaromatic heterocycle having, as ring-constituting atom(s) besides a carbon atom, 1 to 4 hetero atoms selected from a nitrogen atom, an oxygen atom and a sulfur atom can be mentioned. As specific examples of the 5- to 9-membered nonaromatic heterocycle, tetrahydrofuran, tetrahydrothiophene, pyrrolidine, imidazolidine, pyrazolidine, piperidine, dihydropyridine, dihydropyrazine, tetrahydropyrazine, 2,3-dehydromorpholine, 2,3-dehydrothiomorpholine, tetrahydropyrimidine, tetrahydropyridazine, dihydroazepine, tetrahydroazepine, dihydrodiazepine, tetrahydrodiazepine, dihydro[1,4]oxazepine, 2,3,4,7-tetrahydro[1,4]oxazepine, 4,5,6,7-tetrahydro[1,4]oxazepine, dihydro[1,4]thiazepine, 2,3,4,7-tetrahydro[1,4]thiazepine, 4,5,6,7-tetrahydro[1,4]thiazepine, tetrahydroazocine, hexahydroazocine, tetrahydrodiazocine, hexahydrodiazocine, tetrahydrooxazocine, tetrahydrothiazocine, tetrahydroazonine, hexahydroazonine, tetrahydrodiazonine, hexahydrodiazonine, tetrahydrooxazonine, pentahydrooxazonine, tetrahydrothiazonine, pentahydrothiazonine and the like can be mentioned.

The "5- to 9-membered ring" is preferably a 6- to 9-membered ring, more preferably a 6- to 9-membered nonaromatic heterocycle. Among them, a 6- to 9-membered nonaromatic nitrogen-containing heterocycle containing one or more nitrogen atoms as ring-constituting atom is preferable. When ring A is a benzene ring, ring B' is preferably a 6- to 8-membered nonaromatic nitrogen-containing heterocycle.
As preferable specific examples of the "5- to 9-membered ring", the following rings can be mentioned.

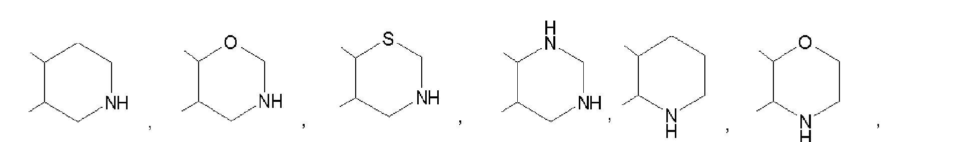

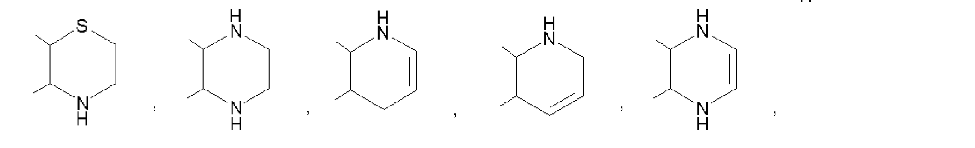

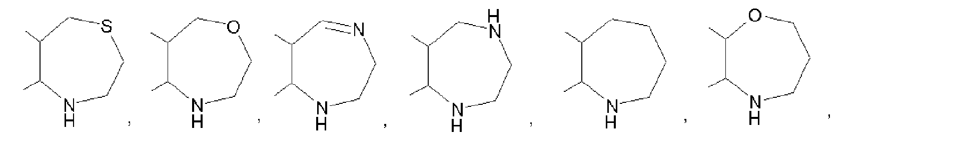

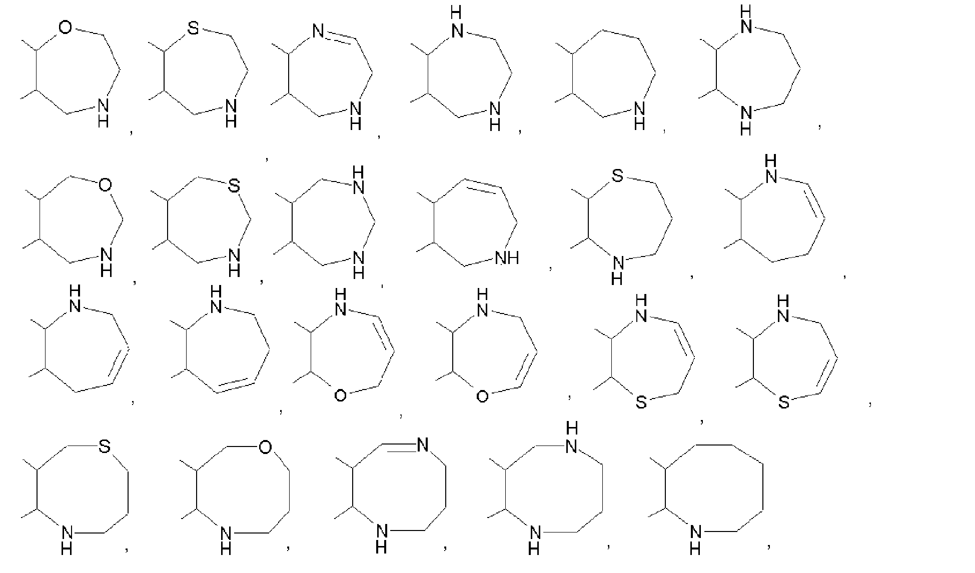

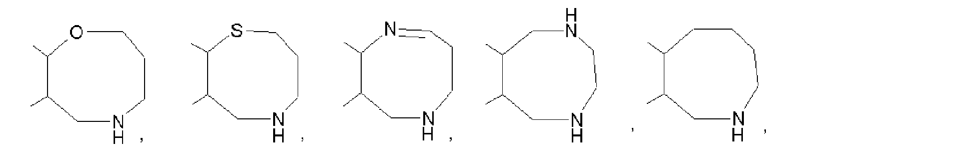

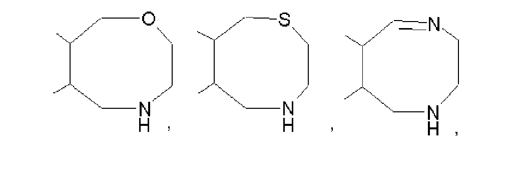

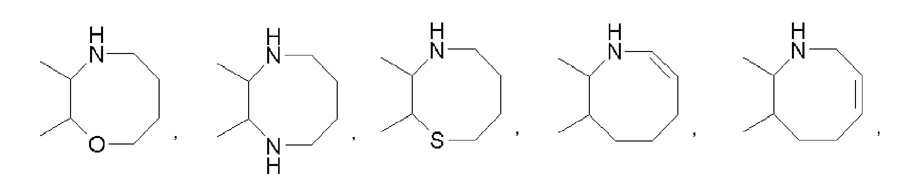

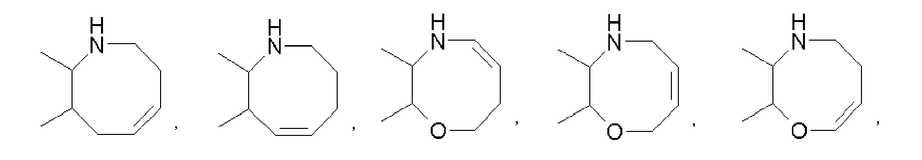

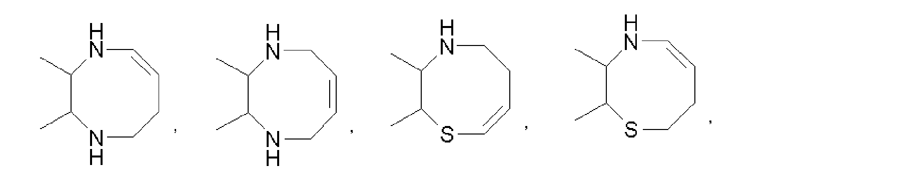

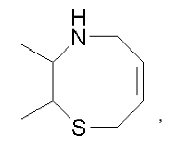

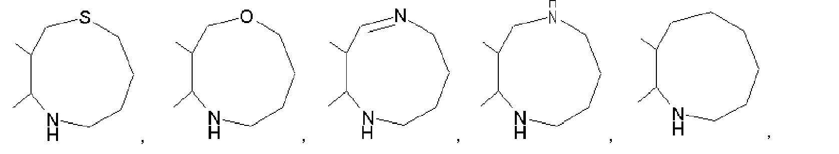

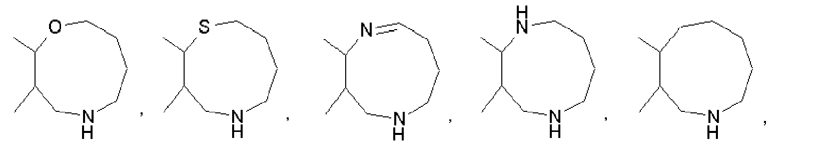

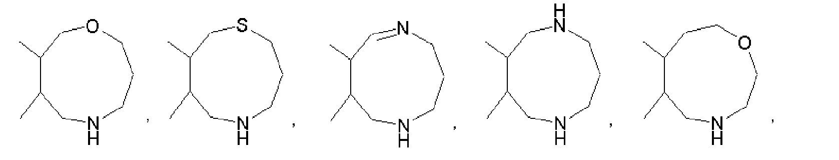

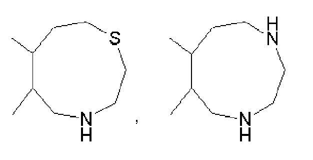

As ring B', particularly preferred are

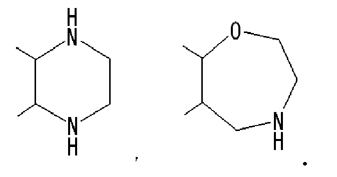

The "5- to 9-membered ring" for ring B' optionally has one or more, preferably 1 to 5, substituents at substitutable positions. Examples of such substituent include a nitro group, an oxo group, a thioxo group, a halogen atom (e.g., fluorine, chlorine, bromine, iodine), a hydroxy group, a cyano group, an optionally halogenated C₁₋₆ alkoxy group (e.g., methoxy, ethoxy, trifluoromethoxy), a C₆₋₁₄ aryloxy group (e.g., phenoxy), a C₇₋₁₄ aralkyloxy group (e.g., benzyloxy, phenethyloxy, phenylpropyloxy), a formyloxy group, a C₁₋₆ alkyl-carbonyloxy group (e.g., acetyloxy), an optionally substituted mercapto group, an optionally substituted heterocyclic group, an optionally substituted hydrocarbon group, a carboxyl group, a formyl group, an optionally substituted C₁₋₆ alkyl-carbonyl group, an optionally substituted C₆₋₁₄ aryl-carbonyl group, an optionally substituted heterocyclic group-carbonyl group, a C₃₋₈ cycloalkyl-carbonyl group (e.g., cyclopropylcarbonyl, cyclobutylcarbonyl, cyclopentylcarbonyl, cyclohexylcarbonyl), an optionally substituted C₇₋₁₄ aralkyl-carbonyl group, a C₁₋₆ alkoxy-carbonyl group (e.g., methoxycarbonyl, ethoxycarbonyl, propoxycarbonyl), a C₆₋₁₄ aryloxy-carbonyl group (e.g., phenoxycarbonyl), a C₇₋₁₄ aralkyloxy-carbonyl group (e.g., benzyloxycarbonyl), an amino group, a mono- or di-C₁₋₆ alkylamino group (e.g., methylamino, ethylamino, dimethylamino, diethylamino), a formylamino group, a C₁₋₆ alkyl-carbonylamino group (e.g., acetylamino, propionylamino, butyrylamino), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), an optionally substituted C₆₋₁₄ aryl-carbamoyl group, an optionally substituted heterocyclic group-carbamoyl group, a C₃₋₈ cycloalkyl-carbamoyl group (e.g., cyclopropylcarbamoyl, cyclobutylcarbamoyl, cyclopentylcarbamoyl, cyclohexylcarbamoyl), a C₇₋₁₄ aralkyl-carbamoyl group (e.g., benzylcarbamoyl), a sulfo group, a C₁₋₆ alkylsulfonyl group (e.g., methylsulfonyl, ethylsulfonyl, propylsulfonyl, sec-propylsulfonyl, butylsulfonyl, tert-butylsulfonyl), an optionally substituted C₆₋₁₄ arylsulfonyl group, a heterocyclic group-sulfonyl group, a C₃₋₈ cycloalkylsulfonyl group (e.g., cyclopropylsulfonyl, cyclobutylsulfonyl, cyclopentylsulfonyl, cyclohexylsulfonyl), a C₇₋₁₄ aralkylsulfonyl group (e.g., benzylsulfonyl), a sulfamoyl group, a mono- or di-C₁₋₆ alkyl-sulfamoyl group (e.g., N-methylsulfamoyl, N-ethylsulfamoyl, N,N-dimethylsulfamoyl, N,N-diethylsulfamoyl), a C₆₋₁₄ arylsulfamoyl group (e.g., phenylsulfamoyl, naphthylsulfamoyl), a heterocyclic group-sulfamoyl group, a C₃₋₈ cycloalkylsulfamoyl group (e.g., cyclopropylsulfamoyl, cyclobutylsulfamoyl, cyclopentylsulfamoyl, cyclohexylsulfamoyl), a C₇₋₁₄ aralkylsulfamoyl group (e.g., benzylsulfamoyl) (hereinafter to be also referred to as substituent group B) and the like. The number of substituents is, for example, 1 to 5.

Examples of the "optionally substituted heterocyclic group", "optionally substituted hydrocarbon group" and "optionally substituted mercapto group" in substituent group B each include those similar to the groups exemplified as the substituent of ring A.

Examples of the substituent of the "optionally substituted C₁₋₆ alkyl-carbonyl group" in substituent group B include an optionally substituted heterocyclic group, a heterocyclic group-oxy group, an optionally substituted C₆₋₁₄ aryloxy group (e.g., phenoxy, naphthyloxy, 5,6,7,8-tetrahydro-2-naphthyloxy, 2,3-dihydro-4-indanyloxy, 5,6,7,8,9-pentahydro-4-benzo-cycloheptyloxy), an optionally substituted C₆₋₁₄ arylthio group (e.g., phenylthio, naphthylthio), an optionally substituted heterocyclic group-thio group and an optionally substituted C₆₋₁₄ arylsulfonyl group (e.g., 3,5-dimethylphenylsulfonyl, 3,5-bis(trifluoromethyl)phenylsulfonyl).
Examples of the "optionally substituted heterocyclic group" include those similar to the groups exemplified as the substituent of ring A. Examples of the "optionally substituted heterocycle" of the "optionally substituted heterocyclic group-thio group" include those similar to the groups exemplified as the substituent of ring A.
Examples of the "heterocyclic group" of the "heterocyclic group-oxy group" include those similar to the groups exemplified as the "heterocyclic group" of (1) "optionally substituted heterocyclic group" recited as the substituent of ring A.
Examples of the substituent of the "optionally substituted C₆₋₁₄ aryloxy group" include a halogen atom (e.g., fluorine, chlorine, bromine, iodine), a cyano group, an oxo group, a hydroxy group, an optionally substituted carbamoyl group (e.g., carbamoyl, dimethylcarbamoyl), an optionally substituted imino group (e.g., imino, hydroxyimino, methoxyimino), an optionally substituted amino group (e.g., acetylamino), a C₁₋₆ alkyl group optionally substituted by halogen (e.g., methyl, ethyl, tert-butyl, trifluoromethyl), a C₁₋₆ alkoxy group (e.g., methoxy, ethoxy), a C₇₋₁₄ aralkyloxy group (e.g., benzyloxy), a C₆₋₁₄ aryl group (e.g., phenyl, naphthyl), a C₁₋₆ alkyl-carbonyl group (e.g., acetyl, propionyl), a C₆₋₁₄ aryl-carbonyl group (e.g., benzoyl), a C₁₋₆ alkylthio group (e.g., methylthio), a C₁₋₆ alkoxy-carbonyl group (e.g., methoxycarbonyl), a hydroxycarbonyl group, a heterocyclic group-carbonyl group and a heterocyclic group. The number of substituents is, for example, 1 to 3.
Examples of the "heterocyclic group" of the "heterocyclic group" and the "heterocyclic group-carbonyl group" include those similar to the groups exemplified as the "heterocyclic group" of (1) "optionally substituted heterocyclic group" recited as the substituent of ring A.
Examples of the substituent of the "optionally substituted carbamoyl group" include a halogen atom (e.g., fluorine, chlorine, bromine, iodine), an optionally halogenated C₁₋₆ alkyl group (e.g., fluorine, chlorine, bromine, iodine), and a C₁₋₆ alkoxy group (e.g., methoxy, ethoxy). The number of substituents is, for example, 1 or 2. Examples of the substituent of the "optionally substituted imino group" include a hydroxy group, an optionally halogenated C₁₋₆ alkyl group (e.g., methyl, trifluoromethyl), and a C₁₋₆ alkoxy group (e.g., methoxy, ethoxy). The number of substituents is, for example, 1 or 2.
Examples of the substituent of the "optionally substituted amino group" include an optionally halogenated C₁₋₆ alkyl group (e.g., methyl, ethyl, trifluoromethyl), a C₁₋₆ alkoxy group (e.g., methoxy, ethoxy), and a C₁₋₆ alkyl-carbonyl group (e.g., acetyl group, ethylcarbonyl group). The number of substituents is, for example, 1 or 2.
Examples of the substituent of the "optionally substituted C₆₋₁₄ arylthio group" include a halogen atom (e.g., fluorine, chlorine, bromine, iodine), an optionally halogenated C₁₋₆ alkyl group (e.g., methyl, trifluoromethyl), and a C₁₋₆ alkoxy group (e.g., methoxy, ethoxy). The number of substituents is, for example, 1 or 2.
Examples of the substituent of the "optionally substituted C₆₋₁₄ arylsulfonyl group" include a halogen atom (e.g., fluorine, chlorine, bromine, iodine), and an optionally halogenated C₁₋₆ alkyl group (e.g., methyl, trifluoromethyl). The number of substituents is, for example, 1 or 2.

Examples of the substituent of the "optionally substituted C₆₋₁₄ aryl-carbonyl group" in substituent group B include a halogen atom (e.g., fluorine, chlorine, bromine, iodine), a nitro group, an optionally substituted C₁₋₆ alkyl group (e.g., methyl, trifluoromethyl, tert-butoxycarbonylaminomethyl, aminomethyl), a C₁₋₆ alkoxy group (e.g., methoxy, ethoxy), a C₁₋₆ alkyl-carbonylamino group (e.g., acetylamino), and a cyano group. The number of substituents is, for example, 1 or 2.

Examples of the substituent of the "optionally substituted heterocyclic group-carbonyl group" in substituent group B include a halogen atom (e.g., fluorine, chlorine, bromine, iodine), a C₁₋₆ alkyl group (e.g., methyl, ethyl), a C₁₋₆ alkyl-carbonyl group (e.g., acetyl, propionyl), a C₁₋₆ alkyl-carbonylamino group (e.g., acetylamino), a hydroxy group, a carbamoyl group, and a cyano group. The number of substituents is, for example, 1 or 2. Examples of the "heterocyclic group" of the above-mentioned "optionally substituted heterocyclic group-carbonyl group" include those similar to the groups exemplified as the "heterocyclic group" of (1) "optionally substituted heterocyclic group" recited as the substituent of ring A.

Examples of the substituent of the "optionally substituted C₇₋₁₄ aralkyl-carbonyl group" in substituent group B include an optionally halogenated C₁₋₆ alkyl group (e.g., methyl, trifluoromethyl). The number of substituents is, for example, 1 or 2.

Examples of the substituent of the "optionally substituted C₆₋₁₄ aryl-carbamoyl group" in substituent group B include an optionally halogenated C₁₋₆ alkyl group (e.g., methyl, trifluoromethyl). The number of substituents is, for example, 1 or 2.

Examples of the substituent of the "optionally substituted heterocyclic group-carbamoyl group" in substituent group B include a halogen atom (e.g., fluorine, chlorine, bromine, iodine), a C₁₋₆ alkyl group (e.g., methyl, ethyl), a C₁₋₆ alkyl-carbonyl group (e.g., acetyl, propionyl), a C₁₋₆ alkyl-carbonylamino group (e.g., acetylamino), a hydroxy group, a carbamoyl group and a cyano group. The number of substituents is, for example, 1 or 2.

Examples of the substituent of the "optionally substituted C₆₋₁₄ arylsulfonyl group" in substituent group B include a halogen atom (e.g., fluorine, chlorine, bromine, iodine), and an optionally halogenated C₁₋₆ alkyl group (e.g., methyl, trifluoromethyl). The number of substituents is, for example, 1 or 2.
Examples of the heterocycle of the "heterocyclic group-sulfonyl group" and "heterocyclic group-sulfamoyl group" in substituent group B include those similar to the heterocycles exemplified as the heterocycle of (1) "optionally substituted heterocycle" recited as the substituent of ring A.
As the "optionally substituted C₁₋₆ alkyl-carbonyl group" in substituent group B, acetyl, propionyl, butyryl or valeryl is preferable.
As the "optionally substituted C₆₋₁₄ aryl-carbonyl group" in substituent group B, benzoyl or naphthylcarbonyl is preferable.
As the "optionally substituted heterocyclic group-carbonyl group" in substituent group B, nicotinoyl, isonicotinoyl, pyrrolidinyl-carbonyl, piperidinyl-carbonyl, morpholinyl-carbonyl, piperazinyl-carbonyl, pyridyl-carbonyl, furyl-carbonyl, thienyl-carbonyl, pyrrolyl-carbonyl, oxazolyl-carbonyl, isoxazolyl-carbonyl, thiazolyl-carbonyl, isothiazolyl-carbonyl, pyrazinyl-carbonyl, quinolyl-carbonyl or isoquinolyl-carbonyl is preferable.
As the "optionally substituted C₇₋₁₄ aralkyl-carbonyl group" in substituent group B, benzylcarbonyl, or 3,5-bis(trifluoromethyl)phenethylcarbonyl is preferable.
As the "optionally substituted C₆₋₁₄ aryl-carbamoyl group" in substituent group B, phenylcarbamoyl or naphthylcarbamoyl is preferable.
As the "optionally substituted heterocyclic group-carbamoyl group" in substituent group B, pyridylcarbamoyl, thienylcarbamoyl, pyrrolidinocarbamoyl, piperidinocarbamoyl, morpholinocarbamoyl or piperazinocarbamoyl is preferable.
As the "optionally substituted C₆₋₁₄ aryl-sulfonyl group" in substituent group B, phenylsulfonyl or naphthylsulfonyl is preferable.
As the "heterocyclic group-sulfonyl group" in substituent group B, pyridylsulfonyl, thienylsulfonyl, pyrrolidinosulfonyl, piperidinosulfonyl, morpholinosulfonyl, piperazinosulfonyl or quinolylsulfonyl is preferable.
As the "heterocyclic group-sulfamoyl group" in substituent group B, pyridylsulfamoyl, thienylsulfamoyl, pyrrolidinosulfamoyl, piperidinosulfamoyl, morpholinosulfamoyl or piperazinosulfamoyl is preferable.

Particularly preferable examples of the substituent for ring B' include a halogen atom, an oxo group, an optionally substituted hydrocarbon group and an optionally substituted C₁₋₆ alkyl-carbonyl group (e.g., acetyl).

Examples of the "hydrocarbon group" of the "optionally substituted hydrocarbon group" which can be recited as a particularly preferable substituent of ring B' include a C₁₋₆ alkyl group (e.g., methyl) and an optionally substituted C₇₋₁₄ aralkyl group (e.g., benzyl). As the substituent, an optionally halogenated C₁₋₆ alkyl group (e.g., methyl, trifluoromethyl) is preferable.
The particularly preferable substituent of the "optionally substituted C₁₋₆ alkyl-carbonyl group" which can be recited as a particularly preferable substituent of ring B' is a heterocyclic group-oxy group (e.g., 5-isoquinolyl-oxy).

The particularly preferable specific example of ring B':

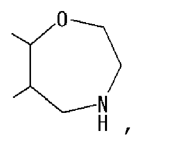

preferably has 1 or 2 substituents selected from (a) a C₇₋₁₄ aralkyl group (e.g., benzyl) optionally having 1 to 3 substituents selected from an optionally halogenated C₁₋₆ alkyl group (e.g., methyl, trifluoromethyl) and (b) an oxo group.

The particularly preferable specific example of ring B':

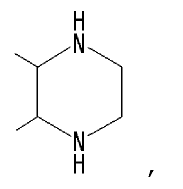

preferably has 4 substituents selected from (a) a C₁₋₆ alkyl group (e.g., methyl), (b) an oxo group, and (c) a C₁₋₆ alkyl-carbonyl group (e.g., acetyl) substituted by a heterocyclic group-oxy group (e.g., 5-isoquinolyl-oxy).

Compound (I) preferably has one or more (preferably 1 to 4, particularly preferably 1 or 2) "substituents having a cyclic group". The substituents may be present on either one of ring A and ring B', or both ring A and ring B'. The two or more "substituents having a cyclic group" that compound (I) has may be the same as or different from each other.
The "substituent having a cyclic group" means a substituent having a cyclic group such as a C₃₋₈ cycloalkyl group, a C₆₋₁₄ aryl group, a heterocyclic group and the like as a constituent element. Concrete examples thereof include "C₃₋₈ cycloalkyl group", "C₇₋₁₄ aralkyl group", "C₆₋₁₄ aryloxy group", "heterocyclic group-oxy group", "C₇₋₁₄ aralkyloxy group", "C₆₋₁₄ aryloxy-carbonyl group", "C₆₋₁₄ aryl-carbonyl-C₁₋₆ alkoxy group", "C₃₋₁₄ cycloalkyl-C₁₋₆ alkoxy group", "heterocyclic group-C₁₋₆ alkoxy group", "C₇₋₁₄ aralkyloxy-carbonyl-C₁₋₆ alkoxy group", "hydroxyphenyl-C₁₋₆ alkoxy group", "C₇₋₁₄ aralkyloxy-carbonyl group", "optionally substituted heterocyclic group", "optionally substituted hydrocarbon group (only that having cyclic group such as C₃₋₈ cycloalkyl group, C₆₋₁₄ aryl group, heterocyclic group and the like as a constituent element)", "optionally substituted C₆₋₁₄ aryl-carbonyl group", "optionally substituted heterocyclic group-carbonyl group", "C₃₋₈ cycloalkyl-carbonyl group", "optionally substituted C₇₋₁₄ aralkyl-carbonyl group", "optionally substituted C₆₋₁₄ aryl-carbamoyl group", "optionally substituted heterocyclic group-carbamoyl group", "C₃₋₈ cycloalkyl-carbamoyl group", "C₇₋₁₄ aralkyl-carbamoyl group", "optionally substituted C₆₋₁₄ arylsulfonyl group", "heterocyclic group-sulfonyl group", "C₃₋₈ cycloalkylsulfonyl group", "C₇₋₁₄ aralkylsulfonyl group", "C₆₋₁₄ arylsulfamoyl group", "heterocyclic group-sulfamoyl group", "C₃₋₈ cycloalkylsulfamoyl group", "C₇₋₁₄ aralkylsulfamoyl group" and the like, which are exemplified as the substituent group A and/or substituent group B.

Compound (I) is preferably a fused ring compound represented by the formula

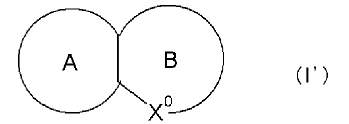

wherein ring A is an optionally substituted aromatic ring; ring B is a 6- to 8-membered ring having 3 or more substituents; X⁰ is - C(R¹)=, -CH(R¹)-, -N(R¹)- or -N=; R¹ is a hydrogen atom or a substituent (hereinafter sometimes to be abbreviated as compound (I')), or a salt thereof.

As the "6- to 8-membered ring having 3 or more substituents" for ring B, a 6- to 8-membered ring having 3 or more substituents from among the "5- to 9-membered ring having one or more substituents" for the aforementioned ring B', can be mentioned.

X⁰ is -C(R¹)=, -CH(R¹)-, -N(R¹)- or -N=, preferably -N(R¹)-.
R¹ is a hydrogen atom or a substituent, preferably a substituent. Examples of the substituent include those exemplified as substituent group B. Among them, an optionally substituted hydrocarbon group, an optionally substituted heterocyclic group and the like are preferable.
R¹ is preferably an optionally substituted hydrocarbon group, more preferably an optionally substituted C₁₋₆ alkyl group. Specifically, a C₁₋₆ alkyl group is preferable and neopentyl is particularly preferable.
Here, examples of the "optionally substituted hydrocarbon group" include those similar to the groups exemplified as the substituent of ring A. Examples of the "optionally substituted C₁₋₆ alkyl group" include those similar to (i) "optionally substituted C₁₋₆ alkyl group" exemplified as the substituent of (2) "optionally substituted hydroxy group" which is the substituent of ring A.
When R¹ is a substituent, the substituent is counted as a substituent for ring B. The number of the substituents for ring B is preferably 4.

Compound (I) is more preferably a fused ring compound represented by the formula

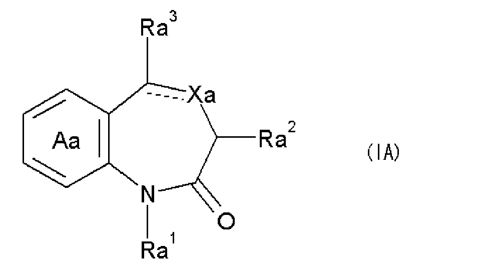

wherein ring Aa is an optionally substituted benzene ring; Xa is =N-, -NR⁶- (R⁶ is a hydrogen atom or a substituent), -O- or -S(O)n-(n is 0, 1 or 2);

is a single bond or double bond; Ra¹ and Ra³ are each independently a hydrogen atom, an optionally substituted hydrocarbon group or an optionally substituted heterocyclic group; and Ra² is an optionally substituted hydrocarbon group or an optionally substituted heterocyclic group (hereinafter sometimes to be abbreviated as compound (IA)), or a salt thereof.

Here, the benzene ring for ring Aa may have 1 to 4 substituents at substitutable positions, and as such substituent, those exemplified as substituent group A can be used. The substituent of ring Aa is preferably a halogen atom (preferably a chlorine atom).

As the substituent for R⁶, those exemplified as substituent group B can be used.
R⁶ is preferably 1) an optionally substituted C₆₋₁₄ aryl-carbonyl group, an optionally substituted heterocyclic group-carbonyl group, a C₃₋₈ cycloalkyl-carbonyl group, an optionally substituted C₇₋₁₄ aralkyl-carbonyl group, a C₆₋₁₄ aryloxy-carbonyl group, a C₇₋₁₄ aralkyloxy-carbonyl group, an optionally substituted C₆₋₁₄ aryl-carbamoyl group, an optionally substituted heterocyclic group-carbamoyl group, a C₃₋₈ cycloalkyl-carbamoyl group, a C₇₋₁₄ aralkyl-carbamoyl group, an optionally substituted C₆₋₁₄ arylsulfonyl group, an optionally substituted heterocyclic group-sulfonyl group,
a C₃₋₈ cycloalkylsulfonyl group, a C₇₋₁₄ aralkylsulfonyl group, a C₆₋₁₄ arylsulfamoyl group, a heterocyclic group-sulfamoyl group, a C₃₋₈ cycloalkylsulfamoyl group or a C₇₋₁₄ aralkylsulfamoyl group [each is as defined for those exemplified as substituent group B]; 2) a C₁₋₁₀ alkyl group (e.g., methyl, ethyl, propyl, isopropyl, butyl, isobutyl, sec-butyl, tert-butyl, pentyl, isopentyl, neopentyl, 1-methylpropyl, hexyl, isohexyl, 1,1-dimethylbutyl, 2,2-dimethylbutyl, 3,3-dimethylbutyl, 3,3-dimethylpropyl, 2-ethylbutyl, heptyl) substituted by a C₇₋₁₄ aralkyl group (e.g., benzyl, phenethyl, phenylpropyl) or an optionally substituted heterocyclic group (as defined for those exemplified as substituent group B) and the like. Among them, an optionally substituted C₆₋₁₄ aryl-carbonyl group, an optionally substituted heterocyclic group-carbonyl group and the like are preferable.

Specifically preferable examples of R⁶ include a C₆₋₁₄ aryl-carbonyl group (preferably benzoyl) and a heterocyclic group-carbonyl group (e.g., nicotinoyl, isonicotinoyl, pyrrolidinyl-carbonyl, piperidinyl-carbonyl, morpholinyl-carbonyl, piperazinyl-carbonyl, pyridyl-carbonyl, furyl-carbonyl, thienyl-carbonyl, pyrrolyl-carbonyl, oxazolyl-carbonyl, isoxazolyl-carbonyl, thiazolyl-carbonyl, isothiazolyl-carbonyl, pyrazinyl-carbonyl, quinolyl-carbonyl, isoquinolyl-carbonyl), which are optionally substituted by 1 to 4 substituents selected from a halogen atom, an optionally halogenated C₁₋₆ alkyl group (e.g., methyl, ethyl, trifluoromethyl), an optionally halogenated C₁₋₆ alkoxy group (e.g., methoxy, ethoxy, trifluoromethoxy), an optionally halogenated C₁₋₆ alkylthio group (e.g., methylthio, ethylthio, trifluoromethylthio), a hydroxy group, a mercapto group, a cyano group, a nitro group, a carboxyl group, a carbamoyl group, a formyl group, an optionally halogenated C₁₋₆ alkyl-carbonyl group (e.g., acetyl, propionyl, butyryl, tert-butylcarbonyl, trifluoroacetyl), a C₁₋₆ alkoxy-carbonyl group (e.g., methoxycarbonyl, ethoxycarbonyl, propoxycarbonyl, sec-propoxycarbonyl, butoxycarbonyl, tert-butoxycarbonyl), an amino group, a mono- or di-C₁₋₆ alkylamino group (e.g., methylamino, ethylamino, dimethylamino, diethylamino), a formylamino group, an optionally halogenated C₁₋₆ alkyl-carbonylamino group (e.g., acetylamino, propionylamino, butyrylamino) and the like.
Xa is preferably -O-, -S(O)n- or -NR⁶-, more preferably -O- or -NR⁶-.

As the "optionally substituted hydrocarbon group" and "optionally substituted heterocyclic group" for each of Ra¹, Ra³ and Ra², those similar to the groups exemplified as the substituent of ring A can be used. Here, as the "optionally substituted hydrocarbon group", an optionally substituted C₁₋₆ alkyl group, an optionally substituted C₂₋₆ alkenyl group, an optionally substituted phenyl group, an optionally substituted aralkyl group (preferably a C₇₋₁₄ aralkyl group) and the like are preferable.

Ra¹ is preferably an optionally substituted hydrocarbon group, more preferably an optionally substituted C₁₋₆ alkyl group. Particularly, a C₁₋₆ alkyl group optionally substituted by substituent(s) selected from 1) a heterocyclic group (preferably furyl, thienyl, quinolyl) optionally substituted by substituent(s) selected from a heterocyclic group (preferably furyl, thienyl) and a phenyl group optionally substituted by a C₁₋₆ alkoxy group, 2) a hydroxy group, 3) a C₁₋₆ alkyl-carbonyloxy group (e.g., acetyloxy) and 4) a C₁₋₆ alkylsulfonyloxy group (e.g., methylsulfonyloxy) is preferable. Examples of the C₁₋₆ alkyl group include methyl, ethyl, propyl, isopropyl, butyl, isobutyl, sec-butyl, tert-butyl, pentyl, isopentyl, neopentyl, hexyl and the like. Ra¹ is particularly preferably a C₁₋₆ alkyl group, specifically preferably neopentyl.

In the formula (IA), when Xa is -O-, =N- or -S(O)n-, Ra³ is preferably an optionally substituted C₆₋₁₄ aryl group or an optionally substituted heterocyclic group. Here, examples of the "C₆₋₁₄ aryl group" of the "optionally substituted C₆₋₁₄ aryl group" include an "aryl group" having a carbon number of 6 to 14 which is from among the "aryl group" of (6) "optionally substituted hydrocarbon group" which is the substituent for the aforementioned ring A. Examples of the substituent of the "optionally substituted C₆₋₁₄ aryl group" include those similar to the substituents exemplified as the substituent of the aforementioned "optionally substituted hydrocarbon group". Here, examples of the "optionally substituted heterocyclic group" include those similar to the groups exemplified as the substituent of ring A. Ra³ is more preferably an optionally substituted phenyl group or an optionally substituted piperidinyl group. As the substituent on the phenyl group, 1) a C₁₋₆ alkyl group optionally substituted by substituent(s) selected from a halogen atom, an optionally substituted amino group, an optionally substituted hydroxy group and an optionally substituted heterocyclic group, 2) an optionally substituted amino group, 3) an optionally substituted heterocyclic group, 4) an optionally substituted hydroxy group, 5) an acyl group and the like are preferable. Of these, a phenyl group having substituent(s) at the meta-position is preferable. Examples of the substituent of the "optionally substituted piperidinyl group" include those similar to the substituents recited as the substituent of the "optionally substituted heterocyclic group" which is exemplified as the substituent of ring A.
Here, as each of the "optionally substituted amino group", "optionally substituted hydroxy group" and "optionally substituted heterocyclic group", those similar to the groups exemplified as the substituent of ring A are used. Examples of the "acyl group" include those similar to (iv) "acyl group" exemplified as the substituent of (2) "optionally substituted hydroxy group" recited as the substituent of ring A.

Specifically preferable examples of the aforementioned substituent on the phenyl group include
1) a C₁₋₆ alkyl group optionally substituted by substituent(s) selected from a halogen atom (preferably, fluorine, chlorine), the "optionally substituted amino group" [same as those exemplified as the substituent of ring A], an optionally substituted hydroxy group (preferably a hydroxy group, a carboxyl-C₁₋₆ alkoxy group, a C₁₋₆ alkoxy-carbonyl-C₁₋₆ alkoxy group), and the "optionally substituted heterocyclic group" [same as those exemplified as the substituent of ring A],
2) an amino group,
3) a heterocyclic group (preferably dioxolanyl),
4) an optionally substituted C₁₋₆ alkoxy group, and
5) an acyl group (preferably formyl).
Examples of the substituent of the "optionally substituted C₁₋₆ alkoxy group" include those similar to the substituents exemplified as the substituent of the "optionally substituted hydrocarbon group" which is exemplified as the substituent of ring A.
The aforementioned substituent on the phenyl group is more preferably (1) a C₁₋₆ alkyl group optionally substituted by an acylamino group or a heterocyclic group (preferably an aromatic nitrogen-containing heterocyclic group (e.g., pyrrolyl, oxazolyl, isoxazolyl, thiazolyl, isothiazolyl, imidazolyl, pyrazolyl, oxadiazolyl, furazanyl, thiadiazolyl, triazolyl, tetrazolyl, pyridyl, pyridazinyl, pyrimidinyl, pyrazinyl)), or (2) a C₁₋₆ alkoxy group optionally substituted by substituent(s) selected from a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), a mono- or di-(heterocyclic group (preferably imidazolyl)-C₁₋₆ alkyl)-carbamoyl group (e.g., imidazolylpropylcarbamoyl) and a mono- or di-C₇₋₁₄ aralkyl-carbamoyl group (e.g., benzylcarbamoyl, phenethylcarbamoyl). As the aforementioned substituent on the phenyl group, an acylaminomethyl group is particularly preferable.

As the aforementioned "acylamino group", formylamino, a C₁₋₆ alkyl-carbonylamino group (e.g., acetylamino, propionylamino, butyrylamino, tert-butylcarbonylamino), a C₆₋₁₄ aryl-carbonylamino group (e.g., benzoylamino), a C₁₋₆ alkoxy-carbonylamino group (e.g., methoxycarbonylamino, ethoxycarbonylamino, propoxycarbonylamino, sec-propoxycarbonylamino, butoxycarbonylamino, tert-butoxycarbonylamino), a C₇₋₁₄ aralkyloxy-carbonylamino group (e.g., benzyloxycarbonylamino), a C₁₋₆ alkylsulfonylamino group (e.g., methylsulfonylamino, ethylsulfonylamino, propylsulfonylamino, sec-propylsulfonylamino, butylsulfonylamino, tert-butylsulfonylamino), a carbamoylamino group, a mono- or di-C₁₋₆ alkyl-carbamoylamino group (e.g., N-methylcarbamoylamino, N-ethylcarbamoylamino, N,N-dimethylcarbamoylamino, N,N-diethylcarbamoylamino) and the like, each of which may have 1 to 3 substituents selected from a halogen atom (e.g., fluorine, chlorine, bromine, iodine), a nitro group, a cyano group, a C₁₋₆ alkyl group, a mono- or di-C₁₋₆ alkylamino group (e.g., methylamino, dimethylamino, ethylamino), a hydroxy group, a C₁₋₆ alkoxy group (e.g., methoxy, ethoxy, propoxy, isopropoxy) and the like, are preferable. Among them, formylamino, a C₁₋₃ alkyl-carbonylamino group, a C₁₋₃ alkoxy-carbonylamino group and the like are preferable.

Ra³ is particularly preferably a phenyl group having an acylaminomethyl group (preferably, formylaminomethyl, C₁₋₃ alkyl-carbonylaminomethyl, C₁₋₃ alkoxy-carbonylaminomethyl) at the meta-position.
In the formula (IA), when Xa is -NR⁶-, moreover, Ra³ is preferably a hydrogen atom.

Ra² is preferably an "optionally substituted hydrocarbon group", more preferably a C₁₋₆ alkyl group substituted by an optionally substituted carbamoyl group (that is, -CON(R⁴) (R⁵) wherein R⁴ is a hydrogen atom or an optionally substituted C₁₋₆ alkyl group, R⁵ is a hydrogen atom, an optionally substituted hydrocarbon group, an optionally substituted heterocyclic group, an optionally substituted amino group, an optionally substituted hydroxy group or an optionally substituted sulfonyl group, and R⁴ and R⁵ may be bonded to each other to form, together with the adjacent nitrogen atom, an optionally substituted nitrogen-containing heterocycle.)

The "optionally substituted C₁₋₆ alkyl group" for R⁴ is as defined for (i) "optionally substituted C₁₋₆ alkyl group" exemplified as the substituent of (2) "optionally substituted hydroxy group" which is the substituent of ring A. As the "optionally substituted C₁₋₆ alkyl group" for R⁴, a C₁₋₆ alkyl group is preferable.

Examples of the "optionally substituted hydrocarbon group", "optionally substituted heterocyclic group", "optionally substituted amino group" and "optionally substituted hydroxy group" for R⁵ each include those similar to the groups exemplified as the substituent of ring A.
Examples of the "optionally substituted sulfonyl group" for R⁵ include an optionally substituted C₆₋₁₀ arylsulfonyl group (e.g., phenylsulfonyl, toluenesulfonyl), an optionally halogenated C₁₋₆ alkylsulfonyl group (e.g., methylsulfonyl, ethylsulfonyl, propylsulfonyl, sec-propylsulfonyl, butylsulfonyl, tert-butylsulfonyl, trifluoromethanesulfonyl) and the like.
Here, examples of the substituent of the "optionally substituted C₆₋₁₀ arylsulfonyl group" include a halogen atom, a nitro group, a cyano group, an optionally halogenated C₁₋₆ alkyl group (e.g., methyl, ethyl, trifluoromethyl), an optionally halogenated C₁₋₆ alkoxy group (e.g., methoxy, ethoxy, trifluoromethoxy), an optionally substituted C₆₋₁₄ aryl group, a heterocyclic group and the like.
Examples of the "C₆₋₁₄ aryl group" of the "optionally substituted C₆₋₁₄ aryl group" include phenyl, naphthyl, anthryl, phenanthryl, acenaphthyl, indenyl and the like. The aryl group may be partially saturated, and examples of the partially saturated aryl group include indanyl, dihydronaphthyl, tetrahydronaphthyl and the like. Examples of the substituent of the "optionally substituted C₆₋₁₄ aryl group" include those similar to the substituents recited as the substituent of (6) "optionally substituted hydrocarbon group" which is exemplified as the substituent of ring A.
Examples of the "heterocyclic group" include those similar to the "heterocyclic group" of (1) "optionally substituted heterocyclic group" which is exemplified as the substituent of ring A.
In addition, the number of substituents is, for example, 1 to 3.

Examples of the "nitrogen-containing heterocycle" of the "optionally substituted nitrogen-containing heterocycle" formed, together with the adjacent nitrogen atom, by R⁴ and R⁵ bonded to each other include a 3- to 8-membered nitrogen-containing heterocycle containing at least one nitrogen atom as a ring-constituting atom besides carbon atom, and optionally further containing 1 to 3 hetero atoms selected from a nitrogen atom, a sulfur atom and an oxygen atom. Specific examples of such nitrogen-containing heterocycle include monocyclic heterocycles such as aziridine, azetidine, morpholine, thiomorpholine, piperidine, piperazine, pyrrolidine, azepane, azocane, hexahydropyrimidine, 1,4-diazepane and the like; and bicyclic heterocycles such as indoline, tetrahydroquinoline, tetrahydroisoquinoline, benzoxazin, benzoazepane, benzooxazepane and the like.
The "nitrogen-containing heterocycle" may have 1 to 4 substituents at substitutable positions, and as such substituent, those exemplified as the substituent of ring A (substituent group A) are used.

R⁴ is preferably a hydrogen atom.
R⁵ is preferably an optionally substituted C₁₋₆ alkyl group, an optionally substituted aralkyl group, an optionally substituted C₃₋₁₀ cycloalkyl-C₁₋₆ alkyl group, an optionally substituted phenyl group, an optionally substituted C₃₋₁₀ cycloalkyl group or an optionally substituted heterocyclic group.
The "optionally substituted C₁₋₆ alkyl group" is as defined for the "optionally substituted C₁₋₆ alkyl group" for R⁴.
The "aralkyl group" of the "optionally substituted aralkyl group" is as defined for the "aralkyl group" exemplified as the "hydrocarbon group" of (6) "optionally substituted hydrocarbon group" recited as the substituent of ring A. Preferably, a C₇₋₁₄ aralkyl group (e.g., benzyl, 1-phenethyl, 2-phenethyl, 2-phenylpropyl, 3-phenylpropyl, 4-phenylbutyl, 2-naphthylmethyl, benzhydryl) can be mentioned. Examples of the substituent of the "optionally substituted aralkyl group" include those similar to the substituents exemplified as the substituent of the aforementioned "optionally substituted hydrocarbon group".
The "C₃₋₁₀ cycloalkyl-C₁₋₆ alkyl group" of the "optionally substituted C₃₋₁₀ cycloalkyl-C₁₋₆ alkyl group" is as defined for the "C₃₋₁₀ cycloalkyl-C₁₋₆ alkyl group" exemplified as the "hydrocarbon group" of (6) "optionally substituted hydrocarbon group" recited as the substituent of ring A. Examples of the substituent of the "optionally substituted C₃₋₁₀ cycloalkyl-C₁₋₆ alkyl group" include those similar to the substituents exemplified as the substituent of the aforementioned "optionally substituted hydrocarbon group".
Examples of the substituent of the "optionally substituted phenyl group" include those similar to the substituents exemplified as the substituent of (6) "optionally substituted hydrocarbon group" recited as the substituent of ring A.
The "C₃₋₁₀ cycloalkyl group" of the "optionally substituted C₃₋₁₀ cycloalkyl group" is as defined for the "C₃₋₁₀ cycloalkyl group" exemplified as the "hydrocarbon group" of (6) "optionally substituted hydrocarbon group" recited as the substituent of ring A. Examples of the substituent of the "optionally substituted C₃₋₁₀ cycloalkyl group" include those similar to the substituents exemplified as the substituent of the aforementioned "optionally substituted hydrocarbon group".
Examples of the "optionally substituted heterocyclic group" include those similar to the groups exemplified as the substituent of ring A.

Compound (IA) is preferably a fused ring compound represented by the formula

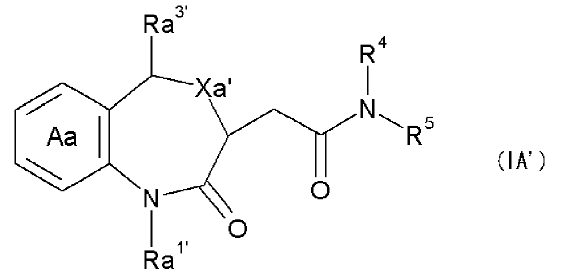

wherein ring Aa is an optionally substituted benzene ring, Xa' is - O-, -S(O)n- (n is 0, 1 or 2) or -NR⁶- (R⁶ is a hydrogen atom or a substituent) ; Ra¹' and Ra³' are each independently a hydrogen atom, an optionally substituted C₁₋₆ alkyl group, an optionally substituted C₂₋₆ alkenyl group, an optionally substituted phenyl group, an optionally substituted aralkyl group or an optionally substituted heterocyclic group; R⁴ is a hydrogen atom or an optionally substituted C₁₋₆ alkyl group; R⁵ is a hydrogen atom, an optionally substituted hydrocarbon group, an optionally substituted heterocyclic group, an optionally substituted amino group, an optionally substituted hydroxy group or an optionally substituted sulfonyl group, R⁴ and R⁵ may be bonded to each other to form, together with the adjacent nitrogen atom, an optionally substituted nitrogen-containing heterocycle [hereinafter sometimes to be abbreviated as compound (IA')], or a salt thereof.

Here, ring Aa and R⁶ are each as defined for ring Aa and R⁶ defined for the formula (IA).
As the "optionally substituted C₁₋₆ alkyl group", "optionally substituted C₂₋₆ alkenyl group", "optionally substituted phenyl group" and "optionally substituted aralkyl group" for Ra¹' and Ra^{3'}, the "optionally substituted C₁₋₆ alkyl group", "optionally substituted C₂₋₆ alkenyl group", "optionally substituted phenyl group" and "optionally substituted aralkyl group (preferably a C₇₋₁₄ aralkyl group)", which are exemplified as the "optionally substituted hydrocarbon group" for the aforementioned Ra¹, are used, respectively.
As the "optionally substituted heterocyclic group" for Ra¹' and Ra³' those exemplified as the substituent of ring A are used.
R⁴ and R⁵ are as defined for R⁴ and R⁵ for the above-mentioned "optionally substituted carbamoyl group (that is, -CON (R⁴) (R⁵) )" recited as a preferable example of Ra² of the formula (IA).

Xa' is preferably -O-, -S- or -NR⁶-, more preferably -O- or - NR⁶-. Among them, -O- is preferable.

Ra¹' is preferably an optionally substituted C₁₋₆ alkyl group or an optionally substituted aralkyl group (preferably a C₇₋₁₄ aralkyl group), more preferably an optionally substituted C₁₋₆ alkyl group. Among them, a C₁₋₆ alkyl group optionally substituted by substituent(s) selected from 1) a heterocyclic group (preferably furyl, thienyl, quinolyl) optionally substituted by substituent(s) selected from a heterocyclic group (preferably furyl, thienyl) and a phenyl group optionally substituted by a C₁₋₆ alkoxy group, 2) a hydroxy group, 3) a C₁₋₆ alkyl-carbonyloxy group (e.g., acetyloxy) and 4) a C₁₋₆ alkylsulfonyloxy group (e.g., methylsulfonyloxy) is preferable. Ra^{1'} is particularly preferably a C₁₋₆ alkyl group (e.g., methyl, ethyl, propyl, isopropyl, butyl, isobutyl, sec-butyl, tert-butyl, pentyl, isopentyl, neopentyl, hexyl), especially preferably neopentyl.

In the formula (IA' ) , when Xa' is -O- or -S (O) n-, Ra^{3'*} is preferably an optionally substituted phenyl group or an optionally substituted piperidinyl group, as is similar to the aforementioned Ra³. Among them, a phenyl group having substituent(s) at the meta-position is preferable. As used herein, as the substituent on the phenyl group, 1) a C₁₋₆ alkyl group optionally substituted by substituent(s) selected from a halogen atom, an optionally substituted amino group, an optionally substituted hydroxy group and an optionally substituted heterocyclic group, 2) an optionally substituted amino group, 3) an optionally substituted heterocyclic group, 4) an optionally substituted hydroxy group, 5) an acyl group and the like are preferable, as in the case of the aforementioned Ra³ .
Of the aforementioned substituents, a C₁₋₆ alkyl group optionally substituted by an optionally substituted amino group is preferable, and an acylaminomethyl group is particularly preferable.
Here, examples of acylamino of the acylaminomethyl group include those similar to the groups exemplified as the aforementioned Ra³. Ra ^{3a'} is particularly preferably a phenyl group having an acylaminomethyl group (e.g., formylaminomethyl, C₁₋₃ alkyl-carbonylaminomethyl, C₁₋₃ alkoxy-carbonylaminomethyl) at the meta-position.
In the formula (IA') , when Xa' is -NR⁶-, moreover, Ra ^{3'} is preferably a hydrogen atom.

Of compounds (IA'), a compound wherein ring Aa is a benzene ring optionally substituted by a halogen atom, Xa' is -O- or-S-,
Ra^{1'} is an optionally substituted C₁₋₆ alkyl group or an optionally substituted aralkyl group,
Ra^{3'} is a phenyl group optionally substituted by substituent(s) selected from 1) a C₁₋₆ alkyl group optionally substituted by an optionally substituted amino group, an optionally substituted hydroxy group or an optionally substituted heterocyclic group, 2) an optionally substituted amino group, 3) an optionally substituted heterocyclic group and 4) an acyl group,
R⁴ is a hydrogen atom, and
R⁵ is an optionally substituted C₁₋₆ alkyl group, an optionally substituted aralkyl group, an optionally substituted phenyl group, an optionally substituted cycloalkyl group or an optionally substituted heterocyclic group; and
a compound wherein
ring Aa is a benzene ring optionally substituted by a halogen atom, Xa' is-NR⁶_
R⁶ is 1) an optionally substituted C₆₋₁₄ aryl-carbonyl group; 2) an optionally substituted heterocycyclic group-carbonyl group; 3) a C₇₋₁₄
aralkyl group; or 4) a C₁₋₁₀ alkyl group substituted by an optionally substituted heterocyclic group,
Ra^{1'} is an optionally substituted C₁₋₆ alkyl group or an optionally substituted aralkyl group,
Ra^{3'} is a hydrogen atom,
R⁴ is a hydrogen atom, and
R⁵ is an optionally substituted C₁₋₆ alkyl group, an optionally substituted aralkyl group, an optionally substituted phenyl group, an optionally substituted cycloalkyl group or an optionally substituted heterocyclic group,
are preferable.

In addition, specifically preferable examples of compound (IA') also include a compound wherein
ring Aa is a benzene ring optionally substituted by a halogen atom, Xa' is -O- or -S-,
Ra^{1'} is a C₁₋₆ alkyl group optionally substituted by substituent(s) selected from 1) a heterocyclic group (preferably furyl, thienyl, quinolyl) optionally substituted by substituent(s) selected from a heterocyclic group (preferably furyl, thienyl) and a phenyl group optionally substituted by a C₁₋₆ alkoxy group, 2) a hydroxy group, 3) a C₁₋₆ alkyl-carbonyloxy group (e.g., acetyloxy) and 4) a C₁₋₆ alkylsulfonyloxy group (e.g., methylsulfonyloxy),
Ra^{3'} is a phenyl group optionally substituted by substituent(s) selected from 1) a C₁₋₆ alkyl group optionally substituted by substituent(s) selected from a halogen atom (preferably, fluorine, chlorine), an acylamino group (preferably, formylamino, a C₁₋₆ alkyl-carbonylamino group, a C₆₋₁₄ aryl-carbonylamino group, a C₁₋₆ alkoxy-carbonylamino group, a C₇₋₁₄ aralkyloxy-carbonylamino group, a C₁₋₆ alkylsulfonylamino group, a carbamoylamino group or a mono- or di-C₁₋₆ alkyl-carbamoylamino group, each of which may have 1 to 3 substituents selected from a halogen atom, a nitro group, a cyano group, a C₁₋₆ alkyl group, a mono- or di-C₁₋₆ alkylamino group, a hydroxy group, a C₁₋₆ alkoxy group and the like), an optionally substituted hydroxy group (preferably a hydroxy group, a carboxyl-C₁₋₆ alkoxy group, a C₁₋₆ alkoxy-carbonyl-C₁₋₆ alkoxy group) and a heterocyclic group (preferably an aromatic nitrogen-containing heterocyclic group (e.g., pyrrolyl, oxazolyl, isoxazolyl, thiazolyl, isothiazolyl, imidazolyl, pyrazolyl, oxadiazolyl, furazanyl, thiadiazolyl, triazolyl, tetrazolyl, pyridyl, pyridazinyl, pyrimidinyl, pyrazinyl)),
2) an amino group,
3) a heterocyclic group (preferably dioxolanyl),
4) a C₁₋₆ alkoxy group optionally substituted by substituent(s) selected from a carbamoyl group, a mono- or di-C₁₋₆ alkyl-carbamoyl group, a mono- or di-(heterocyclic group (preferably imidazolyl)-C₁₋₆ alkyl)-carbamoyl group (e.g., imidazolylpropylcarbamoyl) and a mono- or di-C₇₋₁₄ aralkyl-carbamoyl group, and
5) an acyl group (preferably formyl),
   R⁴ is a hydrogen atom, and
   R⁵ is (1) a C₁₋₆ alkyl group optionally having 1 to 3 substituents selected from a halogen atom, a carboxyl group, a heterocyclic group (preferably furyl, thienyl, pyridyl, tetrahydrofuranyl), a C₁₋₆ alkoxy-carbonyl group and the like, (2) a C₇₋₁₄ aralkyl group optionally having 1 to 3 substituents selected from a halogen atom, an optionally halogenated C₁₋₆ alkyl group, a C₁₋₆ alkylsulfonyl group, a C₁₋₆ alkylthio group, a C₁₋₆ alkoxy group and the like, (3)
   a C₃₋₁₀ cycloalkyl-C₁₋₆ alkyl group optionally having 1 to 3 substituents selected from a carboxyl group, a C₁₋₆ alkoxy-carbonyl group and the like, (4) a phenyl group or (5) a C₃₋₁₀ cycloalkyl group;
   a compound wherein
   ring Aa is a benzene ring optionally substituted by a halogen atom, Xa' is -NR^{6_},
   R⁶ is a C₆-₁₄ aryl-carbonyl group (preferably benzoyl) or a heterocyclic group-carbonyl group (preferably pyridyl-carbonyl, furyl-carbonyl, thienyl-carbonyl, pyrrolyl-carbonyl, oxazolyl-carbonyl, isoxazolyl-carbonyl, thiazolyl-carbonyl, isothiazolyl-carbonyl, pyrazinyl-carbonyl, piperidinyl-carbonyl, quinolyl-carbonyl or isoquinolyl-carbonyl), each of which may have 1 to 4 substituents selected from a halogen atom, an optionally halogenated C₁₋₆ alkyl group, an optionally halogenated C₁₋₆ alkoxy group, an optionally halogenated C₁₋₆ alkylthio group, a hydroxy group, a mercapto group, a cyano group, a nitro group, a carboxyl group, a carbamoyl group, a formyl group, an optionally halogenated C₁₋₆ alkyl-carbonyl group, a C₁₋₆ alkoxy-carbonyl group, an amino group, a mono- or di-C₁₋₆ alkylamino group, a formylamino group, an optionally halogenated C₁₋₆ alkyl-carbonylamino group and the like; a compound wherein
   Ra^{1'} is a C₁₋₆ alkyl group optionally substituted by substituent(s) selected from 1) a heterocyclic group (preferably furyl, thienyl, quinolyl) optionally substituted by substituent(s) selected from a heterocyclic group (preferably furyl, thienyl) and a phenyl group optionally substituted by a C₁₋₆ alkoxy group, 2) a hydroxy group, 3) a C₁₋₆ alkyl-carbonyloxy group (e.g., acetyloxy) and 4) a C₁₋₆ alkylsulfonyloxy group (e.g., methylsulfonyloxy),
   Ra³' is a hydrogen atom,
   R⁴ is a hydrogen atom, and
   R⁵ is (1) a C₁₋₆ alkyl group optionally having 1 to 3 substituents selected from a halogen atom, a carboxyl group, a heterocyclic group (preferably furyl, thienyl, pyridyl, tetrahydrofuranyl), a C₁₋₆ alkoxy-carbonyl group and the like, (2) a C₇₋₁₄ aralkyl group optionally having 1 to 3 substituents selected from a halogen atom, an optionally halogenated C₁₋₆ alkyl group, a C₁₋₆ alkylsulfonyl group, a C₁₋₆ alkylthio group, a C₁₋₆ alkoxy group and the like, (3) a C₃₋₁₀ cycloalkyl-C₁₋₆ alkyl group optionally having 1 to 3 substituents selected from a carboxyl group, a C₁₋₆ alkoxy-carbonyl group and the like, (4) a phenyl group or (5) a C₃₋₁₀ cycloalkyl group; and the like.

Examples of compound (I) also include a fused ring compound represented by the formula

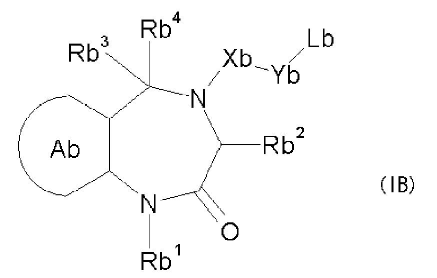

wherein ring Ab is an optionally substituted aromatic ring; Xb is a divalent hydrocarbon group, -CO- or -SO₂-; Yb is a bond, a divalent hydrocarbon group, -O-, -NRb⁵- (Rb⁵ is a hydrogen atom, an optionally substituted hydrocarbon group or an optionally substituted heterocyclic group) or -S(O)_{nb}- (nb is 0, 1 or 2); Lb is an optionally substituted cyclic group; Rb¹, Rb³ and Rb⁴ are each independently a hydrogen atom, an optionally substituted hydrocarbon group or an optionally substituted heterocyclic group, Rb³ and Rb⁴ in combination form an oxo group; Rb² is an optionally substituted hydrocarbon group or an optionally substituted heterocyclic group, or a salt thereof and the like.

Examples of the "optionally substituted aromatic ring" for ring Ab include those similar to the rings exemplified as the aforementioned ring A. The aromatic ring for ring Ab is preferably a benzene ring. The ring Ab is preferably a benzene ring.

As the "divalent hydrocarbon group" for Xb or Yb, for example,
(1) a C₁₋₆ alkylene group (e.g., -CH₂-, - (C_{H}2₎ 2-, - ₍C_{H}2) 3-, _{- (}CH2) 4-, - (CH₂) ₅-, -(CH2) ₆-, -CH (CH₃) -, -C (CH₃) ₂-, -CH (CH₃) CH₂-, -C (CH₃) ₂CH₂-,-CH (CH₂CH₃) CH₂-, - (CH (CH₃))₂-, - (CH₂) ₂C (CH₃) ₂-, -CH₂C (CH₃) ₂CH₂-,-CH(CH₂CH₃)(CH₂)₂-, -(CH₂)₃C(CH₃)₂-, ₋(CH₂)₃CH(CH₃)CH₂-);
(2) a C₂₋₆ alkenylene group (e.g., -CH=CH-, -CH₂-CH=CH-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂-) ;
(3) a C₂₋₆ alkynylene group (e.g., -C_{≡}-, -CH₂-C_{≡}C-, -CH₂-C_{≡}-CH₂-CH₂-);
(4) a C₃₋₆ cycloalkylene group (e.g., cyclopropylene, cyclobutylene, cyclopentylene, cyclohexylene);
(5) a C₃₋₆ cycloalkenylene group (e.g., cyclopropenylene, cyclobutenylene, cyclopentenylene, cyclohexenylene);
(6) a phenylene group;
and the like can be mentioned.
The "divalent hydrocarbon group" is preferably a C₁₋₆ alkylene group.

Xb is preferably a C₁₋₆ alkylene group (preferably-CH₂-) or-CO-, more preferably-CO-.

Yb is preferably a bond, a C₁₋₆ alkylene group (preferably-CH₂-) or-NH-, more preferably a bond.

In the "optionally substituted cyclic group" for Lb, as the cyclic group, for example, a heterocyclic group, an alicyclic hydrocarbon group, an aryl group and the like can be mentioned. Examples of the heterocyclic group include those similar to the "heterocyclic group" of (1) "optionally substituted heterocyclic group" which is the substituent of the aforementioned ring A. In addition, examples of the alicyclic hydrocarbon group and aryl group include those similar to the "alicyclic hydrocarbon group" and "aryl group" exemplified as the "hydrocarbon group" of (6) "optionally substituted hydrocarbon group" which is the substituent of the aforementioned ring A.
The cyclic group may have 1 to 4 substituents at substitutable positions, and examples of such substituent include those similar to the substituents exemplified as substituent group
A. The substituent is preferably a halogen atom (e.g., fluorine, chlorine, bromine, iodine), an optionally halogenated C₁₋₆ alkyl group (e.g., methyl, trifluoromethyl), an optionally halogenated C₁₋₆ alkoxy group (e.g., methoxy, ethoxy, trifluoromethoxy), an optionally halogenated C₁₋₆ alkylthio group (e.g., methylthio, ethylthio, trifluoromethylthio), a hydroxy group, a mercapto group, a cyano group, a nitro group, a carboxyl group, a carbamoyl group, a formyl group, an optionally halogenated C₁₋₆ alkyl-carbonyl group (e.g., acetyl, propionyl, trifluoroacetyl), a C₁₋₆ alkoxy-carbonyl group (e.g., methoxycarbonyl, ethoxycarbonyl, propoxycarbonyl), an amino group, a mono- or di-C₁₋₆ alkylamino group (e.g., methylamino, ethylamino, dimethylamino, diethylamino), a formylamino group, an optionally halogenated C₁₋₆ alkyl-carbonylamino group (e.g., acetylamino, propionylamino, butyrylamino, trifluoroacetylamino) and the like.
The cyclic group is preferably a phenyl group or a heterocyclic group (preferably pyridyl, furyl, thienyl, pyrrolyl, oxazolyl, isoxazolyl, thiazolyl, isothiazolyl, pyrazinyl, piperidinyl, quinolyl or isoquinolyl; more preferably pyridyl or quinolyl; particularly preferably pyridyl), more preferably a pyridyl group (preferably a 4-pyridyl group).

Examples of the "optionally substituted hydrocarbon group" and "optionally substituted heterocyclic group" for Pb¹, Rb², Rb³, Rb⁴ or Rb⁵ each include those similar to the groups exemplified as the substituent of the aforementioned ring A.

Rb¹ is preferably an optionally substituted C₁₋₆ alkyl group, more preferably a C₁₋₆ alkyl group optionally substituted by a C₁₋₆ alkyl-carbonyloxy group (e.g., acetyloxy). Particularly, a C₁₋₆ alkyl group is preferably, and neopentyl is particularly preferable.
Here, examples of the substituent of the "optionally substituted C₁₋₆ alkyl group" include those similar to the substituent of the aforementioned "optionally substituted hydrocarbon group".
Rb³, Rb⁴ and Rb⁵ are preferably hydrogen atoms.
Rb² is preferably an "optionally substituted hydrocarbon group", more preferably a C₁₋₆ alkyl group substituted by - CON (R⁴) (R⁵) wherein R⁴ and R⁵ are as defined above. Among these,

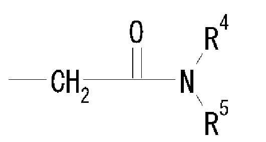

wherein R⁴ and R⁵ are as defined above, is preferable.
R⁴ is preferably a hydrogen atom.
R⁵ is preferably an optionally substituted C₇₋₁₄ aralkyl group (preferably benzyl), more preferably a C₇₋₁₄ aralkyl (preferably benzyl) optionally substituted by substituent(s) selected from a halogen atom and an optionally halogenated C₁₋₆ alkyl group. Particularly, a C₇₋₁₄ aralkyl (preferably benzyl) optionally substituted by a halogen atom (preferably a fluorine atom) is preferable.

Of compounds (IB), a compound wherein
ring Ab is a benzene ring;
Xb is a C₁₋₆ alkylene group or-CO-;
Yb is a bond;
Lb is a pyridyl group (preferably a 4-pyridyl group) optionally having 1 to 4 substituents selected from a halogen atom, an optionally halogenated C₁₋₆ alkyl group, an optionally halogenated C₁₋₆ alkoxy group, an optionally halogenated C₁₋₆ alkylthio group, a hydroxy group, a mercapto group, a cyano group, a nitro group, a carboxyl group, a carbamoyl group, a formyl group, an optionally halogenated C₁₋₆ alkyl-carbonyl group, a C₁₋₆ alkoxy-carbonyl group, an amino group, a mono- or di-C₁₋₆ alkylamino group, a formylamino group, an optionally halogenated C₁₋₆ alkyl-carbonylamino group and the like;
Rb¹ is a C₁₋₆ alkyl group;
Rb³ and Rb⁴ are each a hydrogen atom; and
Rb² is

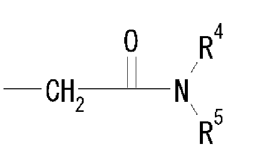

R⁴ is a hydrogen atom, and R⁵ is a C₇₋₁₄ aralkyl (preferably benzyl) optionally substituted by a halogen atom (preferably a fluorine atom),
is preferable.

Preferable examples of compound (I) also include a fused ring compound represented by the formula

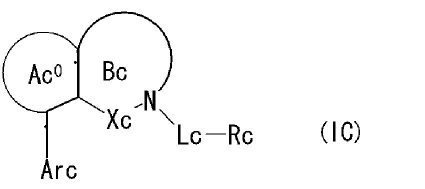

wherein ring Ac° is an aromatic ring optionally further having substituent(s) other than -Arc; ring Bc is a nitrogen-containing 6-to 9-membered ring optionally further having substituent(s) other than -Lc-Rc; Xc is an optionally substituted methylene group; Arc is an optionally substituted aromatic group; Rc is an optionally substituted cyclic group; Lc is an optionally substituted C₁₋₃ alkylene group, -CONH-, -SO₂NH- or -SO₂-, [hereinafter sometimes to be abbreviated as compound (IC)], or a salt thereof and the like.

Examples of the "optionally having substituent(s) aromatic ring" for ring Ac⁰ include those similar to the rings exemplified as the "optionally substituted aromatic ring" of the aforementioned ring A. The aromatic ring is preferably a pyridine ring. In addition, ring Ac⁰ is preferably a pyridine ring optionally substituted by an optionally halogenated C₁₋₆ alkyl group, more preferably a pyridine ring.

Examples of the "nitrogen-containing 6- to 9-membered ring" for ring Bc include a 6- to 9-membered ring containing one or more nitrogen atoms as ring-constituting atom, from among the aforementioned "5- to 9-membered ring" for ring B'. Among these, a 6- to 9-membered nonaromatic nitrogen-containing heterocycle is preferable.
Specifically preferable examples of the "nitrogen-containing 6- to 9-membered ring" include the following rings.

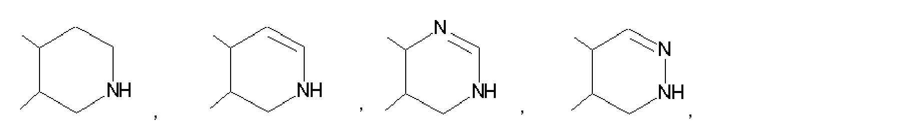

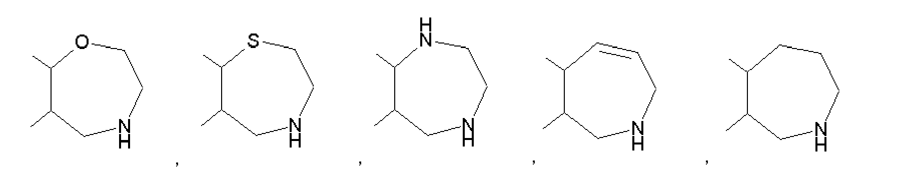

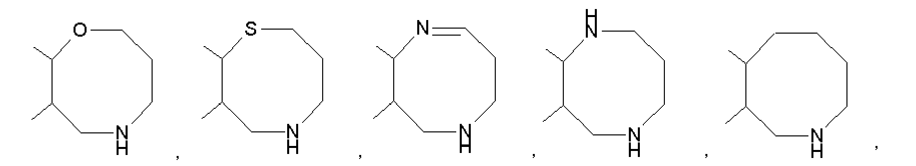

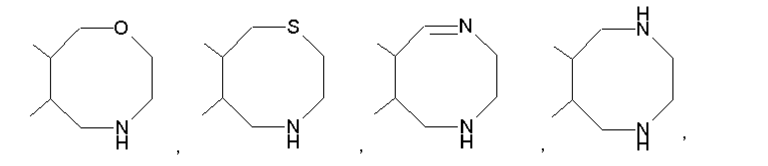

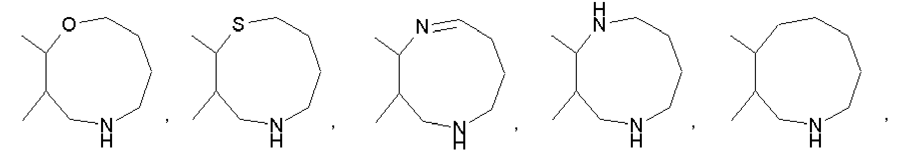

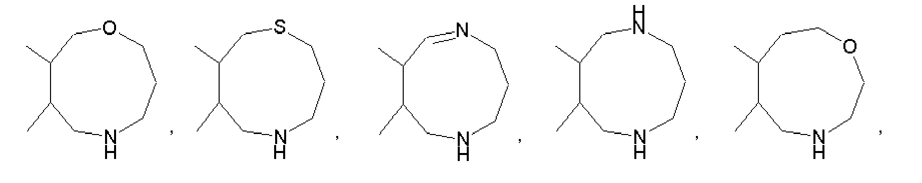

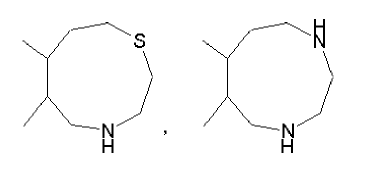

The "nitrogen-containing 6- to 9-membered ring" may have 1 to 4 substituents at substitutable positions. Examples of such substituent include a halogen atom (e.g., fluorine, chlorine, bromine, iodine), a nitro group, a cyano group, an optionally halogenated C₁₋₆ alkyl group (e.g., methyl, ethyl, trifluoromethyl), a mono- or di-C₁₋₆ alkylamino group (e.g., methylamino, dimethylamino, ethylamino), a hydroxy group, an optionally halogenated C₁₋₆ alkoxy group (e.g., methoxy, ethoxy, propoxy, isopropoxy), an oxo group, a thioxo group, a carboxyl group, a formyl group, a C₁₋₆ alkyl-carbonyl group (e.g., acetyl, propionyl), a C₆₋₁₄ aryl-carbonyl group (e.g., benzoyl), a C₁₋₆ alkoxy-carbonyl group (e.g., methoxycarbonyl, ethoxycarbonyl, propoxycarbonyl) and the like. Preferred is an oxo group.
Ring Bc is preferably

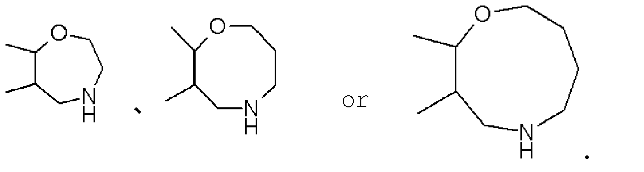

The methylene group for Xc optionally has 1 or 2 substituents. As such substituents, for example, a nitro group, a cyano group, an optionally halogenated C₁₋₆ alkyl group (e.g., methyl, ethyl, trifluoromethyl), a C₃₋₈ cycloalkyl group (e.g., cyclopropyl, cyclobutyl, cyclopentyl, cyclohexyl), an optionally halogenated C₁₋₆ alkylidene group (e.g., methylidene, ethylidene, propylidene), a C₆₋₁₄ aryl group (e.g., phenyl), a heterocyclic group (e.g., thienyl, furyl, pyridyl), a carboxyl group, a C₁₋₆ alkoxy-carbonyl group (e.g., methoxycarbonyl, ethoxycarbonyl, propoxycarbonyl), a C₆₋₁₄ aryloxy-carbonyl group (e.g., phenoxycarbonyl), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), an oxo group, a thioxo group and the like can be mentioned. Xc is preferably a methylene group.

Examples of the "optionally substituted aromatic group" for Arc include the "optionally substituted C₆₋₁₄ aryl group" exemplified as the substituent of the aforementioned substituent group A and those (aromatic ones) exemplified as (1) "optionally substituted heterocyclic group" which is the substituent of and ring A. Arc is preferably an optionally substituted C₆₋₁₄ aryl group, more preferably an optionally substituted phenyl group (preferably a phenyl group optionally substituted by 1 to 3 halogen atoms), particularly preferably a phenyl group or a 2-fluorophenyl group. Preferably, Arc is a phenyl group optionally having substituent(s) at the ortho-position relative to the bond to the ring Ac.

Examples of the cyclic group of the "optionally substituted cyclic group" for Rc include a heterocyclic group, an alicyclic hydrocarbon group, an aryl group and the like. Examples of the heterocyclic group include a heterocyclic group exemplified as (1) "optionally substituted heterocyclic group" which is the substituent of the aforementioned ring A. In addition, examples of the alicyclic hydrocarbon group and aryl group include those similar to the groups exemplified as the hydrocarbon group of (6) "optionally substituted hydrocarbon group" which is the substituent of the aforementioned ring A.
The cyclic group may have 1 to 4 substituents at substitutable positions, and examples of such substituent include those similar to the substituent of substituent group A. The substituent is preferably a halogen atom (e.g., fluorine, chlorine, bromine, iodine), an optionally halogenated C₁₋₆ alkyl group (e.g., methyl, ethyl, trifluoromethyl), an optionally halogenated C₁₋₆ alkoxy group (e.g., methoxy, ethoxy, propoxy, isopropoxy, trifluoromethoxy), an optionally halogenated C₁₋₆ alkylthio group (e.g., methylthio, ethylthio, trifluoromethylthio), a hydroxy group, a mercapto group, a cyano group, a nitro group, a carboxyl group, a carbamoyl group, a formyl group, an optionally halogenated C₁₋₆ alkyl-carbonyl group (e.g., acetyl, propionyl, trifluoroacetyl), a C₁₋₆ alkoxy-carbonyl group (e.g., methoxycarbonyl, ethoxycarbonyl, propoxycarbonyl), an amino group, a mono- or di-C₁₋₆ alkylamino group (e.g., methylamino, ethylamino, dimethylamino, diethylamino), a formylamino group, an optionally halogenated C₁₋₆ alkyl-carbonylamino group (e.g., acetylamino, propionylamino, butyrylamino, trifluoroacetylamino), an amino-C₁₋₆ alkyl group (e.g., aminomethyl), a C₁₋₆ alkoxy-carbonylamino-C₁₋₆ alkyl group (e.g., tert-butoxycarbonylaminomethyl) and the like.

The cyclic group is preferably phenyl, naphthyl (preferably 1-naphthyl group), indanyl, pyridyl, benzothienyl, benzofuryl, quinolyl, isoquinolyl, benzodioxolyl and the like. The substituent of the cyclic group is preferably a halogen atom (e.g., fluorine, chlorine, bromine, iodine), a nitro group, a cyano group, an optionally halogenated C₁₋₆ alkyl group (e.g., methyl, ethyl, trifluoromethyl), an amino-C₁₋₆ alkyl group (e.g., aminomethyl), a C₁₋₆ alkoxy-carbonylamino-C₁₋₆ alkyl group (e.g., tert-butoxycarbonylaminomethyl), a mono- or di-C₁₋₆ alkylamino group (e.g., methylamino, dimethylamino, ethylamino), a hydroxy group, an optionally halogenated C₁₋₆ alkoxy group (e.g., methoxy, ethoxy, propoxy, isopropoxy) and the like.

Rc is preferably a phenyl group optionally having 1 to 4 substituents selected from a halogen atom (e.g., fluorine, chlorine, bromine, iodine), a nitro group, a cyano group, an optionally halogenated C₁₋₆ alkyl group (e.g., methyl, ethyl, trifluoromethyl), an amino-C₁₋₆ alkyl group (e.g., aminomethyl), a C₁₋₆ alkoxy-carbonylamino-C₁₋₆ alkyl group (e.g., tert-butoxycarbonylaminomethyl), a mono- or di-C₁₋₆ alkylamino group (e.g., methylamino, dimethylamino, ethylamino), a hydroxy group, an optionally halogenated C₁₋₆ alkoxy group (e.g., methoxy, ethoxy, propoxy, isopropoxy) and the like. Rc is more preferably a 3,5-bis(trifluoromethyl)phenyl group.

As the "C₁₋₃ alkylene group" of the "optionally substituted C₁₋₃ alkylene group" for Lc, for example, -CH₂-, - (CH₂)₂-, - (CH₂)₃-,-CH (CH₃) -, -C (CH₃) ₂-, -CH (CH₃) CH₂- and the like can be mentioned. Among them, a methylene group is preferable. The "C₁₋₃ alkylene group" optionally has 1 to 3 substituents at substitutable positions. Examples of such substituent include a halogen atom (e.g., fluorine, chlorine, bromine, iodine), a nitro group, a cyano group, a mono- or di-C₁₋₆ alkylamino group (e.g., methylamino, dimethylamino, ethylamino), a hydroxy group, an optionally halogenated C₁₋₆ alkoxy group (e.g., methoxy, ethoxy, propoxy, isopropoxy), an oxo group, a thioxo group, a C₆₋₁₄ aryl group (e.g., phenyl), a heterocyclic group (e.g., thienyl, furyl, pyridyl) and the like. Lc is preferably a C₁₋₃ alkylene group optionally substituted by an oxo group or -SO₂-; more preferably -CH₂- (a methylene group), -CH (CH₃) -, -CO-, -COCH₂- or -SO₂-. Particularly preferred is -CH₂- (a methylene group) or -CH (CH₃)-.

Of compounds (IC), a fused ring compound represented by the formula

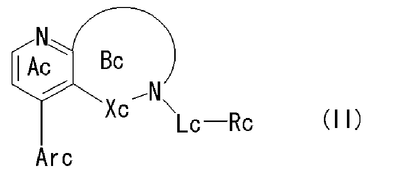

wherein ring Ac is a pyridine ring optionally further having substituent(s) other than -Arc, and each of other symbols is as defined for the formula (IC). However, Xc group is not a methylene group substituted by an oxo group [hereinafter sometimes to be abbreviated as compound (II)], or a salt thereof and the like can also be mentioned.

Examples of the "optionally substituted pyridine ring" for ring Ac include the "optionally substituted aromatic ring" for the aforementioned ring A, wherein an aromatic ring is a pyridine ring. Ring Ac is preferably a pyridine ring optionally substituted by an optionally halogenated C₁₋₆ alkyl group, more preferably a pyridine ring.

As one preferable example of compound (II), a fused ring compound represented by the formula

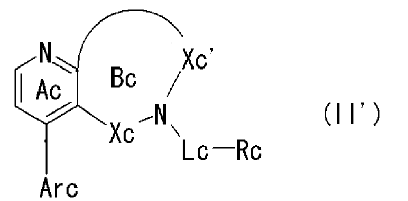

wherein Xc' is an optionally substituted methylene group, and each of other symbols is as defined for the formula (II) [hereinafter sometimes to be abbreviated as compound (II')], or a salt thereof can be mentioned.

The methylene group for Xc' may have 1 or 2 substituents. Examples of such substituent include a nitro group, a cyano group, an optionally substituted C₁₋₆ alkyl group, a C₂₋₆ alkenyl group (e.g., vinyl, propenyl), a C₂₋₆ alkynyl group (e.g., ethynyl, propargyl), a C₃₋₈ cycloalkyl group (e.g., cyclopropyl, cyclobutyl, cyclopentyl, cyclohexyl), a C₆₋₁₄ aryl group (e.g., phenyl), a heterocyclic group (e.g., thienyl, furyl, pyridyl), a carboxyl group, a C₁₋₆ alkoxy-carbonyl group (e.g., methoxycarbonyl, ethoxycarbonyl, propoxycarbonyl), a C₆₋₁₄ aryloxy-carbonyl group (e.g., phenoxycarbonyl), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), an oxo group, a thioxo group and the like.

The "optionally substituted C₁₋₆ alkyl group" is as defined for (i) "optionally substituted C₁₋₆ alkyl group" exemplified as the substituent of (2) "optionally substituted hydroxy group" which is the substituent of ring A.

Xc' is preferably a methylene group optionally substituted by substituent(s) selected from an oxo group and an optionally substituted C₁₋₆ alkyl group, more preferably a methylene group or - C (=O) -.

Preferable examples of compound (II') include fused ring compounds represented by the following formulas

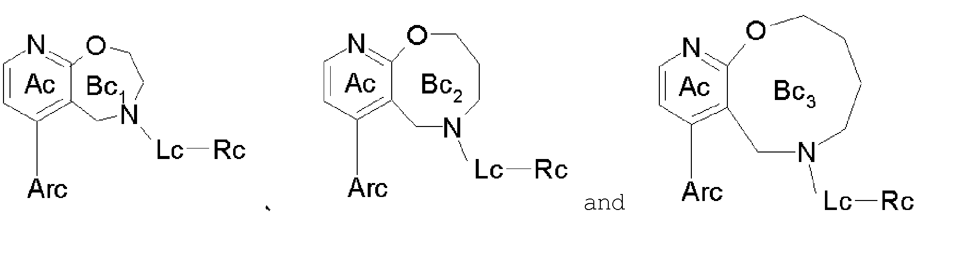

wherein ring Bc₁, ring Bc₂ and ring Bc₃ each may further have substituent(s) other than -Lc-Rc, and each of other symbols is as defined for the formula (II), or a salt thereof and the like.

Examples of the substituent other than -Lc-Rc, which ring Bc₁, ring Bc₂ and ring Bc₃ may each have, include those exemplified as the substituent of the aforementioned ring Bc.

Furthermore, a fused ring compound represented by the following formula

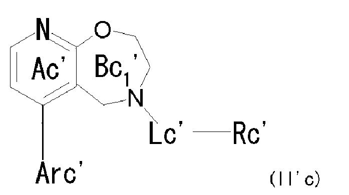

wherein ring Ac' is a pyridine ring optionally further having substituent(s) other than -Arc',
ring Bc₁' may further have a substituent other than -Lc'-Rc',
Lc' is a C₁₋₃ alkylene group optionally substituted by a C₁₋₃ alkyl group or an oxo group,
Rc' is an optionally substituted phenyl group, and Arc' is a phenyl group optionally further having substituent(s) at the ortho-position relative to the bond to the ring Ac'
is also preferable.

Ring Ac' is as defined for the aforementioned ring Ac.
Examples of the substituent that ring Bc₁' may have include those exemplified as the substituent of the aforementioned ring Bc.
Examples of the "C₁₋₃ alkylene group optionally substituted by a C₁₋₃ alkyl group or an oxo group" for Lc' include the "optionally substituted C₁₋₃ alkylene group" for the aforementioned Lc, which have 1 to 3 substituents selected from a C₁₋₃ alkyl group (e.g., methyl, ethyl, propyl) and an oxo group at the substitutable position. Among them, a methyl group is preferable. Examples of the "C₁₋₃ alkylene group" include -CH₂-, - (CH₂)₂-, - (CH₂)₃-, -CH (CH₃) -, -C (CH₃) ₂-, -CH (CH₃) CH₂- and the like. Among them, a methylene group is preferable.
Examples of the substituent which the "optionally substituted phenyl group" for Rc' can have include those exemplified as the substituent of the "optionally substituted cyclic group" for the aforementioned Rc.
Examples of the substituent that Arc' can have at the ortho-position relative to the bond to the ring Ac' include those exemplified as the substituent of the "optionally substituted aromatic group" for the aforementioned Arc.

Preferable examples of compound (I) also include a fused ring compound represented by the formula

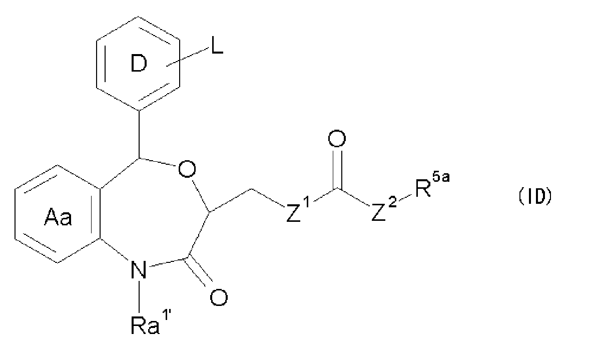

wherein ring Aa and ring D are each independently an optionally substituted benzene ring; Ra¹' is a hydrogen atom, an optionally substituted C₁₋₆ alkyl group, an optionally substituted C₂₋₆ alkenyl group, an optionally substituted phenyl group, an optionally substituted aralkyl group or an optionally substituted heterocyclic group; L is -CH₂NHCOR⁷, -OCH₂CONR⁸R⁹ or -CH₂-Het (R⁷ is a hydrogen atom, a C₁₋₃ alkyl group or a C₁₋₃ alkoxy group; R⁸ is a hydrogen atom or an optionally substituted C₁₋₆ alkyl group; R⁹ is a hydrogen atom, an optionally substituted hydrocarbon group or an optionally substituted heterocyclic group; Het is an aromatic nitrogen-containing heterocyclic group); at least one of Z¹ and Z² is -NR^{4a}-(R^{4a} is a hydrogen atom or an optionally substituted C₁₋₆ alkyl group), and the other is a bond or -NR^{4a}- (R^{4a} is as defined above); R^{5a} is a hydrogen atom, an optionally substituted hydrocarbon group or an optionally substituted heterocyclic group, when Z² is -NR^{4a}-(R^{4a} is as defined above), R^{5a} and R^{4a} may be bonded to each other to form, together with the adjacent nitrogen atom, an optionally substituted nitrogen-containing heterocycle, or a salt thereof and the like.

The "optionally substituted benzene ring" for ring Aa is as defined for ring Aa in compound (IA).

The benzene ring for ring D optionally has 1 to 3 substituents besides L. Examples of such substituent include a halogen atom (e.g., fluorine, chlorine, bromine, iodine), a nitro group, a cyano group, an optionally halogenated C₁₋₆ alkyl group (e.g., methyl, ethyl, trifluoromethyl), an optionally halogenated C₁₋₆ alkoxy group (e.g., methoxy, ethoxy, trifluoromethoxy), a hydroxy group, a carboxyl group, a formyl group, an optionally halogenated C₁₋₆ alkyl-carbonyl group (e.g., acetyl, propionyl, trifluoroacetyl), a C₁₋₆ alkoxy-carbonyl group (e.g., methoxycarbonyl, ethoxycarbonyl, propoxycarbonyl), an amino group,
a mono- or di-C₁₋₆ alkylamino group (e.g., methylamino, ethylamino, dimethylamino, diethylamino) and the like. Of these, a C₁₋₆ alkoxy group (e.g., methoxy, ethoxy, trifluoromethoxy) and the like are preferable.

Ra^{1'} is as defined for Ra^{1'} of compound (IA').
In compound (ID), Ra^{1'} is preferably an optionally substituted C₁₋₆ alkyl group, more preferably, a C₁₋₆ alkyl group optionally substituted by substituent(s) selected from 1) a heterocyclic group (preferably furyl, thienyl, quinolyl) optionally substituted by substituent(s) selected from a heterocyclic group (preferably furyl, thienyl) and a phenyl group optionally substituted by a C₁₋₆ alkoxy group, 2) a hydroxy group, 3) a C₁₋₆ alkyl-carbonyloxy group (e.g., acetyloxy) and 4) a C₁₋₆ alkylsulfonyloxy group (e.g., methylsulfonyloxy).

L is-CH₂NHCOR⁷, -OCH₂CONR⁸R⁹ or-CH₂-Het (wherein R⁷ is a hydrogen atom, a C₁₋₃ alkyl group or a C₁₋₃ alkoxy group; R⁸ is a hydrogen atom or an optionally substituted C₁₋₆ alkyl group; R⁹ is a hydrogen atom, an optionally substituted hydrocarbon group or an optionally substituted heterocyclic group; and Het is a nitrogen-containing aromatic heterocyclic group). Particularly, -CH₂NHCOR⁷ (wherein R⁷ is as defined above) is preferable.
Here, as the C₁₋₃ alkyl group for R⁷, for example, methyl, ethyl, propyl, isopropyl and the like can be mentioned.
As the C₁₋₃ alkoxy group for R⁷, for example, methoxy, ethoxy, propoxy, isopropoxy and the like can be mentioned.
R⁷ is preferably a methyl group or a methoxy group.
As the "optionally substituted C₁₋₆ alkyl group" for R⁸, those exemplified for the aforementioned R⁴ can be used. As the "optionally substituted hydrocarbon group" and "optionally substituted heterocyclic group" for R⁹, those exemplified for the aforementioned R⁵ can be used respectively. R⁸ is preferably a hydrogen atom or a C₁₋₆ alkyl group. R⁹ is preferably a hydrogen atom, a C₁₋₆ alkyl group optionally substituted by a heterocyclic group (preferably imidazolyl) or a C₇₋₁₄ aralkyl group (preferably phenethyl).

As the aromatic nitrogen-containing heterocyclic group for Het, an aromatic heterocyclic group containing at least one nitrogen atom as a ring-constituting atom (e.g., pyrrolyl, oxazolyl, isoxazolyl, thiazolyl, isothiazolyl, imidazolyl, pyrazolyl, oxadiazolyl, furazanyl, thiadiazolyl, triazolyl, tetrazolyl, pyridyl, pyridazinyl, pyrimidinyl, pyrazinyl) from among those exemplified as the "heterocyclic group" of (1) "optionally substituted heterocyclic group" which is the substituent of ring A are used. Among them, imidazolyl and triazolyl are preferable.

Ring D is preferably substituted by L at the meta-position.

As the "optionally substituted C₁₋₆ alkyl group" for R^{4a}, those exemplified for the aforementioned R⁴ can be used. Particularly, a C₁₋₆ alkyl group is preferable. R^{4a} is preferably a hydrogen atom or a methyl group, more preferably a hydrogen atom.

As the "optionally substituted hydrocarbon group" and "optionally substituted heterocyclic group" for R^{5a}, those exemplified for the aforementioned R⁵ can be used respectively. R^{5a} is preferably an "optionally substituted C₁₋₆ alkyl group", an "optionally substituted C₇₋₁₄ aralkyl group", an "optionally substituted C₃₋₁₀ cycloalkyl-C₁₋₆ alkyl group", an "optionally substituted phenyl group", an "optionally substituted C₃₋₁₀ cycloalkyl group" or an "optionally substituted heterocyclic group".
Here, as preferable specific examples of the "optionally substituted C₁₋₆ alkyl group", a C₁₋₆ alkyl group (preferably methyl, ethyl, propyl) optionally having 1 to 3 substituents selected from a halogen atom, a carboxyl group, a heterocyclic group (preferably furyl, thienyl, pyridyl, tetrahydrofuranyl), a C₁₋₆ alkoxy-carbonyl group (preferably tert-butoxycarbonyl) and the like, and the like can be mentioned.
As preferable specific examples of the "optionally substituted C₇₋₁₄ aralkyl group", a C₇₋₁₄ aralkyl group (preferably benzyl, phenethyl, 2-phenylpropyl) optionally having 1 to 3 substituents selected from a halogen atom, an optionally halogenated C₁₋₆ alkyl group (preferably trifluoromethyl), a C₁₋₆ alkylsulfonyl group (preferably methylsulfonyl), a C₁₋₆ alkylthio group (preferably methylthio), a C₁₋₆ alkoxy group (preferably methoxy) and the like, and the like can be mentioned.
As preferable specific examples of the "optionally substituted C₃₋₁₀ cycloalkyl-C₁₋₆ alkyl group", a C₃₋₁₀ cycloalkyl-C₁₋₆ alkyl group (preferably cyclopropylmethyl, cyclohexylmethyl, cycloheptylmethyl) optionally having 1 to 3 substituents selected from a carboxyl group, a C₁₋₆ alkoxy-carbonyl group (preferably methoxycarbonyl) and the like, and the like can be mentioned.
As preferable specific examples of the "optionally substituted phenyl group", a phenyl group and the like can be mentioned.
As preferable specific examples of the "optionally substituted C₃₋₁₀ cycloalkyl group", a C₃₋₁₀ cycloalkyl group (preferably cyclohexyl) and the like can be mentioned.
R^{5a} is more preferably a C₁₋₆ alkyl group substituted by a heterocyclic group (preferably furyl, thienyl, pyridyl, tetrahydrofuranyl), an optionally substituted C₇₋₁₄ aralkyl group or an optionally substituted C₃₋₁₀ cycloalkyl-C₁₋₆ alkyl group.

When Z² is -NR^{4a}- (wherein R^{4a} is as defined above), as the "optionally substituted nitrogen-containing heterocycle" formed by R^{5a} and R^{4a} bonded to each other, together with the adjacent nitrogen atom, those similar to the "optionally substituted nitrogen-containing heterocycle" formed by the aforementioned R⁴ and R⁵ can be mentioned. Particularly, tetrahydroisoquinoline and the like are preferable.

In compound (ID), preferably, one of Z¹ and Z² is-NH- and the other is a bond.

Of compounds (ID), a compound wherein
ring Aa is a benzene ring optionally substituted by a halogen atom (preferably a chlorine atom);
ring D is a benzene ring optionally having a C₁₋₆ alkoxy group besides L;
Ra¹' is a C₁₋₆ alkyl group optionally substituted by substituent(s) selected from 1) a heterocyclic group (preferably furyl, thienyl, quinolyl) optionally substituted by substituent(s) selected from a heterocyclic group (preferably furyl, thienyl) and a phenyl group optionally substituted by a C₁₋₆ alkoxy group, 2) a hydroxy group, 3) a C₁₋₆ alkyl-carbonyloxy group and 4) a C₁₋₆ alkylsulfonyloxy group;
L is -CH₂NHCOR⁷ (wherein R⁷ is a hydrogen atom, a C₁₋₃ alkyl group or a C₁₋₃ alkoxy group) which substitutes the meta-position of ring D; R^{5a} is (1) a C₁₋₆ alkyl group substituted by an optionally substituted heterocyclic group (preferably furyl, thienyl, pyridyl, tetrahydrofuranyl); (2) a C₇₋₁₄ aralkyl group optionally having 1 to 3 substituents selected from a halogen atom, an optionally halogenated C₁₋₆ alkyl group, a C₁₋₆ alkylsulfonyl group, a C₁₋₆ alkylthio group, a C₁₋₆ alkoxy group and the like; or (3) a C₃₋₁₀ cycloalkyl-C₁₋₆ alkyl group optionally having 1 to 3 substituents selected from a carboxyl group, a C₁₋₆ alkoxy-carbonyl group and the like; and
one of Z¹ and Z² is-NH- and the other is a bond (preferably Z¹ is a bond, Z² is-NH-),
is preferable.

Examples of other embodiments of compound (I) include a fused ring compound represented by the formula

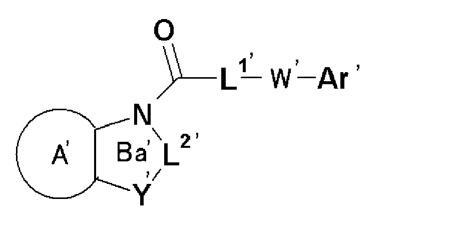

wherein ring A' is an optionally substituted 5- or 6-membered aromatic ring,
ring Ba' is a 6- to 8-membered nonaromatic nitrogen-containing heterocycle,
W' and Y' are each independently a bond, -O-, -NR- (R is a hydrogen atom or an optionally substituted C₁₋₆ alkyl group) or -S(O)n- (n is an integer of 0 to 2),
L1' is a chained C₁₋₅ alkylene group or chained C₂₋₅ alkenylene group, each of which is optionally substituted,
L²' is a chained C₂₋₅ alkylene group or chained C₂₋₅ alkenylene group, each of which is optionally substituted, and
Ar' is an optionally substituted cyclic group.

Examples of the "5- or 6-membered aromatic ring" for ring A' include a benzene ring and a 5- or 6-membered aromatic heterocycle.
Examples of the aromatic heterocycle include a 5- or 6-membered aromatic heterocycle containing, as ring-constituting atom besides carbon atom, 1 to 3 hetero atoms selected from a nitrogen atom, an oxygen atom and a sulfur atom. Specific examples of the aromatic heterocycle include furan, thiophene, pyrrole, oxazole, isoxazole, thiazole, isothiazole, pyrazole, imidazole, 1,2,3-triazole, pyridine, pyridazine, pyrimidine, pyrazine and the like.
The "5- or 6-membered aromatic ring" for ring A' is preferably a benzene ring.

The "5- or 6-membered aromatic ring" for ring A' may have 1 to 3 substituents at substitutable positions. Examples of such substituent include a halogen atom, a nitro group, a cyano group, an optionally substituted hydrocarbon group, an optionally substituted heterocyclic group, an optionally substituted hydroxy group, an optionally substituted mercapto group, an optionally substituted amino group, an acyl group and the like. In addition, when ring A' is pyridine, the pyridine may be N-oxidized.

As each of the "optionally substituted hydrocarbon group", "optionally substituted heterocyclic group", "optionally substituted hydroxy group", "optionally substituted mercapto group" and "optionally substituted amino group", those similar to the groups exemplified as the substituent of ring A are used.
The "acyl group" is as defined for (iv) "acyl group" exemplified as the substituent of (2) "optionally substituted hydroxy group" which is the substituent of ring A.

Ring A' is preferably an optionally substituted benzene ring, more preferably a benzene ring optionally substituted by 1 to 3 substituents selected from a halogen atom, an optionally halogenated C₁₋₆ alkyl group, a carboxyl group, a C₁₋₆ alkoxy-carbonyl group and the like.

Examples of the "6- to 8-membered nonaromatic nitrogen-containing heterocycle" for ring Ba' include a 6- to 8-membered nonaromatic nitrogen-containing heterocycle containing, as ring-constituting atom, at least one nitrogen atom and at least four carbon atoms and further, 1 to 3 atoms selected from a carbon atom, a nitrogen atom, an oxygen atom and a sulfur atom.
Specific examples of the "6- to 8-membered nonaromatic nitrogen-containing heterocycle" for ring Ba' include the following rings.

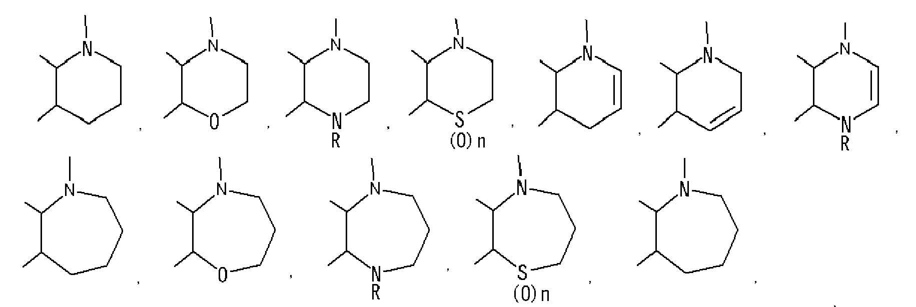

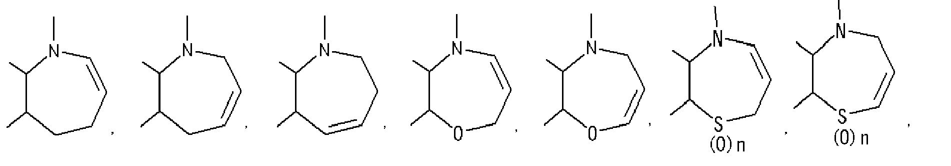

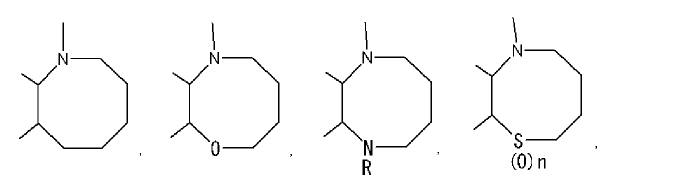

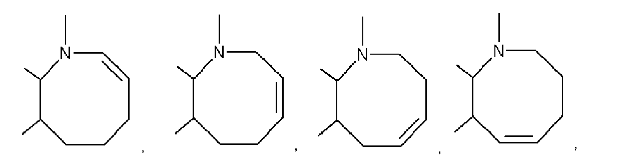

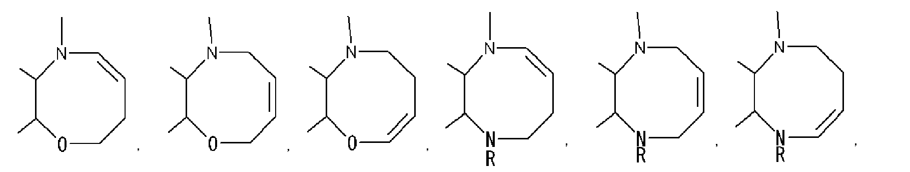

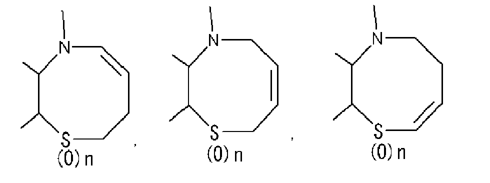

Ring Ba' is preferably a 6-membered ring, more preferably the following rings.

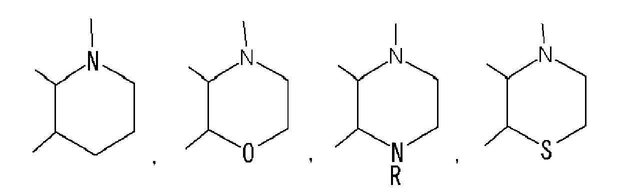

When Y' constituting ring Ba' is -NR-, and R is an optionally substituted C₁₋₆ alkyl group, the "optionally substituted C₁₋₆ alkyl group" is the substituent of ring Ba'.
In addition, when L²' constituting ring Ba' is each substituted chained C₂₋₅ alkylene group or substituted chained C₂₋₅ alkenylene group, the substituent of the chained C₂₋₅ alkylene group or chained C₂₋₅ alkenylene group is the substituent of ring Ba'.

W' and Y' are each independently a bond, -O-, -NR- wherein R is a hydrogen atom or an optionally substituted C₁₋₆ alkyl group, or -S(O)n- wherein n is an integer of 0 to 2.

The C₁₋₆ alkyl group for R may be substituted by 1 to 3 substituents selected from a halogen atom, a nitro group, a cyano group, a mono- or di-C₁₋₆ alkylamino group (e.g., amino, methylamino, dimethylamino, ethylamino), a hydroxy group, a C₁₋₆ alkoxy group (e.g., methoxy, ethoxy, propoxy), a formyloxy group, a C₁₋₆ alkyl-carbonyloxy group (e.g., acetyloxy), a C₁₋₆ alkylthio group (e.g., methylthio, ethylthio), a carboxyl group, a C₁₋₆ alkoxy-carbonyl group (e.g., methoxycarbonyl, ethoxycarbonyl, propoxycarbonyl, tert-butoxycarbonyl), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), an oxo group, a formylamino group, a C₁₋₆ alkoxy-carbonylamino group (e.g., methoxycarbonylamino, ethoxycarbonylamino, tert-butoxycarbonylamino), a dioxoisoindolyl group and the like.
R is preferably a hydrogen atom; or a C₁₋₆ alkyl group optionally substituted by 1 to 3 substituents selected from a cyano group, an amino group, a hydroxy group, a C₁₋₆ alkyl-carbonyloxy group (e.g., acetyloxy), a carboxyl group, a C₁₋₆ alkoxy-carbonyl group (e.g., methoxycarbonyl, ethoxycarbonyl, propoxycarbonyl, tert-butoxycarbonyl), a carbamoyl group, an oxo group, a formylamino group and a C₁₋₆ alkoxy-carbonylamino group (e.g., methoxycarbonylamino, ethoxycarbonylamino, tert-butoxycarbonylamino), more preferably a hydrogen atom or a C₁₋₆ alkyl group (e.g., methyl, ethyl, propyl). Particularly preferred is methyl.

W' is preferably -O-, -S- or -SO₂-, more preferably -O-.
Y' is preferably a bond, -O-, -NR- wherein R is a hydrogen atom or an optionally substituted C₁₋₆ alkyl group, or -S-, more preferably a bond or -NR- wherein R is preferably a hydrogen atom or a C₁₋₆ alkyl group.

Examples of the chained C₁₋₅ alkylene group of the "optionally substituted chained C₁₋₅ alkylene group" for L¹' include -CH₂-, - (CH₂)₂-, -(CH₂)₃-, -(CH₂)₄- and - (CH₂) ₅- .
Examples of the chained C₂₋₅ alkenylene group of the "optionally substituted chained C₂₋₅ alkenylene group" for L^{1'} include -CH=CH-,-CH₂-CH=CH-, -CH₂-CH=CH-CH₂-, -CH₂-CH₂-CH=CH-, - CH=CH-CH₂-CH₂-CH₂- and -CH₂-CH=CH-CH₂-CH₂-. A double bond contained in these alkenylene groups may be of any of cis and trans.
The chained C₁₋₅ alkylene group and chained C₂₋₅ alkenylene group may be substituted by 1 to 4 substituents at substitutable positions. Examples of the substituent include a halogen atom, a nitro group, a cyano group, an optionally halogenated C₁₋₆ alkyl group (e.g., methyl, ethyl, trifluoromethyl), a hydroxy group, an optionally halogenated C₁₋₆ alkoxy group (e.g., methoxy, ethoxy, trifluoromethoxy), a formyloxy group, a C₁₋₆ alkyl-carbonyloxy group (e.g., acetyloxy), an optionally halogenated C₁₋₆ alkylthio group (e.g., methylthio, ethylthio, trifluoromethylthio), a mono- or di-C₁₋₆ alkylamino group (e.g., amino, methylamino, dimethylamino, ethylamino), a carboxyl group, a C₁₋₆ alkoxy-carbonyl group (e.g., methoxycarbonyl, ethoxycarbonyl, propoxycarbonyl, tert-butoxycarbonyl), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), an oxo group, an optionally substituted C₇₋₁₁ aralkyl group (e.g., benzyl, phenethyl) and the like.
The "optionally substituted C₇₋₁₁ aralkyl group" may have 1 to 4 substituents at substitutable positions. Examples of such substituent include a halogen atom, a nitro group, a cyano group, a C₁₋₆ alkyl group (e.g., methyl, ethyl, propyl), a mono- or di-C₁₋₆ alkylamino group (e.g., methylamino, dimethylamino, ethylamino), a hydroxy group, a C₁₋₆ alkoxy group (e.g., methoxy, ethoxy, propoxy) and the like.
L^{1'} is preferably a chained C₁₋₅ alkylene group (preferably - CH₂-,-(CH₂)₃- etc.) optionally substituted by an optionally halogenated C₁₋₆ alkyl group or a chained C₃ alkenylene group (preferably-CH₂-CH=CH-), more preferably -CH₂- (a methylene group) and - (CH₂)₃- (a propylene group).

Examples of the "optionally substituted chained C₂₋₅ alkylene group" for L²' include those that a chained C₁₋₅ alkylene group of the "optionally substituted chained C₁₋₅ alkylene group" for the aforementioned L¹ is a C₂₋₅ alkylene group.
Examples of the "optionally substituted chained C₂₋₅ alkenylene group" for L^{2'} include those exemplified as the aforementioned L¹.
L^{2'} is preferably a chained C₂₋₅ alkylene group optionally substituted by substituent(s) selected from a halogen atom (preferably a fluorine atom), an optionally halogenated C₁₋₆ alkyl group, an optionally substituted C₇₋₁₁ aralkyl group, an optionally halogenated C₁₋₆ alkoxy group, a hydroxy group and an oxo group, more preferably an optionally substituted chained C₂₋₅ alkylene group (preferably - (CH₂)₂- (an ethylene group) and - (CH₂)₃- (a propylene group) optionally substituted by 1 to 4 substituents selected from an optionally halogenated C₁₋₆ alkyl group and an optionally substituted C₇₋₁₁ aralkyl group) .

Examples of the cyclic group of the "optionally substituted cyclic group" for Ar' include a C₆₋₁₄ aryl group, a nonaromatic cyclic hydrocarbon group, a heterocyclic group and the like.
Here, examples of the "heterocyclic group" include those similar to the "heterocyclic group" of the "optionally substituted heterocyclic group" which is the substituent of the aforementioned ring A.
The C₆₋₁₄ aryl group is preferably a phenyl group or a bicyclic benzene fused ring group selected from an indanyl group, a naphthyl group, a dihydronaphthyl group, a tetrahydronaphthyl group and the like, more preferably phenyl, indanyl and tetrahydronaphthyl.
Preferable examples of the heterocyclic group include bicyclic benzene fused ring groups such as benzofuryl, isobenzofuryl, dihydrobenzofuryl, dihydroisobenzofuryl, benzo[b]thienyl, indolyl, isoindolyl, 1H-indazolyl, benzimidazolyl, benzoxazolyl, 1,2-benzoisooxazolyl, benzothiazolyl, 1,2-benzoisothiazolyl, 1H-benzotriazolyl, quinolinyl, isoquinolinyl, dihydroquinolinyl, dihydroisoquinolinyl, tetrahydroquinolinyl, tetrahydroisoquinolinyl, cinnolinyl, quinazolinyl, quinoxalinyl, phthalazinyl, indolizinyl, isochromanyl, chromanyl, indolinyl, isoindolinyl, isochromenyl, chromenyl, benzodioxolyl and the like.
Examples of the nonaromatic cyclic hydrocarbon group include a C₃₋₁₀ cycloalkyl group, a C₃₋₁₀ cycloalkenyl group and the like, which is each optionally condensed with a benzene ring.
Examples of the C₃₋₁₀ cycloalkyl group include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and the like.
Examples of the C₃₋₁₀ cycloalkenyl group include 2-cyclopenten-1-yl, 3-cyclopenten-1-yl, 2-cyclohexen-1-yl, 3-cyclohexen-1-yl and the like.

The cyclic group is preferably a phenyl group and a bicyclic benzene fused ring group, more preferably a phenyl group, an indanyl group, a tetrahydronaphthyl group and a 5-isoquinolinyl group.
The "cyclic group" for Ar' may have 1 to 5 substituents (preferably 1 to 3) at substitutable positions. Examples of the substituent include those exemplified as the substituent in substituent group A.
In addition, examples of the substituent of Ar' also include an oxo group, a hydroxyimino group, a C₁₋₆ alkoxyimino group (e.g., methoxyimino) and the like.
The substituent of Ar' is preferably a halogen atom, an optionally halogenated C₁₋₆ alkyl group (e.g., methyl, ethyl, tert-butyl, trifluoromethyl), a heterocyclic group (preferably benzoxazolyl), an optionally halogenated C₁₋₆ alkoxy group (e.g., methoxy, ethoxy, trifluoromethoxy), a C₇₋₁₄ aralkyloxy group (e.g., benzyloxy, phenethyloxy, phenylpropyloxy), an optionally halogenated C₁₋₆ alkylthio group (e.g., methylthio, ethylthio, trifluoromethylthio), a hydroxy group, a mercapto group, a cyano group, a carboxyl group, a formyl group, an optionally halogenated C₁₋₆ alkyl-carbonyl group (e.g., acetyl, propionyl, trifluoroacetyl), a C₆₋₁₄ aryl-carbonyl group (e.g., benzoyl), a heterocyclic group-carbonyl group (e.g., nicotinoyl, isonicotinoyl, pyrrolidinyl-carbonyl, piperidinyl-carbonyl, morpholinyl-carbonyl, piperazinyl-carbonyl, pyridyl-carbonyl, furyl-carbonyl, thienyl-carbonyl, pyrrolyl-carbonyl, oxazolyl-carbonyl, isoxazolyl-carbonyl, thiazolyl-carbonyl, isothiazolyl-carbonyl, pyrazinyl-carbonyl, quinolyl-carbonyl, isoquinolyl-carbonyl), a C₁₋₆ alkoxy-carbonyl group (e.g., methoxycarbonyl, ethoxycarbonyl, propoxycarbonyl), a mono- or di-C₁₋₆ alkylamino group (e.g., methylamino, ethylamino, dimethylamino, diethylamino), a formylamino group, an optionally halogenated C₁₋₆ alkyl-carbonylamino group (e.g., acetylamino, propionylamino, butyrylamino, trifluoroacetylamino), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), a C₆₋₁₄ aryl group (preferably phenyl), an oxo group, a hydroxyimino group, a C₁₋₆ alkoxyimino group (e.g., methoxyimino) and the like.

Ar' is preferably a phenyl group, an indanyl group, a tetrahydronaphthyl group and a 5-isoquinolinyl group, which is optionally substituted by 1 to 3 substituents selected from a halogen atom, an optionally halogenated C₁₋₆ alkyl group (e.g., methyl, ethyl, tert-butyl, trifluoromethyl), a heterocyclic group (preferably benzoxazolyl), an optionally halogenated C₁₋₆ alkoxy group (e.g., methoxy, ethoxy, trifluoromethoxy), a C₇₋₁₄ aralkyloxy group (e.g., benzyloxy, phenethyloxy, phenylpropyloxy), an optionally halogenated C₁₋₆ alkylthio group (e.g., methylthio, ethylthio, trifluoromethylthio), a hydroxy group, a mercapto group, a cyano group, a carboxyl group, a formyl group, an optionally halogenated C₁₋₆ alkyl-carbonyl group (e.g., acetyl, propionyl, trifluoroacetyl), a C₆₋₁₄ aryl-carbonyl group (e.g., benzoyl), a heterocyclic group-carbonyl group (preferably pyrrolidinocarbonyl), a C₁₋₆ alkoxy-carbonyl group (e.g., methoxycarbonyl, ethoxycarbonyl, propoxycarbonyl), a mono- or di-C₁₋₆ alkylamino group (e.g., methylamino, ethylamino, dimethylamino, diethylamino), a formylamino group, an optionally halogenated C₁₋₆ alkyl-carbonylamino group (e.g., acetylamino, propionylamino, butyrylamino, trifluoroacetylamino), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), a C₆₋₁₄ aryl group (preferably phenyl), an oxo group, a hydroxyimino group, a C₁₋₆ alkoxyimino group (e.g., methoxyimino) and the like.

Particularly, a fused ring compound represented by the formula

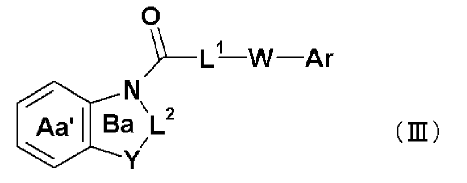

wherein ring Aa' is an optionally substituted benzene ring,
W is -O- or- S (O) nₐ- (nₐ is an integer of 0 to 2),
Y is a bond, -O-, -NR- (R is a hydrogen atom or an optionally substituted C₁₋₆ alkyl group (as defined for R in the above-mentioned Y' ) ) or -S(O)n_{b}- (n_{b} is an integer of 0 to 2),
L¹ is (1) a chained C₁₋₅ alkylene group optionally substituted by an optionally halogenated C₁₋₆ alkyl group or (2) an optionally substituted chained C₂₋₅ alkenylene group,
L² is (1) a chained C₂₋₅ alkylene group optionally substituted by substituent(s) selected from a fluorine atom, an optionally halogenated C₁₋₆ alkyl group, an optionally substituted C₇₋₁₁ aralkyl group, an optionally halogenated C₁₋₆ alkoxy group, a hydroxy group and an oxo group, or (2) an optionally substituted chained C₂₋₅ alkenylene group,
Ar is an optionally substituted phenyl group or an optionally substituted bicyclic benzene fused ring group, [hereinafter sometimes to be abbreviated as compound (III)] or a salt thereof is preferable.

Examples of the substituent of the "optionally substituted benzene ring" for ring Aa' include those exemplified as the substituent of the "optionally substituted 5- or 6-membered aromatic ring" for the aforementioned ring A'.

The "6- to 8-membered nonaromatic nitrogen-containing heterocycle" for ring Ba is as defined for the aforementioned ring Ba'.

L¹ is, of the "optionally substituted chained C₁₋₅ alkylene group" for L^{1'}, those that the substituent is an optionally halogenated C₁₋₆ alkyl group; or an optionally substituted chained C₂₋₅ alkenylene group (as defined for the "optionally substituted chained C₂₋₅ alkenylene group" for L^{1'}) .

L² is, of the "optionally substituted chained C₂₋₅ alkylene group" for L^{2'}, those that the substituent is selected from a fluorine atom, an optionally halogenated C₁₋₆ alkyl group, an optionally substituted C₇₋₁₁ aralkyl group, an optionally halogenated C₁₋₆ alkoxy group, a hydroxy group and an oxo group; or an optionally substituted chained C₂₋₅ alkenylene group (as defined for the "optionally substituted chained C₂₋₅ alkenylene group" for L^{2'}).

Examples of the substituent of the "optionally substituted phenyl group" for Ar include those exemplified as the substituent of the "optionally substituted cyclic group" for the aforementioned Ar'. Examples of the "optionally substituted bicyclic benzene fused ring group" for Ar further include those exemplified as the "optionally substituted cyclic group" for the aforementioned Ar'.

Of compound (III), particularly preferably are
a fused ring compound wherein ring Aa' is a benzene ring optionally substituted by 1 to 3 substituents selected from a halogen atom, an optionally halogenated C₁₋₆ alkyl group (e.g., methyl, ethyl, trifluoromethyl), a carboxyl group, a C₁₋₆ alkoxy-carbonyl group (e.g., methoxycarbonyl, ethoxycarbonyl) and the like; a fused ring compound wherein ring Ba is a 6-membered ring, particularly the following ring:

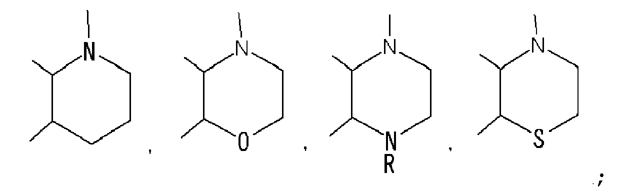

a fused ring compound wherein W is -O-; a fused ring compound wherein Y is a bond, -O-, -NR- (R is a hydrogen atom or an optionally substituted C₁₋₆ alkyl group) or -S- (Y is preferably a bond or -NR- (R is preferably a hydrogen atom or a C₁₋₆ alkyl group)); a fused ring compound wherein L¹ is a chained C₁₋₅ alkylene group optionally substituted by an optionally halogenated C₁₋₆ alkyl group, preferably a chained C₁₋₅ alkylene group (preferably,-CH₂- or-(CH₂)₃-); a fused ring compound wherein L² is preferably a chained C₂₋₅ alkylene group optionally substituted by substituent(s) selected from a fluorine atom, an optionally halogenated C₁₋₆ alkyl group (e.g., methyl, ethyl, propyl, trifluoromethyl), an optionally substituted C₇₋₁₁ aralkyl group (e.g., benzyl, phenethyl), an optionally halogenated C₁₋₆ alkoxy group (e.g., methoxy, ethoxy, trifluoromethoxy), a hydroxy group and an oxo group (preferably - (CH₂)₂- and -(CH₂)₃-); a fused ring compound wherein Ar is a phenyl group, an indanyl group, a tetrahydronaphthyl group or a 5-isoquinolinyl group, each of which is optionally substituted by 1 to 3 substituents selected from a halogen atom, an optionally halogenated C₁₋₆ alkyl group (e.g., methyl, ethyl, tert-butyl, trifluoromethyl), a heterocyclic group (preferably benzoxazolyl), an optionally halogenated C₁₋₆ alkoxy group (e.g., methoxy, ethoxy, trifluoromethoxy), a C₇₋₁₄ aralkyloxy group (e.g., benzyloxy, phenethyloxy, phenylpropyloxy), an optionally halogenated C₁₋₆ alkylthio group (e.g., methylthio, ethylthio, trifluoromethylthio), a hydroxy group, a mercapto group, a cyano group, a carboxyl group, a formyl group, an optionally halogenated C₁₋₆ alkyl-carbonyl group (e.g., acetyl, propionyl, trifluoroacetyl), a C₆₋₁₄ aryl-carbonyl group (e.g., benzoyl), a heterocyclic group-carbonyl group (preferably pyrrolidinyl-carbonyl (e.g., pyrrolidinocarbonyl)), a C₁₋₆ alkoxy-carbonyl group (e.g., methoxycarbonyl, ethoxycarbonyl, propoxycarbonyl), a mono- or di-C₁₋₆ alkylamino group (e.g., methylamino, ethylamino, dimethylamino, diethylamino), a formylamino group, an optionally halogenated C₁₋₆ alkyl-carbonylamino group (e.g., acetylamino, propionylamino, butyrylamino, trifluoroacetylamino), a carbamoyl group, a mono- or di-C₁₋₆ alkyl-carbamoyl group (e.g., N-methylcarbamoyl, N-ethylcarbamoyl, N,N-dimethylcarbamoyl, N,N-diethylcarbamoyl), C₆₋₁₄ aryl group (preferably phenyl), an oxo group, a hydroxyimino group, a C₁₋₆ alkoxyimino group (e.g., methoxyimino) etc., and the like.

Of compounds (III), moreover, preferred is a fused ring compound wherein ring Aa' is a benzene ring optionally substituted by a halogen atom, ring Ba is a 6-membered nonaromatic nitrogen-containing heterocycle, W is -O-, Y is -NR- (R is a hydrogen atom or an optionally substituted C₁₋₆ alkyl group), L¹ is a methylene group optionally substituted by an optionally halogenated C₁₋₆ alkyl group, L² is a chained C₂₋₅ alkylene group optionally substituted by substituent(s) selected from a C₁₋₆ alkyl group and an oxo group, and Ar is an optionally substituted bicyclic benzene fused ring group.

Furthermore, the present invention can provide an agent for the prophylaxis or treatment of irritable bowel syndrome (suitably, diarrhea type irritable bowel syndrome), comprising a compound represented by the following formula

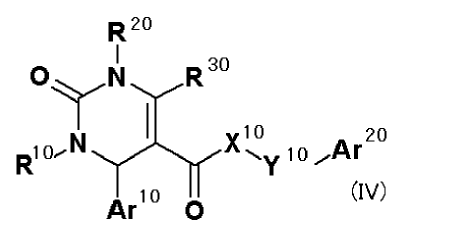

wherein R¹⁰, R²⁰ and R³⁰ are each a hydrogen atom or an optionally halogenated C₁₋₆ alkyl group, X¹⁰ is a bond, -O-, -NR⁰- (R⁰ is a hydrogen atom or a C₁₋₆ alkyl group) or -S-, Y¹⁰ is an optionally substituted C₁₋₅ alkylene group, Ar¹⁰ and Ar²⁰ are each an optionally substituted monocyclic aromatic group (hereinafter sometimes to be abbreviated as compound (IV)) or a salt thereof or a prodrug thereof. The compound is disclosed as a TGR5 agonist in patent document 2 (WO 2004/043468), and can be produced according to the description of the publication.

The definition of each symbol of compound (IV) is explained in the following.
As the "C₁₋₆ alkyl group" of the "optionally halogenated C₁₋₆ alkyl group" for R¹⁰, R²⁰ or R³⁰, a linear or branched C₁₋₆ alkyl group such as methyl, ethyl, propyl, isopropyl, butyl, isobutyl, sec-butyl, tert-butyl, pentyl, hexyl etc., and the like are used. Among them, a C₁₋₃ alkyl group such as methyl, ethyl, propyl, isopropyl and the like is preferable, and a methyl group is particularly preferable.

As the halogen atom optionally substituted for the C₁₋₆ alkyl group, a fluorine atom, a chlorine atom, a bromine atom, an iodine atom and the like are used, and a fluorine atom is particularly preferable.

As R¹⁰, R ²⁰ and R³⁰ , a hydrogen atom and a C₁₋₆ alkyl group are preferable, and a hydrogen atom and a methyl group are particularly preferable.

As the lower alkyl group for R⁰, a linear or branched C₁₋₆ alkyl group such as methyl, ethyl, propyl, isopropyl, butyl, isobutyl, sec-butyl, tert-butyl, pentyl, hexyl etc., and the like are used. Among them, a C₁₋₃ alkyl group such as methyl, ethyl, propyl and the like is preferable.

As the "C₁₋₅ alkylene group" of the "optionally substituted C₁₋₅ alkylene group" for Y¹⁰, methylene, ethylene, propylene, butylene and pentylene are used. Among them, a C₁₋₃ alkylene group such as methylene, ethylene, propylene and the like is preferable.

As the substituent that the "C₁₋₅ alkylene group" may have, (i) a nitro group, (ii) a hydroxy group, an oxo group, (iii) a cyano group, (iv) a carbamoyl group, (v) a mono- or di-C₁₋₆ alkyl-carbamoyl group (e.g., N-methylcarbamoyl, N-ethylcarbamoyl, N,N-dimethylcarbamoyl, N,N-diethylcarbamoyl and the like; the alkyl group is optionally substituted by a halogen atom, a hydroxy group, a C₁₋₆ alkoxy group etc.), a mono- or di-C₂₋₄ alkenyl-carbamoyl group (e.g., N-allylcarbamoyl and the like; the alkenyl group is optionally substituted by a halogen atom, a hydroxy group, a C₁₋₆ alkoxy group etc.), a mono- or di-phenyl-carbamoyl group (the phenyl group is optionally substituted by a halogen atom, C₁₋₆ alkyl optionally substituted by a halogen atom, a C₁₋₆ alkoxy group etc.), a mono- or di-benzyl-carbamoyl group (the benzyl group is optionally substituted by a halogen atom, C₁-₆ alkyl optionally substituted by a halogen atom, a C₁₋₆ alkoxy group etc.), a C₁₋₆ alkoxy-carbonyl-carbamoyl group, a C₁₋₆ alkylsulfonyl-carbamoyl group, a C₁₋₆ alkoxy-carbamoyl group, an amino-carbamoyl group, a mono- or di-C₁₋₆ alkylamino-carbamoyl group, a mono- or di-phenylamino-carbamoyl group, (vi) a carboxyl group, (vii) a C₁₋₆ alkoxy-carbonyl group (e.g., methoxycarbonyl, ethoxycarbonyl, propoxycarbonyl, isopropoxycarbonyl etc.), (viii) a halogen atom (e.g., fluorine, chlorine, bromine, iodine etc.), (ix) an optionally halogenated C₁₋₆ alkoxy group (e.g., methoxy, ethoxy, propoxy, isopropoxy etc.), a C₁₋₆ alkoxy group optionally substituted by a hydroxy group, a C₁₋₆ alkoxy group optionally substituted by a carboxyl group, a C₁₋₆ alkoxy group optionally substituted by a C₁₋₆ alkoxy-carbonyl group, a C₁₋₆ alkoxy-C₁₋₆ alkoxy group, a C₁₋₆ alkoxy-C₁₋₆ alkoxy-C₁₋₆ alkoxy group, (x) a phenoxy-C₁₋₆ alkyl group, a phenoxy-C₁₋₆ alkoxy group, a C₁₋₆ alkylcarbonyl-oxy group, a carbamoyloxy group, a mono- or di-C₁₋₆ alkyl-carbamoyloxy group, (xi) an optionally halogenated phenyl group, an optionally halogenated phenyl-C₁₋₆ alkyl group, an optionally halogenated phenyl-C₂₋₄ alkenyl group, an optionally halogenated phenoxy group (e.g., o-, m- or p-chlorophenoxy, o-, m- or p-bromophenoxy etc.), a pyridyloxy group, a C₃₋₁₀ cycloalkyl group, a C₃₋₁₀ cycloalkyl-C₁₋₆ alkoxy group, a C₃₋₁₀ cycloalkyl-C₁₋₆ alkyl group, (xii) an optionally halogenated C₁₋₆ alkyl group (e.g., methyl, ethyl, propyl, isopropyl, butyl etc.), an optionally halogenated C₂₋₆ alkenyl group (e.g., vinyl, allyl, 2-butenyl, 3-butenyl etc.), an optionally halogenated C₁₋₆ alkylthio group (e.g., methylthio, ethylthio, n-propylthio, isopropylthio, n-butylthio etc.), a C₁₋₆ alkyl group optionally substituted by a hydroxy group, a C₁₋₆ alkylthio group optionally substituted by a hydroxy group, (xiii) a mercapto group, a thioxo group, (xiv) a benzyloxy group or a benzylthio group, each of which is optionally substituted by substituent(s) selected from a halogen atom, a carboxyl group and a C₁₋₆ alkoxy-carbonyl group, (xv) an optionally halogenated phenylthio group, a pyridylthio group, a phenylthio-C₁₋₆ alkyl group, a pyridylthio-C₁₋₆ alkyl group, (xvi) an optionally halogenated C₁₋₆ alkylsulfinyl group (e.g., methylsulfinyl, ethylsulfinyl etc.), a phenylsulfinyl group, a phenylsulfinyl-C₁₋₆ alkyl group, (xvii) an optionally halogenated C₁₋₆ alkylsulfonyl group (e.g., methylsulfonyl, ethylsulfonyl etc.), a phenylsulfonyl group, a phenylsulfonyl-C₁₋₆ alkyl group, (xviii) an amino group, an aminosulfonyl group, a mono- or di-C₁₋₆ alkylaminosulfonyl group (e.g., methylaminosulfonyl, ethylaminosulfonyl, N,N-dimethylaminosulfonyl, N,N-diethylaminosulfonyl etc.; the alkyl group is optionally substituted by a halogen atom, a hydroxy group, a C₁₋₆ alkoxy group etc.), (xix) a C₁₋₁₀ acyl-amino group (e.g., C₁₋₆ alkanoylamino (e.g., formylamino, acetylamino, trifluoroacetylamino, propionylamino, pivaloylamino etc.), benzoylamino, C₁₋₆ alkylsulfonylamino (e.g., methanesulfonylamino, trifluoromethanesulfonylamino etc.), C₆₋₁₀ arylsulfonylamino (e.g., benzenesulfonylamino, toluenesulfonylamino etc.); C₁₋₁₀ acyl is optionally substituted by a halogen atom, a C₁₋₆ alkyl group, a C₁₋₆ alkoxy group, a hydroxy group, a carboxyl group etc.), benzyloxycarbonylamino, optionally halogenated C₁₋₆ alkoxycarbonylamino, a carbamoylamino group, a mono- or di-C₁₋₆ alkylcarbamoylamino group, (xx) a mono- or di-C₁₋₆ alkylamino group (e.g., methylamino, ethylamino, dimethylamino, diethylamino etc.; the alkyl group is optionally substituted by a halogen atom, a hydroxy group, a C₁₋₆ alkoxy group etc.), a mono- or di-C₁₋₆ alkanoylamino group (e.g., formylamino, acetylamino etc.; the alkanoyl group is optionally substituted by a halogen atom, a hydroxy group, a C₁₋₆ alkoxy group etc.), phenylamino, benzylamino, C₁₋₆ alkyl (benzyl) amino, C₁₋₆ alkanoyl (benzyl) amino, (xxi) a 4- to 8-membered cyclic amino group (e.g., 1-azetidinyl, 1-pyrrolidinyl, piperidino, morpholino, thiomorpholino, 1-piperazinyl etc.), a 4-to 8-membered cyclic amino-carbonyl group (e.g., 1-azetidinylcarbonyl, 1-pyrrolidinylcarbonyl, piperidinocarbonyl, morpholinocarbonyl, thiomorpholinocarbonyl, 1-piperazinylcarbonyl etc.), a 4- to 8-membered cyclic amino-carbonyl-oxy group (e.g., 1-pyrrolidinylcarbonyloxy, piperidinocarbonyloxy, morpholinocarbonyloxy, thiomorpholinocarbonyloxy, 1-piperazinylcarbonyloxy etc.), a 4- to 8-membered cyclic amino-carbonyl-amino group (e.g., 1-pyrrolidinylcarbonylamino, piperidinocarbonylamino, morpholinocarbonylamino, thiomorpholinocarbonylamino, 1-piperazinylcarbonylamino etc.), a 4-to 8-membered cyclic amino-sulfonyl group (e.g., 1-pyrrolidinylsulfonyl, piperidinosulfonyl, morpholinosulfonyl, thiomorpholinosulfonyl, 1-piperazinylsulfonyl etc.), a 4- to 6-membered cyclic amino-C₁₋₆ alkyl group, (xxii) a C₁₋₆ acyl group or a benzoyl group, each optionally substituted by substituent(s) selected from a halogen atom, a Cᵥ alkyl group, a C₁₋₆ alkoxy group, a carboxyl group and a C₁₋₆ alkoxy-carbonyl group (e.g., optionally halogenated C₂₋₆ alkanoyl such as formyl, acetyl etc., etc.), (xxiii) a 4- to 10-membered heterocyclic group containing at least one (preferably 1 to 4, more preferably 1 or 2) of 1 to 3 kinds (preferably 1 or 2 kinds) of hetero atoms selected from an oxygen atom, a sulfur atom and a nitrogen atom and the like (e.g., 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 1,2,3- or 1,2,4-triazolyl, 1H- or 2H-tetrazolyl, 2-, 3- or 4-pyridyl, 2-, 4- or 5-pyrimidyl, 3- or 4-pyridazinyl, quinolyl, isoquinolyl, indolyl and the like; the heterocyclic group is optionally substituted by a C₁₋₆ alkyl group etc.), (xxiv) a 5- to 10-membered heterocyclic group-carbonyl group containing at least one (preferably 1 to 4, more preferably 1 or 2) of 1 to 3 kinds (preferably 1 or 2 kinds) of hetero atoms selected from an oxygen atom, a sulfur atom and a nitrogen atom and the like (e.g., 2- or 3-thienylcarbonyl, 2- or 3-furylcarbonyl, 3-, 4- or 5-pyrazolylcarbonyl, 2-, 4- or 5-thiazolylcarbonyl, 3-, 4- or 5-isothiazolylcarbonyl, 2-, 4- or 5-oxazolylcarbonyl, 1,2,3- or 1,2,4-triazolylcarbonyl, 1H- or 2H-tetrazolylcarbonyl, 2-, 3- or 4-pyridylcarbonyl, 2-, 4- or 5-pyrimidylcarbonyl, 3- or 4-pyridazinylcarbonyl, quinolylcarbonyl, isoquinolylcarbonyl, indolylcarbonyl and the like; the heterocyclic group is optionally substituted by a C₁₋₆ alkyl group etc.), (xxv) a hydroxyimino group, a C₁₋₆ alkoxyimino group, a C₆₋₁₄ aryl group (e.g., 1- or 2-naphthyl etc.) and (xxvi) an optionally halogenated linear or branched C₁₋₆ alkylenedioxy group (e.g., methylenedioxy, ethylenedioxy, propylenedioxy, tetrafluoroethylenedioxy etc.) (hereinafter to be also referred to as substituent group C) and the like are used.

As the "monocyclic aromatic group" of the "optionally substituted monocyclic aromatic group" for Ar¹⁰ or Ar²⁰, a monocyclic aromatic hydrocarbon group or a monocyclic aromatic heterocyclic group is used.

As the monocyclic aromatic hydrocarbon group, a monocyclic C₆₋₈ aryl group such as a phenyl group etc., and the like are used, and a phenyl group is particularly preferable.

As the monocyclic aromatic heterocyclic group, for example, a 5- to 8-membered monocyclic aromatic heterocyclic group containing, as ring-constituting atom (ring atom), at least one (preferably 1 to 4, more preferably 1 or 2) of 1 to 3 kinds (preferably 1 or 2 kinds) of hetero atoms selected from an oxygen atom, a sulfur atom, a nitrogen atom etc., and the like is used. Specifically, a 5- or 6-membered monocyclic aromatic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like are preferably used. Particularly, a thienyl group is preferable.

Examples of the substituent that the "monocyclic aromatic group" for Ar¹⁰ or Ar²⁰ may have include a halogen atom (e.g., fluorine, chlorine, bromine, iodine etc.), a nitro group, a cyano group, an optionally substituted hydrocarbon group, an optionally substituted heterocyclic group, an optionally substituted hydroxy group, an optionally substituted thiol group, a substituted sulfinyl group, a substituted sulfonyl group, an optionally substituted amino group, an acyl group, an optionally substituted carbamoyl group, an optionally esterified carboxyl group or C₁₋₃ alkylenedioxy group (hereinafter to be also referred to as substituent group D) and the like.

Examples of the "hydrocarbon group" of the "optionally substituted hydrocarbon group" as the substituent that the "monocyclic aromatic group" may have include an alkyl group, a cycloalkyl group, an alkenyl group, a cycloalkenyl group, an alkynyl group, an aralkyl group, an aryl group and the like.

As the "alkyl group", for example, a "linear or branched C₁₋₁₅ alkyl group" such as methyl, ethyl, propyl, isopropyl, butyl, isobutyl, sec-butyl, tert-butyl, pentyl, hexyl, heptyl, octyl, nonyl, decyl, undecyl, tridecyl, tetradecyl, pentadecyl etc., and the like are used. A C₁₋₈ alkyl group is preferably used, a C₁₋₆ alkyl group is more preferably used, and a C₁₋₄ alkyl group is further preferably used.

As the "cycloalkyl group", for example, a "C₃₋₁₀ cycloalkyl group" such as cyclopropyl, cyclobutyl, cyclopentyl, cyclohexyl, cycloheptyl, cyclooctyl, adamantyl etc., and the like are used. A C₃₋₈ cycloalkyl group is more preferably used, and a C₅₋₇ cycloalkyl group is further preferably used.

As the "alkenyl group", for example, a "C₂₋₁₈ alkenyl group" such as vinyl, allyl, isopropenyl, 3-butenyl, 3-octenyl, 9-octadecenyl etc., and the like are used. A C₂₋₆ alkenyl group group is more preferably used, and a C₂₋₄ alkenyl group is further preferably used.

As the "cycloalkenyl group", for example, a "C₃₋₁₀ cycloalkenyl group" such as cyclopropenyl, cyclobutenyl, cyclopentenyl, cyclohexenyl, cycloheptenyl, cyclooctenyl etc., and the like are used, and a C₃₋₈ cycloalkenyl group is more preferable and a C₅₋₇ cycloalkenyl group is further preferable.

As the "alkynyl group", for example, a "C₂₋₈ alkynyl group" such as ethynyl, 1-propynyl, propargyl, 1-butynyl, 2-butynyl, 1-pentynyl, 2-pentynyl, 3-pentynyl etc., and the like are used, and a C₂₋₆ alkynyl group is more preferable and a C₂₋₄ alkynyl group is further preferable.

As the "aralkyl group", a C₇₋₁₆ aralkyl group and the like are used. Specifically, for example, a phenyl-C₁₋₆ alkyl group such as benzyl, phenethyl, 3-phenylpropyl, 4-phenylbutyl and the like, and a naphthyl-C₁₋₆ alkyl group such as (1-naphthyl)methyl, 2-(1-naphthyl)ethyl, 2-(2-naphthyl)ethyl etc., and the like are used.

As the "aryl group", for example, a monocyclic, bicyclic or tricyclic aromatic C₆₋₁₄ aryl group such as phenyl, 1-naphthyl, 2-naphthyl, phenanthryl, anthryl and the like, a biphenyl group, a tolyl group and the like are used. Preferred is a C₆₋₁₀ aryl group such as phenyl, naphthyl and the like, and more preferred is phenyl.

As the substituent optionally possessed by the "hydrocarbon group" of the "optionally substituted hydrocarbon group" as the substituent that the "monocyclic aromatic group" may have, for example, (i) a hydroxy group, (ii) an oxo group, (iii) a cyano group, (iv) a carbamoyl group, (v) a mono- or di-C₁₋₆ alkyl-carbamoyl group (e.g., N-methylcarbamoyl, N-ethylcarbamoyl, N,N-dimethylcarbamoyl, N,N-diethylcarbamoyl etc.; the alkyl group is optionally substituted by a halogen atom, a hydroxy group, a C₁₋₆ alkoxy group etc.), a mono- or di-C₂₋₄ alkenyl-carbamoyl group (e.g., N-allylcarbamoyl etc.; the alkenyl group is optionally substituted by a halogen atom, a hydroxy group, a C₁₋₆ alkoxy group etc.), a mono- or di-phenyl-carbamoyl group (the phenyl group is optionally substituted by a halogen atom, a C₁₋₆ alkyl optionally substituted by a halogen atom, a C₁₋₆ alkoxy group etc.), a mono- or di-benzyl-carbamoyl group (the benzyl group is optionally substituted by a halogen atom, C₁₋₆ alkyl optionally substituted by a halogen atom, a C₁₋₆ alkoxy group etc.), a C₁₋₆ alkoxy-carbonyl-carbamoyl group, a C₁₋₆ alkylsulfonyl-carbamoyl group, a C₁₋₆ alkoxy-carbamoyl group, an amino-carbamoyl group, a mono- or di-C₁₋₆ alkylamino-carbamoyl group, a mono- or di-phenylamino-carbamoyl group, (vi) a carboxyl group, (vii) a C₁₋₆ alkoxy-carbonyl group (e.g., methoxycarbonyl, ethoxycarbonyl, propoxycarbonyl, isopropoxycarbonyl etc.), (viii) a halogen atom (e.g., fluorine, chlorine, bromine, iodine etc.), (ix) an optionally halogenated C₁₋₆ alkoxy group (e.g., methoxy, ethoxy, propoxy, isopropoxy etc.), a C₁₋₆ alkoxy group optionally substituted by a hydroxy group, a C₁₋₆ alkoxy group optionally substituted by a carboxyl group, a C₁₋₆ alkoxy group optionally substituted by a C₁₋₆ alkoxy-carbonyl group, a C₁₋₆ alkoxy-C₁₋₆ alkoxy group, a C₁₋₆ alkoxy-C₁₋₆ alkoxy-C₁₋₆ alkoxy group, (x) a phenoxy-C₁₋₆ alkyl group, a phenoxy-C₁₋₆ alkoxy group, a C₁₋₆ alkylcarbonyl-oxy group, a carbamoyloxy group, a mono- or di-C₁₋₆ alkyl-carbamoyloxy group, (xi) an optionally halogenated phenyl group, an optionally halogenated phenyl-C₁₋₆ alkyl group, an optionally halogenated phenyl-C₂₋₄ alkenyl group, an optionally halogenated phenoxy group (e.g., o-, m- or p-chlorophenoxy, o-, m- or p-bromophenoxy etc.), a pyridyloxy group, a C₃₋₁₀ cycloalkyl group, a C₃₋₁₀ cycloalkyl-C₁₋₆ alkoxy group, a C₃₋₁₀ cycloalkyl-C₁₋₆ alkyl group, (xii) an optionally halogenated C₁₋₆ alkyl group (e.g., methyl, ethyl, propyl, isopropyl, butyl and the like), an optionally halogenated C₂₋₆ alkenyl group (e.g., vinyl, allyl, 2-butenyl, 3-butenyl etc.), an optionally halogenated C₁₋₆ alkylthio group (e.g., methylthio, ethylthio, n-propylthio, isopropylthio, n-butylthio etc.), a C₁₋₆ alkyl group optionally substituted by a hydroxy group, a C₁₋₆ alkylthio group optionally substituted by a hydroxy group, (xiii) a mercapto group, (xiv) a thioxo group, (xv) a benzyloxy group or a benzylthio group, each of which is optionally substituted by substituent(s) selected from a halogen atom, a carboxyl group and a C₁₋₆ alkoxy-carbonyl group, (xvi) an optionally halogenated phenylthio group, a pyridylthio group, a phenylthio-C₁₋₆ alkyl group, a pyridylthio-C₁₋₆ alkyl group, (xvii) an optionally halogenated C₁₋₆ alkylsulfinyl group (e.g., methylsulfinyl, ethylsulfinyl etc.), a phenylsulfinyl group, a phenylsulfinyl-C₁₋₆ alkyl group, (xviii) an optionally halogenated C₁₋₆ alkylsulfonyl group (e.g., methylsulfonyl, ethylsulfonyl etc.), a phenylsulfonyl group, a phenylsulfonyl-C₁₋₆ alkyl group, (xix) an amino group, an aminosulfonyl group, a mono- or di-C₁₋₆ alkylaminosulfonyl group (e.g., methylaminosulfonyl, ethylaminosulfonyl, N,N-dimethylaminosulfonyl, N,N-diethylaminosulfonyl etc.; the alkyl group is optionally substituted by a halogen atom, a hydroxy group, a C₁₋₆ alkoxy group etc.), (xx) a C₁₋₁₀ acyl-amino group (e.g., C₁₋₆ alkanoylamino (e.g., formylamino, acetylamino, trifluoroacetylamino, propionylamino, pivaloylamino etc.), benzoylamino, C₁₋₆ alkylsulfonylamino (e.g., methanesulfonylamino, trifluoromethanesulfonylamino etc.), C₆₋₁₀ arylsulfonylamino (e.g., benzenesulfonylamino, toluenesulfonylamino etc.); C₁₋₁₀ acyl is optionally substituted by a halogen atom, a C₁₋₆ alkyl group, a C₁₋₆ alkoxy group, a hydroxy group, a carboxyl group etc.), benzyloxycarbonylamino, optionally halogenated C₁₋₆ alkoxycarbonylamino, a carbamoylamino group, a mono- or di-C₁₋₆ alkylcarbamoylamino group, (xxi) a mono- or di-C₁₋₆ alkylamino group (e.g., methylamino, ethylamino, dimethylamino, diethylamino etc.; the alkyl group is optionally substituted by a halogen atom, a hydroxy group, a C₁₋₆ alkoxy group etc.), a mono- or di-C₁₋₆ alkanoylamino group (e.g., formylamino, acetylamino etc.; the alkanoyl group is optionally substituted by a halogen atom, a hydroxy group, a C₁₋₆ alkoxy group etc.), phenylamino, benzylamino, C₁₋₆ alkyl (benzyl) amino, C₁₋₆ alkanoyl (benzyl) amino, (xxii) a 4- to 8-membered cyclic amino group (e.g., 1-azetidinyl, 1-pyrrolidinyl, piperidino, morpholino, thiomorpholino, 1-piperazinyl etc.), a 4-to 8-membered cyclic amino-carbonyl group (e.g., 1-azetidinylcarbonyl, 1-pyrrolidinylcarbonyl, piperidinocarbonyl, morpholinocarbonyl, thiomorpholinocarbonyl, 1-piperazinylcarbonyl etc.), a 4- to 8-membered cyclic amino-carbonyl-oxy group (e.g., 1-pyrrolidinylcarbonyloxy, piperidinocarbonyloxy, morpholinocarbonyloxy, thiomorpholinocarbonyloxy, 1-piperazinylcarbonyloxy etc.), a 4- to 8-membered cyclic amino-carbonyl-amino group (e.g., 1-pyrrolidinylcarbonylamino, piperidinocarbonylamino, morpholinocarbonylamino, thiomorpholinocarbonylamino, 1-piperazinylcarbonylamino etc.), a 4-to 8-membered cyclic amino-sulfonyl group (e.g., 1-pyrrolidinylsulfonyl, piperidinosulfonyl, morpholinosulfonyl, thiomorpholinosulfonyl, 1-piperazinylsulfonyl etc.), a 4- to 8-membered cyclic amino-C₁₋₆ alkyl group, (xxiii) a C₁₋₆ acyl group (e.g., formyl, optionally halogenated C₂₋₆ alkanoyl such as acetyl etc., and the like) or a benzoyl group, each of which is optionally substituted by substituent(s) selected from a halogen atom, a C₁₋₆ alkyl group, a C₁₋₆ alkoxy group, a carboxyl group and a C₁₋₆ alkoxy-carbonyl group , (xxiv) a 4- to 10-membered heterocyclic group containing at least one (preferably 1 to 4, more preferably 1 or 2) of 1 to 3 kinds (preferably 1 or 2 kinds) of hetero atoms selected from an oxygen atom, a sulfur atom and a nitrogen atom and the like (e.g., 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 1,2,3- or 1,2,4-triazolyl, 1H- or 2H-tetrazolyl, 2-, 3- or 4-pyridyl, 2-, 4- or 5-pyrimidyl, 3- or 4-pyridazinyl, quinolyl, isoquinolyl, indolyl etc.; the heterocyclic group is optionally substituted by a C₁₋₆ alkyl group etc.), (xxv) a 4- to 10-membered heterocyclic group-carbonyl group containing at least one (preferably 1 to 4, more preferably 1 or 2) of 1 to 3 kinds (preferably 1 or 2 kinds) of hetero atoms selected from an oxygen atom, a sulfur atom, a nitrogen atom and the like (e.g., 2- or 3-thienylcarbonyl, 2- or 3-furylcarbonyl, 3-, 4- or 5-pyrazolylcarbonyl, 2-, 4- or 5-thiazolylcarbonyl, 3-, 4- or 5-isothiazolylcarbonyl, 2-, 4- or 5-oxazolylcarbonyl, 1,2,3- or 1,2,4-triazolylcarbonyl, 1H- or 2H-tetrazolylcarbonyl, 2-, 3- or 4-pyridylcarbonyl, 2-, 4- or 5-pyrimidylcarbonyl, 3- or 4-pyridazinylcarbonyl, quinolylcarbonyl, isoquinolylcarbonyl, indolylcarbonyl etc.; the heterocyclic group is optionally substituted by a C₁₋₆ alkyl group etc.), (xxvi) a hydroxyimino group, a C₁₋₆ alkoxyimino group, a C₆₋₁₄ aryl group (e.g., 1- or 2-naphthyl etc.) and (xxvii) an optionally halogenated linear or branched C₁₋₆ alkylenedioxy group (e.g., methylenedioxy, ethylenedioxy, propylenedioxy, tetrafluoroethylenedioxy etc.) (hereinafter to be also referred to as substituent group E) and the like are used. The "hydrocarbon group" may have 1 to 5 substituents therefrom at substitutable positions. When two or more substituents are present, they may be the same or different.

Examples of the "heterocyclic group" of the "optionally substituted heterocyclic group" as the substituent that the "monocyclic aromatic group" may have include a 4- to 16-membered mono- to tricyclic aromatic heterocyclic group, a saturated or unsaturated nonaromatic heterocyclic group (an aliphatic heterocyclic group) and the like, which containing, as ring-constituting atom (ring atom), at least one (preferably 1 to 4,
more preferably 1 or 2) hetero atoms of 1 to 3 kinds (preferably 1 or 2 kinds) selected from an oxygen atom, a sulfur atom, a nitrogen atom and the like,.

Examples of the "aromatic heterocyclic group" include a 5- or 6-membered monocyclic aromatic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like, and a 8- to 16-membered (preferably, 8- to 12-membered) aromatic fused heterocyclic group such as benzofuranyl, isobenzofuranyl, benzo[b]thienyl, indolyl, isoindolyl, 1H-indazolyl, benzimidazolyl, benzoxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, buterizinyl, carbazolyl, α-carbolinyl, β-carbolinyl, γ-carbolinyl, acrydinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, benzo[1,2,5]thiadiazolyl, benzo[1,2,5]oxadiazolyl and the like (preferably, heterocycle wherein 1 or 2, preferably 1, ring selected from the aforementioned 5- or 6-membered monocyclic aromatic heterocyclic group is condensed with 1 or 2, preferably 1, benzene ring or heterocycle wherein the same or different 2 or 3, preferably 2, heterocycles selected from the aforementioned 5- or 6-membered monocyclic aromatic heterocyclic group are condensed, more preferably heterocycle wherein the aforementioned 5- or 6-membered monocyclic aromatic heterocyclic group is condensed with a benzene ring, and the like.

Examples of the "nonaromatic heterocyclic group" include a 3-to 8-membered (preferably 5- or 6-membered) saturated or unsaturated (preferably saturated) monocyclic nonaromatic heterocyclic group (an aliphatic monocyclic heterocyclic group) such as oxiranyl, azetidinyl, oxetanyl, thietanyl, pyrrolidinyl (preferably, 1-pyrrolidinyl), tetrahydrofuryl, thioranyl, piperidinyl (preferably, 1-piperidinyl or 4-piperidinyl), tetrahydropyranyl, morpholinyl, thiomorpholinyl, piperazinyl and the like, a heterocyclic group wherein 1 or 2, preferably 1, heterocyclic group selected from the aforementioned monocyclic nonaromatic heterocyclic group is condensed with 1 or 2, preferably 1, benzene ring, such as 2,3-dihydroindolyl, 1,3-dihydroisoindolyl and the like, a heterocyclic group wherein 1 or 2, preferably 1, heterocyclic group selected from the aforementioned monocyclic nonaromatic heterocyclic group is condensed with 1 or 2, preferably 1, heterocycle of the aforementioned 5- or 6-membered monocyclic aromatic heterocyclic group, or a nonaromatic heterocyclic group wherein a part or whole of the double bond of the aforementioned monocyclic aromatic heterocycle or the aforementioned fused aromatic heterocycle is saturated, such as 1,2,3,4-tetrahydroquinolyl, 1,2,3,4-tetrahydroisoquinolyl and the like, and the like.

As the "heterocyclic group" of the "optionally substituted heterocyclic group", a 5- or 6-membered monocyclic aromatic heterocyclic group and the like are preferable.

As the substituent that the "heterocyclic group" may have, a similar number of groups similar to the substituents that the "hydrocarbon group" of the "optionally substituted hydrocarbon group" as the substituent optionally possessed by the "monocyclic aromatic group" optionally has, and the like are used.

Examples of the "optionally substituted amino group", "optionally substituted hydroxy group" and "optionally substituted thiol group" as the substituent that the "monocyclic aromatic group" may have respectively include an amino group optionally having substituent(s) such as an optionally substituted hydrocarbon group, an acyl group, an optionally esterified carboxyl group, an optionally substituted carbamoyl group, an optionally substituted heterocyclic group or the like, a hydroxy group, a thiol group and the like. As the "hydrocarbon group" of the "optionally substituted hydrocarbon group" and the "heterocyclic group" of the "optionally substituted heterocyclic group", groups similar to the "hydrocarbon group" of the "optionally substituted hydrocarbon group" and the "heterocyclic group" of the "optionally substituted heterocyclic group", each as the substituent that the "monocyclic aromatic group" may have, and the like are respectively used.

In addition, as the "acyl group" and the "optionally esterified carboxyl group" as the substituent, groups similar to the "optionally esterified carboxyl group" and "acyl group" as the substituents that the below-mentioned "monocyclic aromatic group" may have and the like are respectively used.

As the "optionally substituted carbamoyl group", groups similar to the "optionally substituted carbamoyl group" as the substituent that the below-mentioned "monocyclic aromatic group" may have and the like are used.

Moreover, as the substituent of the "optionally substituted hydrocarbon group" and the "optionally substituted heterocyclic group", a similar number of groups similar to the substituent of the "optionally substituted hydrocarbon group" and the "optionally substituted heterocyclic group" as the substituent that the "monocyclic aromatic group" optionally has and the like are used. Among them, "amino group", "hydroxy group" and "thiol group" each optionally having substituent(s) such as
lower alkyl (e.g., C₁₋₆ alkyl such as methyl, ethyl, propyl, isopropyl, butyl, isobutyl, tert-butyl, pentyl, hexyl etc., and the like) optionally substituted by substituent(s) selected from a halogen atom (e.g., fluorine, chlorine, bromine, iodine etc.), an optionally halogenated C₁₋₆ alkoxy (e.g., methoxy, ethoxy, trifluoromethoxy, 2,2,2-trifluoroethoxy, trichloromethoxy, 2,2,2-trichloroethoxy etc.), optionally substituted phenyl (preferably, phenyl optionally substituted by substituent(s) selected from an optionally halogenated C₁₋₆ alkyl group, an optionally halogenated C₁₋₆ alkoxy group, a carboxyl group and a halogen atom, etc.) and a 5- to 10-membered heterocyclic group containing at least one (preferably 1 to 4, more preferably 1 or 2) of 1 to 3 kinds (preferably 1 or 2 kinds) of hetero atoms selected from an oxygen atom, a sulfur atom, a nitrogen atom and the like (e.g., 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 1,2,3-
or 1,2,4-triazolyl, 1H- or 2H-tetrazolyl, 2-, 3- or 4-pyridyl, 2-, 4- or 5-pyrimidyl, 3- or 4-pyridazinyl, quinolyl, isoquinolyl, indolyl etc.; the heterocyclic group is optionally substituted by a C₁₋₄ alkyl group etc.),
acyl (C₁₋₆ alkanoyl (e.g., formyl, acetyl, propionyl, pivaloyl etc.), benzoyl, C₁₋₆ alkylsulfonyl (e.g., methanesulfonyl etc.), benzenesulfonyl etc.),
optionally halogenated C₁₋₆ alkoxycarbonyl (e.g., methoxycarbonyl, ethoxycarbonyl, trifluoromethoxycarbonyl, 2,2,2-trifluoroethoxycarbonyl, trichloromethoxycarbonyl, 2,2,2-trichloroethoxycarbonyl etc.),
C₁₋₆ alkoxycarbonyl optionally substituted by phenyl (e.g., benzyloxycarbonyl etc.),
an optionally substituted carbamoyl group (e.g., a carbamoyl group optionally substituted by 1 or 2 substituents such as a lower (C₁₋₆) alkyl group, a phenyl group etc., such as carbamoyl, N-methylcarbamoyl, N,N-dimethylcarbamoyl, phenylcarbamoyl etc., and the like),
a heterocyclic group (group similar to the "heterocyclic group" of the "optionally substituted heterocyclic group", each as the substituent that the "monocyclic aromatic group" optionally has, etc.) and the like, and the like. In addition, two substituents of N,N-disubstituted amino may form a "cyclic amino group" together with the nitrogen atom, and as the "cyclic amino group", for example, a 3- to 8-membered (preferably 5- or 6-membered) cyclic amino group such as 1-azetidinyl, 1-pyrrolidinyl, piperidino, morpholino, thiomorpholino (sulfur atom may be oxidized), 1-piperazinyl, 1-piperazinyl optionally having, at the 4-position, lower alkyl (e.g., C₁₋₆ alkyl such as methyl, ethyl, propyl, isopropyl, butyl, tert-butyl, pentyl, hexyl etc., and the like), aralkyl (e.g., C₇₋₁₀ aralkyl such as benzyl, phenethyl etc., and the like), aryl (e.g., C₆₋₁₀ aryl such as phenyl, 1-naphthyl, 2-naphthyl etc., and the like) and the like, and the like, and the like are used.

The "substituted sulfinyl group" and "substituted sulfonyl group" as the substituent that the "monocyclic aromatic group" may have respectively mean a sulfinyl group or sulfonyl group substituted by substituent(s) such as an "optionally substituted hydroxy group", an "optionally substituted amino group", an "optionally substituted hydrocarbon group" or an "optionally substituted heterocyclic group" and the like.

As the "hydrocarbon group" of the "optionally substituted hydrocarbon group", groups similar to the "hydrocarbon group" of the "optionally substituted hydrocarbon group" as the substituent that the "monocyclic aromatic group" may have and the like are used. As the "heterocyclic group" of the "optionally substituted heterocyclic group", groups similar to the "heterocyclic group" of the "optionally substituted heterocyclic group" as the substituent that the "monocyclic aromatic group" may have and the like are used. In addition, as the substituent that the hydroxy group and the amino group as the substituents of the "substituted sulfinyl group" and the "substituted sulfonyl group" may have, groups similar to the substituent that the "hydroxy group" of the "optionally substituted hydroxy group" and the "amino group" of the "optionally substituted amino group", each as the substituent that the "monocyclic aromatic group" optionally has, and the like are used. Preferable examples include a C₁₋₆ alkyl group, a C₃₋₈ cycloalkyl group, a C₂₋₄ alkenyl group, a C₆₋₁₀ aryl group, an acyl group, an amino group, a heterocyclic group (groups similar to the "heterocyclic group" of the"optionally substituted heterocyclic group" as the substituent that the "monocyclic aromatic group" may have etc.) and the like.

In addition, as the substituent of the "optionally substituted hydrocarbon group" and "optionally substituted heterocyclic group" as the substituents of the "substituted sulfinyl group" and "substituted sulfonyl group", a similar number of groups similar to the substituent of the "optionally substituted hydrocarbon group" and the substituent of the "optionally substituted heterocyclic group", each as the substituent that the "monocyclic aromatic group" optionally has, and the like are used.

As the "acyl group" as the substituent that the "monocyclic aromatic group" may have, an acyl group obtained by removing OH group from, for example, carboxylic acid such as R^{A}COOH and the like, sulfone acid such as R^{A}SO₃H and the like, sulfinic acid such as R^{A}SO₂H and the like, phosphoric acid such as R^{A}OPO (OR^{B}) OH wherein R^{A} is a hydrogen atom, an optionally substituted hydrocarbon group or an optionally substituted heterocyclic group, and R^{B} is a hydrogen atom or an optionally substituted hydrocarbon group, etc., and the like are used. Specifically, R^{A}CO, R^{A}SO₂, R^{A}SO, R^{A}OPO (OR^{B}) wherein the symbols in the formulas are as defined above, and the like are used.

As the "hydrocarbon group" of the "optionally substituted hydrocarbon group" and the "heterocyclic group" of the "optionally substituted heterocyclic group" for R^{A} (or R^{B}), groups similar to the "hydrocarbon group" of the "optionally substituted hydrocarbon group" and the "heterocyclic group" of the "optionally substituted heterocyclic group", each as the substituent that the "monocyclic aromatic group" may have, and the like are respectively used. In addition, as the substituent of the "optionally substituted hydrocarbon group" and the "optionally substituted heterocyclic group", a similar number of groups similar to the substituent of the "optionally substituted hydrocarbon group" and the "optionally substituted heterocyclic group", each as the substituent that the "monocyclic aromatic group" optionally has, and the like are used.

Examples of the R^{A}CO include formyl, acetyl, propionyl, butyryl, isobutyryl, valeryl, isovaleryl, pivaloyl, hexanoyl, cyclobutanecarbonyl, cyclopentanecarbonyl, cyclohexanecarbonyl, crotonyl, benzoyl, nicotinoyl, isonicotinoyl, trifluoroacetyl and the like. Among them, R^{A}CO wherein R^{A} is a lower (C₁₋₆) alkyl group such as acetyl, propionyl, butyryl, valeryl etc., and the like are more preferable.

Examples of the "optionally substituted carbamoyl group" as the substituent that the "monocyclic aromatic group" may have include N-mono-substituted carbamoyl and N,N-di-substituted carbamoyl besides unsubstituted carbamoyl.

Examples of the substituent that the "carbamoyl group" of the "optionally substituted carbamoyl group" may have include, the "optionally substituted hydrocarbon group", "acyl group", "optionally esterified carboxyl group" and "optionally substituted heterocyclic group" exemplified as the substituents of the "amino group" of the "optionally substituted amino group" as the substituent that the "monocyclic aromatic group" may have, as well as a "carbamoyl group optionally substituted by 1 or 2 substituents such as a lower (C₁₋₆) alkyl group, a phenyl group and the like (e.g., carbamoyl, N-methylcarbamoyl, N,N-dimethylcarbamoyl, phenylcarbamoyl etc.)" and the like. The substituent may also be a "carbamoyl group" having the aforementioned "amino group optionally substituted (by an optionally substituted hydrocarbon group, an acyl group, an optionally esterified carboxyl group or an optionally substituted heterocyclic group)" (that is, "optionally substituted carbazoyl group"), a "carbamoyl group" having the aforementioned "hydroxyl group optionally substituted (by an optionally substituted hydrocarbon group, an acyl group, an optionally esterified carboxyl group or an optionally substituted heterocyclic group)" (that is, "optionally substituted N-hydroxycarbamoyl group") and the like. In addition, two substituents of N,N-di-substituted carbamoyl may form cyclic amino together with a nitrogen atom, and as the cyclic aminocarbonyl in this case, for example, 1-azetidinylcarbonyl, 1-pyrrolidinylcarbonyl, piperidinocarbonyl, morpholinocarbonyl, thiomorpholinocarbonyl (wherein sulfur atom may be oxidized), 1-piperazinylcarbonyl, 1-homopiperazinylcarbonyl, and 3- to 8-membered (preferably 5- or 6-membered) cyclic aminocarbonyl (e.g., 1-piperazinylcarbonyl and the like) optionally having lower alkyl (e.g., C₁₋₆ alkyl such as methyl, ethyl, propyl, isopropyl, butyl, tert-butyl, pentyl, hexyl etc., and the like), aralkyl (e.g., C₇₋₁₀ aralkyl such as benzyl, phenethyl etc., and the like), aryl (e.g., C₆₋₁₀ aryl such as phenyl, 1-naphthyl, 2-naphthyl etc., and the like), a C₁₋₁₀ acyl group (e.g., formyl, acetyl, benzoyl, methoxycarbonyl, benzyloxycarbonyl, methylsulfonyl etc.) etc., and the like at the 4-position are used.

Specifically, as the optionally substituted carbamoyl group, for example, a carbamoyl group optionally substituted by 1 or 2 substituents such as a lower (C₁₋₆) alkyl group, a phenyl group etc., and the like are used. Specifically, carbamoyl, N-methylcarbamoyl, N,N-dimethylcarbamoyl, phenylcarbamoyl and the like are preferably used.

Examples of the "optionally esterified carboxyl group" as the substituent that the "monocyclic aromatic group" may have include a group represented by the formula -COOR^{C} wherein R^{C} is a hydrogen atom, an optionally substituted hydrocarbon group or an optionally substituted heterocyclic group, and the like. Among them, free carboxyl, lower alkoxycarbonyl, aryloxycarbonyl, aralkyloxycarbonyl, heterocyclic group-oxycarbonyl, heterocyclic group-methyloxycarbonyl and the like are preferably used.

As the "hydrocarbon group" of the "optionally substituted hydrocarbon group" and the "heterocyclic group" of the "optionally substituted heterocyclic group" for R^{C}, groups similar to the "hydrocarbon group" of the "optionally substituted hydrocarbon group" and the "heterocyclic group" of the "optionally substituted heterocyclic group", each as the substituent that the "monocyclic aromatic group" optionally has, and the like are used. As the substituents that the "hydrocarbon group" and "heterocyclic group" may have, a similar number of groups similar to the substituent that the "hydrocarbon group" of the "optionally substituted hydrocarbon group" and the "heterocyclic group" of the "optionally substituted heterocyclic group", each as the substituent that the "monocyclic aromatic group" optionally has, optionally has and the like are used.

Examples of the "lower alkoxycarbonyl" include C₁₋₆ alkoxycarbonyl such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl etc., and the like. Among these, C₁₋₃ alkoxycarbonyl such as methoxycarbonyl, ethoxycarbonyl, propoxycarbonyl etc. and the like are preferable.

The "lower alkoxycarbonyl" optionally has substituent(s) at the "lower alkyl" moiety of "lower alkoxy". As the substituent, a similar number of groups similar to those recited as the substituents that the "hydrocarbon group" of the "optionally substituted hydrocarbon group" as the substituent optionally possessed by the "monocyclic aromatic group" optionally has are used.

As "aryloxycarbonyl", for example, C₇₋₁₂ aryloxycarbonyl such as phenoxycarbonyl, 1-naphthoxycarbonyl, 2-naphthoxycarbonyl etc. and the like are preferable.

As "aralkyloxycarbonyl", for example, C₇₋₁₅ aralkyloxycarbonyl such as benzyloxycarbonyl, phenethyloxycarbonyl etc. (preferably, C₆₋₁₀ aryl-C₁₋₆ alkoxy-carbonyl and the like) and the like are preferable.

As the heterocyclic group of the "heterocyclic group-oxycarbonyl" and the "heterocyclic group-methyloxycarbonyl", those similar to the "heterocyclic group" of the "optionally substituted heterocyclic group" as the substituent that the "monocyclic aromatic group" optionally has, and the like are used and, for example, pyridyl, quinolyl, indolyl, piperidinyl, tetrahydropyranyl and the like are preferably used.

The "aryloxycarbonyl", "aralkyloxycarbonyl" and "heterocycleoxycarbonyl" each optionally have substituent(s). As such substituents, a similar number of groups similar to those recited as the substituents that the "hydrocarbon group" of the "optionally substituted hydrocarbon group" as the substituent that the "monocyclic aromatic group" optionally has and the like are used.

As the "C₁₋₃ alkylenedioxy group" as the substituent that the "monocyclic aromatic group" optionally has, methylenedioxy, ethylenedioxy and the like are used.

Of those mentioned above, as Ar¹⁰, an optionally substituted phenyl group or an optionally substituted thienyl group is preferable. Particularly, (1) an unsubstituted phenyl group or (2) a thienyl group (e.g., 2-thienyl group, 3-thienyl group) optionally substituted by C₁₋₆ alkyl (e.g., C₁₋₃ alkyl such as methyl, ethyl, propyl and the like, particularly methyl and the like) or a halogen atom (e.g., fluorine atom, chlorine atom, bromine atom, iodine atom and the like) is preferable.

As Ar²⁰, an optionally substituted phenyl group is preferable, and an unsubstituted phenyl group is particularly preferable.

As R¹⁰, a hydrogen atom or an optionally halogenated C₁₋₃ alkyl group (e.g., methyl, ethyl, propyl, isopropyl) is preferable. Among these, a hydrogen atom or a C₁₋₃ alkyl group is preferable. Specifically, as R¹⁰, a hydrogen atom or a methyl group is preferable, and a methyl group is particularly preferable.

As R²⁰, a hydrogen atom or an optionally halogenated C₁₋₃ alkyl group (e.g., methyl, ethyl, propyl, isopropyl) is preferable. Among these, an optionally halogenated C₁₋₃ alkyl group is preferable. Specifically, as R²⁰, a hydrogen atom or a methyl group is preferable, and a methyl group is particularly preferable.

As R³⁰, a hydrogen atom or an optionally halogenated C₁-₃ alkyl group (e.g., methyl, ethyl, propyl, isopropyl) is preferable. Among these, an optionally halogenated C₁₋₃ alkyl group is preferable, and a methyl group is particularly preferable.

As X¹⁰, -O- is preferable.

As Y¹⁰, a C₁₋₃ alkylene group (e.g., methylene, ethylene, propylene) is preferable, and a methylene group is preferable.

As a combination of X¹⁰ and Y¹⁰, a combination of X¹⁰ being -O- and Y¹⁰ being a methylene group is preferable.

As compound (I), the following compounds can be specifically mentioned.
(1) N-[3-[3,5-trans-7-chloro-3-[2-[(2-fluorobenzyl)amino]-2-oxoethyl]-1-neopentyl-2-oxo-1,2,3,5-tetrahydro-4,1-benzoxazepin-5-yl]benzyl]propanamide
(2) N-[3-[3,5-trans-7-chloro-3-[2-[(2-fluorobenzyl)amino]-2-oxoethyl]-1-neopentyl-2-oxo-1,2,3,5-tetrahydro-4,1-benzoxazepin-5-yl]benzyl]butaneamide
(3) ethyl [3-[3,5-trans-7-chloro-3-[2-[(2-fluorobenzyl)amino]-2-oxoethyl]-1-neopentyl-2-oxo-1,2,3,5-tetrahydro-4,1-benzoxazepin-5-yl]benzyl]carbamate
(4) 2-[3,5-trans-5-[3-(acetylaminomethyl)phenyl]-7-chloro-1-neopentyl-2-oxo-1,2,3,5-tetrahydro-4,1-benzoxazepin-3-yl]-N-phenylacetamide
(5) 2-[3,5-trans-5-[3-(acetylaminomethyl)phenyl]-7-chloro-1-neopentyl-2-oxo-1,2,3,5-tetrahydro-4,1-benzoxazepin-3-yl]-N-benzylacetamide
(6) 2-[3,5-trans-5-[3-(acetylaminomethyl)phenyl]-7-chloro-1-neopentyl-2-oxo-1,2,3,5-tetrahydro-4,1-benzoxazepin-3-yl]-N-propylacetamide
(7) 2-[3,5-trans-5-[3-(acetylaminomethyl)phenyl]-7-chloro-1-neopentyl-2-oxo-1,2,3,5-tetrahydro-4,1-benzoxazepin-3-yl]-N-benzyl-N-methylacetamide
(8) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2-pyridylmethyl)acetamide
(9) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(cyclohexylmethyl)acetamide
(10) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2-phenylethyl)acetamide
(11) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(1-methyl-1-phenylethyl)acetamide
(12) 2-[3,5-trans-5-[3-(acetylaminomethyl)phenyl]-7-chloro-1-neopentyl-2-oxo-1,2,3,5-tetrahydro-4,1-benzoxazepin-3-yl]-N-[4-(methylsulfonyl)benzyl]acetamide
(13) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(tetrahydrofuran-2-ylmethyl)acetamide
(14) 2-[3,5-trans-5-[3-(acetylaminomethyl)phenyl]-7-chloro-1-neopentyl-2-oxo-1,2,3,5-tetrahydro-4,1-benzoxazepin-3-yl]-N-[2-(trifluoromethyl)benzyl]acetamide
(15) tert-butyl [2-[3,5-trans-5-[3-(acetylaminomethyl)phenyl]-7-chloro-1-neopentyl-2-oxo-1,2,3,5-tetrahydro-4,1-benzoxazepin-3-yl]acetylamino] acetate
(16) 2-[3,5-trans-5-[3-(acetylaminomethyl)phenyl]-7-chloro-1-neopentyl-2-oxo-1,2,3,5-tetrahydro-4,1-benzoxazepin-3-yl]-N-[3,5-bis(trifluoromethyl)benzyl]acetamide
(17) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(cycloheptylmethyl)acetamide
(18) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(cyclopropylmethyl)acetamide
(19) 2-[3,5-trans-5-[3-(acetylaminomethyl)phenyl]-7-chloro-1-neopentyl-2-oxo-1,2,3,5-tetrahydro-4,1-benzoxazepin-3-yl]-N-[2-(methylsulfanyl)benzyl]acetamide
(20) 2-[3,5-trans-5-[3-(acetylaminomethyl)phenyl]-7-chloro-1-neopentyl-2-oxo-1,2,3,5-tetrahydro-4,1-benzoxazepin-3-yl]-N-[2-(methylsulfonyl)benzyl]acetamide
(21) 2-[3,5-trans-5-[3-(acetylaminomethyl)phenyl]-7-chloro-1-neopentyl-2-oxo-1,2,3,5-tetrahydro-4,1-benzoxazepin-3-yl]-N-[3-(trifluoromethyl)benzyl]acetamide
(22) 2-[3,5-trans-5-[3-(acetylaminomethyl)phenyl]-7-chloro-1-neopentyl-2-oxo-1,2,3,5-tetrahydro-4,1-benzoxazepin-3-yl]-N-[4-(trifluoromethyl)benzyl]acetamide
(23) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2-bromobenzyl)acetamide
(24) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2-chlorobenzyl)acetamide
(25) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2-methoxybenzyl)acetamide
(26) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2,6-difluorobenzyl)acetamide
(27) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2,2,2-trifluoroethyl)acetamide
(28) [2-[3,5-trans-5-[3-(acetylaminomethyl)phenyl]-7-chloro-1-neopentyl-2-oxo-1,2,3,5-tetrahydro-4,1-benzoxazepin-3-yl]acetylamino]acetic acid
(29) methyl [3-[3,5-trans-7-chloro-l-neopentyl-2-oxo-3-[2-oxo-2-[[2-(trifluoromethyl)benzyl]amino]ethyl]-1,2,3,5-tetrahydro-4,1-benzoxazepin-5-yl]benzyl]carbamate
(30) methyl [3-[3,5-trans-7-chloro-3-[2-[(cyclohexylmethyl)amino]-2-oxoethyl]-1-neopentyl-2-oxo-1,2,3,5-tetrahydro-4,1-benzoxazepin-5-yl]benzyl]carbamate
(31) methyl [3-[3,5-trans-7-chloro-3-[2-(3,4-dihydroisoquinolin-2(1H)-yl)-2-oxoethyl]-1-neopentyl-2-oxo-1,2,3,5-tetrahydro-4,1-benzoxazepin-5-yl]benzyl]carbamate
(32) N-{[3,5-trans-5-[3-(acetylaminomethyl)phenyl]-7-chloro-1-neopentyl-2-oxo-1,2,3,5-tetrahydro-4,1-benzoxazepin-3-yl]methyl}-2-(2-fluorophenyl)acetamide
(33) N-[3-[3,5-trans-7-chloro-3-[[[[(2-fluorobenzyl)amino]carbonyl]amino]methyl]-1-neopentyl-2-oxo-1,2,3,5-tetrahydro-4,1-benzoxazepin-5-yl]benzyl]acetamide
(34) 2-[3,5-trans-5-[3-(2-amino-2-oxoethoxy)-2-methoxyphenyl]-7-chloro-1-neopentyl-2-oxo-1,2,3,5-tetrahydro-4,1-benzoxazepin-3-yl]-N-(2-fluorobenzyl) acetamide
(35) 2-[3,5-trans-7-chloro-5-[3-[2-(dimethylamino)-2-oxoethoxy]-2-methoxyphenyl]-1-neopentyl-2-oxo-1,2,3,5-tetrahydro-4,1-benzoxazepin-3-yl]-N-(2-fluorobenzyl)acetamide
(36) 2-[3-(3,5-trans-7-chloro-3-{2-[(2-fluorobenzyl)amino]-2-oxoethyl}-1-neopentyl-2-oxo-1,2,3,5-tetrahydro-4,1-benzoxazepin-5-yl)-2-methoxyphenoxy]-N-(2-phenylethyl)acetamide
(37) 2-[3-[3,5-trans-7-chloro-3-[2-[(2-fluorobenzyl)amino]-2-oxoethyl]-1-neopentyl-2-oxo-1,2,3,5-tetrahydro-4,1-benzoxazepin-5-yl]-2-methoxyphenoxy]-N-[3-(1H-imidazol-1-yl)propyl] acetamide
(38) 2-[3,5-trans-5-[3-(acetylaminomethyl)phenyl]-7-chloro-2-oxo-1,2,3,5-tetrahydro-4,1-benzoxazepin-3-yl]-N-(2-fluorobenzyl)acetamide
(39) 2-[3,5-trans-5-[4-(acetylaminomethyl)phenyl]-7-chloro-1-neopentyl-2-oxo-1,2,3,5-tetrahydro-4,1-benzoxazepin-3-yl]-N-(2-fluorobenzyl)acetamide
(40) 2-[3,5-trans-5-[3-(acetylaminomethyl)phenyl]-7-chloro-1-isobutyl-2-oxo-1,2,3,5-tetrahydro-4,1-benzoxazepin-3-yl]-N-(2-fluorobenzyl)acetamide
(41) 2-[3,5-trans-5-[3-(acetylaminomethyl)phenyll-7-chloro-2-oxo-1-propyl-1,2,3,5-tetrahydro-4,1-benzoxazepin-3-yl]-N-(2-fluorobenzyl)acetamide
(42) 2-[3,5-trans-5-[3-(acetylaminomethyl)phenyl]-7-chloro-2-oxo-1-(2-thienylmethyl)-1,2,3,5-tetrahydro-4,1-benzoxazepin-3-yl]-N-(2-fluorobenzyl)acetamide
(43) methyl [3-[3,5-trans-7-chloro-3-[2-[(2-fluorobenzyl)amino]-2-oxoethyl]-1-isobutyl-2-oxo-1,2,3,5-tetrahydro-4,1-benzoxazepin-5-yl]benzyl]carbamate
(44) methyl [3-[3,5-trans-7-chloro-3-[2-[(2-fluorobenzyl)amino]-2-oxoethyl]-2-oxo-1-propyl-1,2,3,5-tetrahydro-4,1-benzoxazepin-5-yl]benzyl]carbamate
(45) 3-[3,5-trans-5-[3-(acetylaminomethyl)phenyl]-7-chloro-3-[2-[(2-fluorobenzyl)amino]-2-oxoethyl]-2-oxo-2,3-dihydro-4,1-benzoxazepin-1(5H)-yl]-2,2-dimethylpropyl acetate
(46) 3-[3,5-trans-7-chloro-3-[2-[(2-fluorobenzyl)amino]-2-oxoethyl]-5-[3-(methoxycarbonylaminomethyl)phenyl]-2-oxo-2,3-dihydro-4,1-benzoxazepin-1(5H)-yl]-2,2-dimethylpropyl acetate
(47) 2-[3,5-trans-5-[3-(acetylaminomethyl)phenyl]-7-chloro-1-(3-hydroxy-2,2-dimethylpropyl)-2-oxo-1,2,3,5-tetrahydro-4,1-benzoxazepin-3-yl]-N-(2-fluorobenzyl)acetamide
(48) methyl [3-[3,5-trans-7-chloro-3-[2-[(2-fluorobenzyl)amino]-2-oxoethyl]-1-(3-hydroxy-2,2-dimethylpropyl)-2-oxo-1,2,3,5-tetrahydro-4,1-benzoxazepin-5-yl]benzyl]carbamate
(49) 3-[3,5-trans-7-chloro-5-[3-(methoxycarbonylaminomethyl)phenyl]-2-oxo-3-[2-oxo-2-[[2-(trifluoromethyl)benzyl]amino]ethyl]-2,3-dihydro-4,1-benzoxazepin-1(5H)-yl]-2,2-dimethylpropyl acetate
(50) methyl [3-[3,5-trans-7-chloro-1-(3-hydroxy-2,2-dimethylpropyl)-2-oxo-3-[2-oxo-2-[[2-(trifluoromethyl)benzyl]amino]ethyl]-1,2,3,5-tetrahydro-4,1-benzoxazepin-5-yl]benzyl]carbamate
(51) 3-[3,5-trans-7-chloro-5-[3-(methoxycarbonylaminomethyl)phenyl]-2-oxo-3-[2-oxo-2-[[2-(trifluoromethyl)benzyl]amino]ethyl]-2,3-dihydro-4,1-benzoxazepin-1(5H)-yl]-2,2-dimethylpropyl methanesulfonate
(52) methyl 4-trans-[[3,5-trans-5-[3-(acetylaminomethyl)phenyl]-7-chloro-1-neopentyl-2-oxo-1,2,3,5-tetrahydro-4,1-benzoxazepin-3-yl]acetylaminomethyl]cyclohexanecarboxylate
(53) 2-[3,5-trans-5-[3-(acetylaminomethyl)phenyl]-7-chloro-1-neopentyl-2-oxo-1,2,3,5-tetrahydro-4,1-benzoxazepin-3-yl]-N-cyclohexylacetamide
(54) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2-thienylmethyl)acetamide
(55) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(3-thienylmethyl)acetamide
(56) 2-[3,5-trans-5-[3-(acetylaminomethyl)phenyl]-7-chloro-1-neopentyl-2-oxo-1,2,3,5-tetrahydro-4,1-benzoxazepin-3-yl]-N-(2-furylmethyl)acetamide
(57) 4-trans-[[3,5-trans-5-[3-(acetylaminomethyl)phenyl]-7-chloro-1-neopentyl-2-oxo-1,2,3,5-tetrahydro-4,1-benzoxazepin-3-yl]acetylaminomethyl]cyclohexanecarboxylic acid
(58) 2-[3,5-trans-5-[3-(acetylaminomethyl)phenyl]-1-neopentyl-2-oxo-1,2,3,5-tetrahydro-4,1-benzoxazepin-3-yl]-N-(2-fluorobenzyl)acetamide
(59) 2-[3,5-trans-7-chloro-l-neopentyl-2-oxo-5-[3-(4H-1,2,4-triazol-4-ylmethyl)phenyl]-1,2,3,5-tetrahydro-4,1-benzoxazepin-3-yl]-N-(2-fluorobenzyl)acetamide
(60) 2-[3,5-trans-7-chloro-5-[3-(1H-imidazol-1-ylmethyl)phenyl]-1-neopentyl-2-oxo-1,2,3,5-tetrahydro-4,1-benzoxazepin-3-yl]-N-(2-fluorobenzyl)acetamide
(61) 2-[3,5-trans-5-[3-(acetylaminomethyl)phenyl]-7-chloro-1-(6-methoxy-2-naphthylmethyl)-2-oxo-1,2,3,5-tetrahydro-4,1-benzoxazepin-3-yl]-N-(2-fluorobenzyl)acetamide
(62) 2-[3,5-trans-5-[3-(acetylaminomethyl)phenyl]-7-chloro-2-oxo-1-(quinolin-2-ylmethyl)-1,2,3,5-tetrahydro-4,1-benzoxazepin-3-yl]-N-(2-fluorobenzyl)acetamide
(63) 2-[3,5-trans-5-[3-(acetylaminomethyl)phenyl]-7-chloro-1-(9H-fluoren-2-ylmethyl)-2-oxo-1,2,3,5-tetrahydro-4,1-benzoxazepin-3-yl]-N-(2-fluorobenzyl)acetamide
(64) 2-[3,5-trans-5-[3-(acetylaminomethyl)phenyl]-7-chloro-1-[5-(2-methoxyphenyl)-2-furylmethyl]-2-oxo-1,2,3,5-tetrahydro-4,1-benzoxazepin-3-yl]-N-(2-fluorobenzyl)acetamide
(65) 2-[3,5-trans-5-[3-(acetylaminomethyl)phenyl]-1-(2,3'-bithien-5-ylmethyl)-7-chloro-2-oxo-1,2,3,5-tetrahydro-4,1-benzoxazepin-3-yl]-N-(2-fluorobenzyl)acetamide

(1B) N-(2-fluorobenzyl)-2-(4-isonicotinoyl-1-neopentyl-2-oxo-2,3,4,5-tetrahydro-1H-1,4-benzodiazepin-3-yl)acetamide
(2B) N-(2-fluorobenzyl)-2-[1-neopentyl-2-oxo-4-(3-pyridylcarbonyl)-2,3,4,5-tetrahydro-1H-1,4-benzodiazepin-3-yl]acetamide
(3B) N-(2-fluorobenzyl)-2-[1-neopentyl-2-oxo-4-(2-pyridylcarbonyl)-2,3,4,5-tetrahydro-1H-1,4-benzodiazepin-3-yl]acetamide
(4B) N-(2-fluorobenzyl)-2-[1-neopentyl-2-oxo-4-(4-quinolinylcarbonyl)-2,3,4,5-tetrahydro-1H-1,4-benzodiazepin-3-yl]acetamide
(5B) N-(2-fluorobenzyl)-2-[1-neopentyl-2-oxo-4-(3-thienylcarbonyl)-2,3,4,5-tetrahydro-1H-1,4-benzodiazepin-3-yl]acetamide
(6B) N-(2-fluorobenzyl)-2-[4-(3-furoyl)-1-neopentyl-2-oxo-2,3,4,5-tetrahydro-1H-1,4-benzodiazepin-3-yl]acetamide
(7B) 2-(4-benzoyl-1-neopentyl-2-oxo-2,3,4,5-tetrahydro-1H-1,4-benzodiazepin-3-yl)-N-(2-fluorobenzyl)acetamide
(8B) N-(2-fluorobenzyl)-2-[1-neopentyl-2-oxo-4-(2-pyrazinylcarbonyl)-2,3,4,5-tetrahydro-1H-1,4-benzodiazepin-3-yl]acetamide
(9B) N-(2-fluorobenzyl)-2-[1-neopentyl-2-oxo-4-(1H-pyrrol-2-ylcarbonyl)-2,3,4,5-tetrahydro-1H-1,4-benzodiazepin-3-yl]acetamide
(10B) 2-[4-[(2,4-dimethyl-1,3-thiazol-5-yl)carbonyl]-1-neopentyl-2-oxo-2,3,4,5-tetrahydro-1H-1,4-benzodiazepin-3-yl]-N-(2-fluorobenzyl)acetamide
(11B) N-(2-fluorobenzyl)-2-[1-neopentyl-2-oxo-4-(4-pyridylacetyl)-2,3,4,5-tetrahydro-1H-1,4-benzodiazepin-3-yl]acetamide
(12B) N-(2-fluorobenzyl)-2-[1-neopentyl-2-oxo-4-(3-pyridylacetyl)-2,3,4,5-tetrahydro-1H-1,4-benzodiazepin-3-yl]acetamide
(13B) 2-[4-(2-chloroisonicotinoyl)-1-neopentyl-2-oxo-2,3,4,5-tetrahydro-1H-1,4-benzodiazepin-3-yl]-N-(2-fluorobenzyl)acetamide
(14B) N-(2-fluorobenzyl)-2-[4-(2-methylisonicotinoyl)-1-neopentyl-2-oxo-2,3,4,5-tetrahydro-1H-1,4-benzodiazepin-3-yl]acetamide
(15B) 4-[[3-[2-[(2-fluorobenzyl)amino]-2-oxoethyl]-1-neopentyl-2-oxo-1,2,3,5-tetrahydro-4H-1,4-benzodiazepin-4-yl]carbonyl]-2-pyridinecarboxyamide
(16B) 2-[4-[4-(acetylamino)benzoyl]-1-neopentyl-2-oxo-2,3,4,5-tetrahydro-1H-1,4-benzodiazepin-3-yl]-N-(2-fluorobenzyl)acetamide
(17B) 2-[4-[(1-acetyl-4-piperidinyl)carbonyl]-1-neopentyl-2-oxo-2,3,4,5-tetrahydro-1H-1,4-benzodiazepin-3-yl]-N-(2-fluorobenzyl)acetamide
(18B) N-(2-fluorobenzyl)-2-[1-neopentyl-4-(1-oxidoisonicotinoyl)-2-oxo-2,3,4,5-tetrahydro-1H-1,4-benzodiazepin-3-yl]acetamide
(19B) 2-[4-(2-cyanoisonicotinoyl)-1-neopentyl-2-oxo-2,3,4,5-tetrahydro-1H-1,4-benzodiazepin-3-yl]-N-(2-fluorobenzyl)acetamide
(20B) 2-[4-[3,5-bis(trifluoromethyl)benzoyl]-1-neopentyl-2-oxo-2,3,4,5-tetrahydro-1H-1,4-benzodiazepin-3-yl]-N-(2-fluorobenzyl)acetamide
(21B) N-(2-fluorobenzyl)-2-(1-isobutyl-4-isonicotinoyl-2-oxo-2,3,4,5-tetrahydro-1H-1,4-benzodiazepin-3-yl)acetamide
(22B) 2-[1-(2,4-dimethoxybenzyl)-4-isonicotinoyl-2-oxo-2,3,4,5-tetrahydro-1H-1,4-benzodiazepin-3-yl]-N-(2-fluorobenzyl)acetamide
(23B) N-(2-fluorobenzyl)-2-[1-isobutyl-4-(2-methylisonicotinoyl)-2-oxo-2,3,4,5-tetrahydro-1H-1,4-benzodiazepin-3-yl]acetamide
(24B) 3-[3-[2-[(2-fluorobenzyl)amino]-2-oxoethyl]-4-isonicotinoyl-2-oxo-2,3,4,5-tetrahydro-1H-1,4-benzodiazepin-1-yl]-2,2-dimethylpropylacetate
(25B) 3-[4-(2-chloroisonicotinoyl)-3-[2-[(2-fluorobenzyl)amino]-2-oxoethyl]-2-oxo-2,3,4,5-tetrahydro-1H-1,4-benzodiazepin-1-yl]-2,2-dimethylpropylacetate
(26B) 3-[4-isonicotinoyl-2-oxo-3-[2-oxo-2-[[2-(trifluoromethyl)benzyl]amino]ethyl]-2,3,4,5-tetrahydro-1H-1,4-benzodiazepin-1-yl]-2,2-dimethylpropylacetate
(27B) 3-[4-(2-chloroisonicotinoyl)-2-oxo-3-[2-oxo-2-[[2-(trifluoromethyl)benzyl]amino]ethyl]-2,3,4,5-tetrahydro-1H-1,4-benzodiazepin-1-yl]-2,2-dimethylpropylacetate
(28B) 2-[4-[2-(acetylamino)isonicotinoyl]-1-neopentyl-2-oxo-2,3,4,5-tetrahydro-1H-1,4-benzodiazepin-3-yl]-N-(2-fluorobenzyl)acetamide
(29B) N-(2,6-dichloropyridin-4-yl)-3-[2-[(2-fluorobenzyl)amino]-2-oxoethyl]-1-neopentyl-2-oxo-1,2,3,5-tetrahydro-4H-1,4-benzodiazepine-4-carboxamide
(30B) N-(2-fluorobenzyl)-2-[1-neopentyl-2-oxo-4-(pyridin-4-ylmethyl)-2,3,4,5-tetrahydro-1H-1,4-benzodiazepin-3-yl]acetamide
(31B) N-(2-fluorobenzyl)-2-(4-isonicotinoyl-2-oxo-2,3,4,5-tetrahydro-1H-1,4-benzodiazepin-3-yl)acetamide

(1C) 4-(4-fluorobenzyl)-6-phenyl-2,3,4,5-tetrahydropyrido[3,2-f][1,4]oxazepine hydrochloride
(2C) 4-benzyl-6-phenyl-2,3,4,5-tetrahydropyrido[3,2-f][1,4]oxazepine hydrochloride
(3C) 4-(3-methoxybenzyl)-6-phenyl-2,3,4,5-tetrahydropyrido[3,2-f][1,4]oxazepine hydrochloride
(4C) 4-(3,5-dimethoxybenzyl)-6-phenyl-2,3,4,5-tetrahydropyrido[3,2-f][1,4]oxazepine hydrochloride
(5C) 6-phenyl-4-(3,4,5-trimethoxybenzyl)-2,3,4,5-tetrahydropyrido[3,2-f][1,4]oxazepine
(6C) 4-(2-chlorobenzyl)-6-phenyl-2,3,4,5-tetrahydropyrido[3,2-f][1,4]oxazepine hydrochloride
(7C) 4-(3,4-dichlorobenzyl)-6-phenyl-2,3,4,5-tetrahydropyrido[3,2-f][1,4]oxazepine hydrochloride
(8C) 4-(2,6-dichlorobenzyl)-6-phenyl-2,3,4,5-tetrahydropyrido[3,2-f][1,4]oxazepine hydrochloride
(9C) 4-[3,5-bis(trifluoromethyl)benzyl]-6-phenyl-2,3,4,5-tetrahydropyrido[3,2-f][1,4]oxazepine
(10C) 4-(2-nitrobenzyl)-6-phenyl-2,3,4,5-tetrahydropyrido[3,2-f][1,4]oxazepine hydrochloride
(11C) 4-(3,5-dinitrobenzyl)-6-phenyl-2,3,4,5-tetrahydropyrido[3,2-f][1,4]oxazepine
(12C) 6-phenyl-4-(2-phenylethyl)-2,3,4,5-tetrahydropyrido[3,2-f][1,4]oxazepine hydrochloride
(13C) 4-[3,5-bis(trifluoromethyl)benzyl]-8-methyl-6-phenyl-2,3,4,5-tetrahydropyrido[3,2-f][1,4]oxazepine hydrochloride
(14C) 4-[3,5-bis(trifluoromethyl)benzoyl]-6-phenyl-2,3,4,5-tetrahydropyrido[3,2-f][1,4]oxazepine
(15C) 5-benzyl-7-phenyl-3,4,5,6-tetrahydro-2H-pyrido[2,3-b][1,5]oxazocine 1/2 sulfate
(16C) 5-(3,5-dimethoxybenzyl)-7-phenyl-3,4,5,6-tetrahydro-2H-pyrido[2,3-b][1,5]oxazocine 1/2 sulfate
(17C) 5-(3,4-dichlorobenzyl)-7-phenyl-3,4,5,6-tetrahydro-2H-pyrido[2,3-b][1,5]oxazocine hydrochloride
(18C) 5-[3,5-bis(trifluoromethyl)benzyl]-7-phenyl-3,4,5,6-tetrahydro-2H-pyrido[2,3-b][1,5]oxazocine 1/2 sulfate
(19C) 5-[3,5-bis(trifluoromethyl)benzoyl]-7-phenyl-3,4,5,6-tetrahydro-2H-pyrido[2,3-b][1,5]oxazocine
(20C) 4-{[3,5-bis(trifluoromethyl)phenyl]acetyl}-6-phenyl-2,3,4,5-tetrahydropyrido[3,2-f][1,4]oxazepine
(21C) 5-{[3,5-bis(trifluoromethyl)phenyl]acetyl}-7-phenyl-3,4,5,6-tetrahydro-2H-pyrido[2,3-b][1,5]oxazocine
(22C) 4-{[3,5-bis(trifluoromethyl)phenyl]sulfonyl}-6-phenyl-2,3,4,5-tetrahydropyrido[3,2-f][1,4]oxazepine
(23C) 5-{[3,5-bis(trifluoromethyl)phenyl]sulfonyl}-7-phenyl-3,4,5,6-tetrahydro-2H-pyrido[2,3-b][1,5]oxazocine
(24C) 4-(4-chlorobenzoyl)-6-phenyl-2,3,4,5-tetrahydropyrido[3,2-f][1,4]oxazepine
(25C) 4-(4-nitrobenzoyl)-6-phenyl-2,3,4,5-tetrahydropyrido[3,2-f][1,4]oxazepine
(26C) 4-(3-methylbenzoyl)-6-phenyl-2,3,4,5-tetrahydropyrido[3,2-f][1,4]oxazepine
(27C) 4-(1-naphthoyl)-6-phenyl-2,3,4,5-tetrahydropyrido[3,2-f][1,4]oxazepine
(28C) 4-(1-benzothien-2-ylcarbonyl)-6-phenyl-2,3,4,5-tetrahydropyrido[3,2-f][1,4]oxazepine
(29C) 6-phenyl-4- [3-(trifluoromethyl)benzoyl]-2,3,4,5-tetrahydropyrido[3,2-f][1,4]oxazepine
(30C) 6-phenyl-4-[4-(trifluoromethyl)benzoyl]-2,3,4,5-tetrahydropyrido[3,2-f][1,4]oxazepine
(31C) 4-(3-chlorobenzoyl)-6-phenyl-2,3,4,5-tetrahydropyrido[3,2-f][1,4]oxazepine
(32C) 4-(3,5-dichlorobenzoyl)-6-phenyl-2,3,4,5-tetrahydropyrido[3,2-f][1,4]oxazepine
(33C) 4-{1-[3,5-bis(trifluoromethyl)phenyl]ethyl}-6-phenyl-2,3,4,5-tetrahydropyrido[3,2-f][1,4]oxazepine hydrochloride
(34C) 4-(3,5-dimethylbenzoyl)-6-phenyl-2,3,4,5-tetrahydropyrido[3,2-f][1,4]oxazepine
(35C) 6-phenyl-4-[2-(trifluoromethyl)benzoyl]-2,3,4,5-tetrahydropyrido[3,2-f][1,4]oxazepine
(36C) 4-(2-naphthoyl)-6-phenyl-2,3,4,5-tetrahydropyrido[3,2-f][1,4]oxazepine
(37C) 4-(1,3-benzodioxol-5-ylcarbonyl)-6-phenyl-2,3,4,5-tetrahydropyrido[3,2-f][1,4]oxazepine
(38C) 6-phenyl-4-(pyridin-3-ylcarbonyl)-2,3,4,5-tetrahydropyrido[3,2-f][1,4]oxazepine
(39C) 3-[(6-phenyl-2,3-dihydropyrido[3,2-f][1,4]oxazepin-4(5H)-yl)carbonyl]benzonitrile
(40C) 4-[(6-phenyl-2,3-dihydropyrido[3,2-f][1,4]oxazepin-4(5H)-yl)carbonyl]indan-1-one
(41C) 4-[(3,5-dichlorophenyl)sulfonyl]-6-phenyl-2,3,4,5-tetrahydropyrido[3,2-f][1,4]oxazepine
(42C) 4-(1-naphthylsulfonyl)-6-phenyl-2,3,4,5-tetrahydropyrido[3,2-f][1,4]oxazepine
(43C) 6-phenyl-4-(quinolin-8-ylsulfonyl)-2,3,4,5-tetrahydropyrido[3,2-f][1,4]oxazepine
(44C) 4-{3-[3,5-bis(trifluoromethyl)phenyl]propanoyl}-6-phenyl-2,3,4,5-tetrahydropyrido[3,2-f][1,4]oxazepine
(45C) 4-{2-[3,5-bis(trifluoromethyl)phenyl]ethyl}-6-phenyl-2,3,4,5-tetrahydropyrido[3,2-f][1,4]oxazepine hydrochloride
(46C) N-[3,5-bis(trifluoromethyl)phenyl]-6-phenyl-2,3-dihydropyrido[3,2-f][1,4]oxazepine-4(5H)-carboxamide
(47C) 4-[3,5-bis(trifluoromethyl)benzoyl]-6-phenyl-2,3,4,5-tetrahydropyrido[3,2-f][1,4]oxazepine 9-oxide
(48C) 5-{1-[3,5-bis(trifluoromethyl)phenyl]ethyl}-7-phenyl-3,4,5,6-tetrahydro-2H-pyrido[2,3-b][1,5]oxazocine hydrochloride
(49C) 6-[3,5-bis(trifluoromethyl)benzyl]-8-phenyl-2,3,4,5,6,7-hexahydropyrido[2,3-b][1,5]oxazonine
(50C) 6-{1-[3,5-bis(trifluoromethyl)phenyl]ethyl}-8-phenyl-2,3,4,5,6,7-hexahydropyrido[2,3-b][1,5]oxazonine
(51C) 6-[3,5-bis(trifluoromethyl)benzoyl]-8-phenyl-2,3,4,5,6,7-hexahydropyrido[2,3-b][1,5]oxazonine
(52C) 6-{[3,5-bis(trifluoromethyl)phenyl]sulfonyl}-8-phenyl-2,3,4,5,6,7-hexahydropyrido[2,3-b][1,5]oxazonine
(53C) 4-[3,5-bis(trifluoromethyl)benzyl]-6-(4-fluorophenyl)-2,3,4,5-tetrahydropyrido[3,2-f][1,4]oxazepine
(54C) 4-{1-[3,5-bis(trifluoromethyl)phenyl]ethyl}-6-(4-fluorophenyl)-2,3,4,5-tetrahydropyrido[3,2-f][1,4]oxazepine
(55C) 4-[3,5-bis(trifluoromethyl)benzoyl]-6-(4-fluorophenyl)-2,3,4,5-tetrahydropyrido[3,2-f][1,4]oxazepine
(56C) 4-{[3,5-bis(trifluoromethyl)phenyl]sulfonyl}-6-(4-fluorophenyl)-2,3,4,5-tetrahydropyrido[3,2-f][1,4]oxazepine
(57C) 5-[3,5-bis(trifluoromethyl)benzyl]-7-(4-fluorophenyl)-3,4,5,6-tetrahydro-2H-pyrido[2,3-b][1,5]oxazocine
(58C) 5-{1-[3,5-bis(trifluoromethyl)phenyl]ethyl}-7-(4-fluorophenyl)-3,4,5,6-tetrahydro-2H-pyrido[2,3-b][1,5]oxazocine
(59C) 5-[3,5-bis(trifluoromethyl)benzoyl]-7-(4-fluorophenyl)-3,4,5,6-tetrahydro-2H-pyrido[2,3-b][1,5]oxazocine
(60C) 5-{[3,5-bis(trifluoromethyl)phenyl]sulfonyl}-7-(4-fluorophenyl)-3,4,5,6-tetrahydro-2H-pyrido[2,3-b][1,5]oxazocine
(61C) 6-[3,5-bis(trifluoromethyl)benzyl]-8-(4-fluorophenyl)-2,3,4,5,6,7-hexahydropyrido[2,3-b][1,5]oxazonine
(62C) 6-{1-[3,5-bis(trifluoromethyl)phenyl]ethyl}-8-(4-fluorophenyl)-2,3,4,5,6,7-hexahydropyrido[2,3-b][1,5]oxazonine
(63C) 6-[3,5-bis(trifluoromethyl)benzoyl]-8-(4-fluorophenyl)-2,3,4,5,6,7-hexahydropyrido[2,3-b][1,5]oxazonine
(64C) 6-{[3,5-bis(trifluoromethyl)phenyl]sulfonyl}-8-(4-fluorophenyl)-2,3,4,5,6,7-hexahydropyrido[2,3-b][1,5]oxazonine
(65C) 4-[3,5-bis(trifluoromethyl)benzoyl]-6-(3-fluorophenyl)-2,3,4,5-tetrahydropyrido[3,2-f][1,4]oxazepine
(66C) 4-[3,5-bis(trifluoromethyl)benzenesulfonyl]-6-(3-fluorophenyl)-2,3,4,5-tetrahydropyrido[3,2-f][1,4]oxazepine
(67C) 4-[3,5-bis(trifluoromethyl)benzyl]-6-(3-fluorophenyl)-2,3,4,5-tetrahydropyrido[3,2-f][1,4]oxazepine
(68C) 5-[3,5-bis(trifluoromethyl)benzoyl]-7-(3-fluorophenyl)-3,4,5,6-tetrahydro-2H-pyrido[2,3-b][1,5]oxazocine
(69C) 5-[3,5-bis(trifluoromethyl)benzenesulfonyl]-7-(3-fluorophenyl)-3,4,5,6-tetrahydro-2H-pyrido[2,3-b][1,5]oxazocine
(70C) 5-[3,5-bis(trifluoromethyl)benzyl]-7-(3-fluorophenyl)-3,4,5,6-tetrahydro-2H-pyrido[2,3-b][1,5]oxazocine
(71C) 4-{1-[3,5-bis(trifluoromethyl)phenyl]ethyl}-6-(3-fluorophenyl)-2,3,4,5-tetrahydropyrido[3,2-f][1,4]oxazepine
(72C) tert-butyl 3-{[6-(4-fluorophenyl)-2,3-dihydropyrido[3,2-f][1,4]oxazepin-4(5H)-yl]carbonyl}benzylcarbamate
(73C) 3-{[6-(4-fluorophenyl)-2,3-dihydropyrido[3,2-f][1,4]oxazepin-4(5H)-yl]carbonyl}benzylamine hydrochloride
(74C) 4-[[3,5-bis(trifluoromethyl)phenyl]sulfonyl]-6-(4-chlorophenyl)-2,3,4,5-tetrahydropyrido[3,2-f][1,4]oxazepine
(75C) 4-[3,5-bis(trifluoromethyl)benzoyl]-6-(4-chlorophenyl)-2,3,4,5-tetrahydropyrido[3,2-f][1,4]oxazepine
(76C) 4-[3,5-bis(trifluoromethyl)benzyl]-6-(4-chlorophenyl)-2,3,4,5-tetrahydropyrido[3,2-f][1,4]oxazepine
(77C) 4-[1-[3,5-bis(trifluoromethyl)phenyl]ethyl]-6-(4-chlorophenyl)-2,3,4,5-tetrahydropyrido[3,2-f][1,4]oxazepine
(78C) 5-[[3,5-bis(trifluoromethyl)phenyl]sulfonyl]-7-(4-chlorophenyl)-3,4,5,6-tetrahydro-2H-pyrido[2,3-b][1,5]oxazocine
(79C) 5-[3,5-bis(trifluoromethyl)benzoyl]-7-(4-chlorophenyl)-3,4,5,6-tetrahydro-2H-pyrido[2,3-b][1,5]oxazocine
(80C) 5-[3,5-bis(trifluoromethyl)benzyl]-7-(4-chlorophenyl)-3,4,5,6-tetrahydro-2H-pyrido[2,3-b][1,5]oxazocine
(81C) 5-[[3,5-bis(trifluoromethyl)phenyl]sulfonyl]-7-(4-chlorophenyl)-3,4,5,6-tetrahydro-2H-pyrido[2,3-b][1,5]oxazocine 10-oxide
(82C) 4-[1-[3,5-bis(trifluoromethyl)phenyl]ethyl]-6-(4-methylphenyl)-2,3,4,5-tetrahydropyrido[3,2-f][1,4]oxazepine dihydrochloride
(83C) 4-[(2,2-difluoro-1,3-benzodioxol-4-yl)carbonyl]-6-phenyl-2,3,4,5-tetrahydropyrido[3,2-f][1,4]oxazepine
(84C) 4-[1-[3,5-bis(trifluoromethyl)phenyl]propyl]-6-phenyl-2,3,4,5-tetrahydropyrido[3,2-f][1,4]oxazepine hydrochloride

(1D) 4-[3,5-bis(trifluoromethyl)benzyl]-6-phenyl-4,5-dihydropyrido[3,2-f][1,4]oxazepin-3(2H)-one
(2D) 4-[3,5-bis(trifluoromethyl)benzyl]-6-phenyl-4,5-dihydropyrido[3,2-f][1,4]thiazepin-3(2H)-one
(3D) 4-[3,5-bis(trifluoromethyl)benzyl]-6-(4-fluorophenyl)-4,5-dihydropyrido[3,2-f][1,4]oxazepin-3(2H)-one
(4D) 4-{1-[3,5-bis(trifluoromethyl)phenyl]ethyl}-6-(4-fluorophenyl)-4,5-dihydropyrido[3,2-f][1,4]oxazepin-3(2H)-one
(5D) 4-[3,5-bis(trifluoromethyl)benzyl]-8-methyl-6-phenyl-4,5-dihydropyrido[3,2-f][1,4]oxazepin-3(2H)-one
(6D) 4-[3,5-bis(trifluoromethyl)benzyl]-6-(4-fluorophenyl)-2,2-dimethyl-4,5-dihydropyrido[3,2-f][1,4]oxazepin-3(2H)-one
(7D) 4-[3,5-bis(trifluoromethyl)benzyl]-6-(3-fluorophenyl)-4,5-dihydropyrido[3,2-f][1,4]oxazepin-3(2H)-one
(8D) 4-[3,5-bis(trifluoromethyl)benzyl]-6-(4-fluorophenyl)-5-methyl-4,5-dihydropyrido[3,2-f][1,4]oxazepin-3(2H)-one
(9D) 4-[3,5-bis(trifluoromethyl)benzyl]-6-(4-chlorophenyl)-4,5-dihydropyrido[3,2-f][1,4]oxazepin-3(2H)-one
(10D) 4-[3,5-bis(trifluoromethyl)benzyl]-6-(4-methylphenyl)-4,5-dihydropyrido[3,2-f][1,4]oxazepin-3(2H)-one
(11D) 6-[3,5-bis(trifluoromethyl)benzyl]-4-(4-fluorophenyl)-5,6-dihydro-7H-pyrido[3,2-c]azepin-7-one
(12D) 6-[3,5-bis(trifluoromethyl)benzyl]-4-(4-fluorophenyl)-5,6,8,9-tetrahydro-7H-pyrido[3,2-c]azepin-7-one
(13D) 6-[3,5-bis(trifluoromethyl)benzyl]-4-(4-fluorophenyl)-5,6-dihydroprimido[5,4-f]oxazepin-7(8H)-one
(14D) 4-[3,5-bis(trifluoromethyl)benzyl]-6-(2-fluorophenyl)-2,3,4,5-tetrahydropyrido[3,2-f][1,4]oxazepine
(15D) 4-{1-[3,5-bis(trifluoromethyl)phenyl]ethyl}-6-(2-fluorophenyl)-2,3,4,5-tetrahydropyrido[3,2-f][1,4]oxazepine
(16D) 4-[3,5-bis(trifluoromethyl)benzyl]-6-(2-fluorophenyl)-4,5-dihydropyrido[3,2-f][1,4]oxazepin-3(2H)-one
(17D) 4-[3,5-bis(trifluoromethyl)benzyl]-6-(2,6-difluorophenyl)-4,5-dihydropyrido[3,2-f][1,4]oxazepin-3(2H)-one
(18D) 4-{1-[3,5-bis(trifluoromethyl)phenyl]ethyl}-6-(2-fluorophenyl)-4,5-dihydropyrido[3,2-f][1,4]oxazepin-3(2H)-one
(19D) 5-{[3,5-bis(trifluoromethyl)phenyl]sulfonyl}-7-(2-fluorophenyl)-3,4,5,6-tetrahydro-2H-pyrido[2,3-b][1,5]oxazocine
(20D) 6-[3,5-bis(trifluoromethyl)benzyl]-8-(2-fluorophenyl)-2,3,4,5,6,7-hexahydropyrido[2,3-b][1,5]oxazonine
(21D) 6-{1-[3,5-bis(trifluoromethyl)phenyl]ethyl}-8-(2-fluorophenyl)-2,3,4,5,6,7-hexahydropyrido[2,3-b][1,5]oxazonine
(22D) 4-[3,5-bis(trifluoromethyl)benzyl]-6-(2,6-difluorophenyl)-2,3,4,5-tetrahydropyrido[3,2-f][1,4]oxazepine
(23D) 4-{1-[3,5-bis(trifluoromethyl)phenyl]ethyl}-6-(2,6-difluorophenyl)-2,3,4,5-tetrahydropyrido[3,2-f][1,4]oxazepine dihydrochloride
(24D) 4-{1-[3,5-bis(trifluoromethyl)phenyl]ethyl}-6-(2,6-difluorophenyl)-2,3,4,5-tetrahydropyrido[3,2-f][1,4]oxazepine
(25D) 4-[3,5-bis(trifluoromethyl)benzyl]-6-(2,4-difluorophenyl)-4,5-dihydropyrido[3,2-f][1,4]oxazepin-3(2H)-one
(26D) 4-[3,5-bis(trifluoromethyl)benzyl]-6-(2-chlorophenyl)-4,5-dihydropyrido[3,2-f][1,4]oxazepin-3(2H)-one
(27D) 4-[3,5-bis(trifluoromethyl)benzyl]-6-(2-chlorophenyl)-2,3,4,5-tetrahydropyrido[3,2-f][1,4]oxazepine
(28D) 4-{1-[3,5-bis(trifluoromethyl)phenyl]ethyl}-6-(2-chlorophenyl)-2,3,4,5-tetrahydropyrido[3,2-f][1,4]oxazepine
(29D) 6-[3,5-bis(trifluoromethyl)benzyl]-8-phenyl-3,4,6,7-tetrahydropyrido[2,3-b][1,5]oxazonin-5(2H)-one
(30D) 6-[3,5-bis(trifluoromethyl)benzyl]-8-(4-fluorophenyl)-3,4,6,7-tetrahydropyrido[2,3-b][1,5]oxazonin-5(2H)-one
(31D) {4-[3,5-bis(trifluoromethyl)benzyl]-6-phenyl-2,3,4,5-tetrahydropyrido[3,2-f][1,4]oxazepin-3-yl}methanol
(32D) 4-[3,5-bis(trifluoromethyl)benzyl]-6-(4-fluorophenyl)-4,5-dihydropyrido[3,4-f][1,4]oxazepin-3(2H)-one
(33D) 6-phenyl-4-[3-(trifluoromethoxy)benzyl]-4,5-dihydropyrido[3,2-f][1,4]oxazepin-3(2H)-one
(34D) 4-[3,5-bis(trifluoromethyl)benzyl]-6-(2-methoxyphenyl)-4,5-dihydropyrido[3,2-f][1,4]oxazepin-3(2H)-one
(35D) 5-{[3,5-bis(trifluoromethyl)phenyl]sulfonyl}-7-(2-methoxyphenyl)-3,4,5,6-tetrahydro-2H-pyrido[2,3-b][1,5]oxazocine
(36D) 4-{1-[3,5-bis(trifluoromethyl)phenyl]ethyl}-6-(2,4-difluorophenyl)-2,3,4,5-tetrahydropyrido[3,2-f][1,4]oxazepine
(37D) 4-[3,5-bis(trifluoromethyl)benzyl]-6-(3-thienyl)-4,5-dihydropyrido[3,2-f][1,4]oxazepin-3(2H)-one
(38D) 5-{[3,5-bis(trifluoromethyl)phenyl]sulfonyl}-7-(3-thienyl)-3,4,5,6-tetrahydro-2H-pyrido[2,3-b][1,5]oxazocine
(39D) 4-[3,5-bis(trifluoromethyl)benzyl]-6-(3-furyl)-4,5-dihydropyrido[3,2-f][1,4]oxazepin-3(2H)-one
(40D) 4-[3,5-bis(trifluoromethyl)benzyl]-3-methyl-6-phenyl-2,3,4,5-tetrahydropyrido[3,2-f][1,4]oxazepine
(41D) 4-[3,5-bis(trifluoromethyl)benzoyl]-3-methyl-6-phenyl-2,3,4,5-tetrahydropyrido[3,2-f][1,4]oxazepine
(42D) 4-{[3,5-bis(trifluoromethyl)phenyl]sulfonyl}-3-methyl-6-phenyl-2,3,4,5-tetrahydropyrido[3,2-f][1,4]oxazepine
(43D) 4-{[3,5-bis(trifluoromethyl)phenyl]sulfonyl}-3,3-dimethyl-6-phenyl-2,3,4,5-tetrahydropyrido[3,2-f][1,4]oxazepine
(44D) 4-[3,5-bis(trifluoromethyl)benzyl]-6-[4-(dimethylamino)phenyl]-4,5-dihydropyrido[3,2-f][1,4]oxazepin-3(2H)-one
(45D) 6-[3-(benzyloxy)phenyl]-4-[3,5-bis(trifluoromethyl)benzyl]-4,5-dihydropyrido[3,2-f][1,4]oxazepin-3(2H)-one
(46D) 4-[3,5-bis(trifluoromethyl)benzyl]-6-(3-hydroxyphenyl)-4,5-dihydropyrido[3,2-f][1,4]oxazepin-3(2H)-one
(47D) tert-butyl (3-{4-[3,5-bis(trifluoromethyl)benzyl]-3-oxo-2,3,4,5-tetrahydropyrido[3,2-f][1,4]oxazepin-6-yl}phenoxy)acetate
(48D) (3-{4-[3,5-bis(trifluoromethyl)benzyl]-3-oxo-2,3,4,5-tetrahydropyrido[3,2-f][1,4]oxazepin-6-yl)phenoxy)acetic acid 0.5 trifluoroacetate
(49D) 6-[2-(benzyloxy)phenyl]-4-[3,5-bis(trifluoromethyl)benzyl]-4,5-dihydropyrido[3,2-f][1,4]oxazepin-3(2H)-one
(50D) 4-[3,5-bis(trifluoromethyl)benzyl]-6-(2-hydroxyphenyl)-4,5-dihydropyrido[3,2-f][1,4]oxazepin-3(2H)-one
(51D) 4-[3,5-bis(trifluoromethyl)benzyl]-6-[2-(2-methoxyethoxy)phenyl]-4,5-dihydropyrido[3,2-f][1,4]oxazepin-3(2H)-one
(52D) 4-(3,5-dichlorobenzyl)-6-phenyl-4,5-dihydropyrido[3,2-f][1,4]oxazepin-3(2H)-one
(53D) 4-(3,5-difluorobenzyl)-6-phenyl-4,5-dihydropyrido[3,2-f][1,4]oxazepin-3(2H)-one
(54D) 4-(3,5-dimethoxybenzyl)-6-phenyl-4,5-dihydropyrido[3,2-f][1,4]oxazepin-3(2H)-one
(55D) 6-phenyl-4-(3,4,5-trimethoxybenzyl)-4,5-dihydropyrido[3,2-f][1,4]oxazepin-3(2H)-one
(56D) 4-(1,3-benzodioxol-5-ylmethyl)-6-phenyl-4,5-dihydropyrido[3,2-f][1,4]oxazepin-3(2H)-one
(57D) 5-{[3,5-bis(trifluoromethyl)phenyl]sulfonyl}-7-(2-chlorophenyl)-3,4,5,6-tetrahydro-2H-pyrido[2,3-b][1,5]oxazocine
(58D) tert-butyl {3-[(3-oxo-6-phenyl-2,3-dihydropyrido[3,2-f][1,4]oxazepin-4(5H)-yl)methyl]benzyl}carbamate
(59D) 5-{[3,5-bis(trifluoromethyl)phenyl]sulfonyl}-7-pyridin-3-yl-3,4,5,6-tetrahydro-2H-pyrido[2,3-b][1,5]oxazocine
(60D) 4-[3,5-bis(trifluoromethyl)benzyl]-6-(4-fluorophenyl)-1,2,4,5-tetrahydro-3H-pyrido[2,3-e][1,4]diazepin-3-one hydrochloride
(61D) 4-[3,5-bis(trifluoromethyl)benzyl]-6-phenyl-1,2,4,5-tetrahydro-3H-pyrido[2,3-e][1,4]diazepin-3-one
(62D) 1-acetyl-4-[3,5-bis(trifluoromethyl)benzyl]-6-phenyl-1,2,4,5-tetrahydro-3H-pyrido[2,3-e][1,4]diazepin-3-one
(63D) 4-[3,5-bis(trifluoromethyl)benzyl]-1-methyl-6-phenyl-1,2,4,5-tetrahydro-3H-pyrido[2,3-e][1,4]diazepin-3-one
(64D) tert-butyl (2-{4-[3,5-bis(trifluoromethyl)benzyl]-3-oxo-2,3,4,5-tetrahydropyrido[3,2-f][1,4]oxazepin-6-yl}phenoxy) acetate
(65D) (2-{4-[3,5-bis(trifluoromethyl)benzyl]-3-oxo-2,3,4,5-tetrahydropyrido[3,2-f][1,4]oxazepin-6-yl}phenoxy)acetic acid
(66D) 2-(2-f4-[3,5-bis(trifluoromethyl)benzyl]-3-oxo-2,3,4,5-tetrahydropyrido[3,2-f][1,4]oxazepin-6-yl}phenoxy)acetamide
(67D) 6-[4-(benzyloxy)phenyl]-4-[3,5-bis(trifluoromethyl)benzyl]-4,5-dihydropyrido[3,2-f][1,4]oxazepin-3(2H)-one
(68D) 4-[3,5-bis(trifluoromethyl)benzyl]-6-(4-hydroxyphenyl)-4,5-dihydropyrido[3,2-f][1,4]oxazepin-3(2H)-one
(69D) 4-[3,5-bis(trifluoromethyl)benzyl]-6-[2-(methylthio)phenyl]-4,5-dihydropyrido[3,2-f][1,4]oxazepin-3(2H)-one
(70D) 4-[3,5-bis(trifluoromethyl)benzyl]-6-[2-(methylsulfonyl)phenyl]-4,5-dihydropyrido[3,2-f][1,4]oxazepin-3(2H)-one
(71D) 4-[3,5-bis(trifluoromethyl)benzyl]-6-[4-(methylsulfonyl)phenyl]-4,5-dihydropyrido[3,2-f][1,4]oxazepin-3(2H)-one
(72D) tert-butyl (2-{4-[3,5-bis(trifluoromethyl)benzyl]-3-oxo-2,3,4,5-tetrahydropyrido[3,2-f][1,4]oxazepin-6-yl}phenyl)carbamate
(73D) 6-(2-aminophenyl)-4-[3,5-bis(trifluoromethyl)benzyl]-4,5-dihydropyrido[3,2-f][1,4]oxazepin-3(2H)-one
(74D) N-acetyl-N-(2-{4-[3,5-bis(trifluoromethyl)benzyl]-3-oxo-2,3,4,5-tetrahydropyrido[3,2-f][1,4]oxazepin-6-yl}phenyl)acetamide
(75D) tert-butyl (4-f4-[3,5-bis(trifluoromethyl)benzyl]-3-oxo-2,3,4,5-tetrahydropyrido[3,2-f][1,4]oxazepin-6-yl}phenyl)carbamate
(76D) 6-(4-aminophenyl)-4-[3,5-bis(trifluoromethyl)benzyl]-4,5-dihydropyrido[3,2-f][1,4]oxazepin-3(2H)-one
(77D) 4-[3,5-bis(trifluoromethyl)benzyl]-6-[2-(hydroxymethyl)phenyl]-4,5-dihydropyrido[3,2-f][1,4]oxazepin-3(2H)-one
(78D) 6-[2-(benzyloxy)-6-fluorophenyl]-4-[3,5-bis(trifluoromethyl)benzyl]-4,5-dihydropyrido[3,2-f][1,4]oxazepin-3(2H)-one
(79D) 4-[3,5-bis(trifluoromethyl)benzyl]-6-(2-fluoro-6-hydroxyphenyl)-4,5-dihydropyrido[3,2-f][1,4]oxazepin-3(2H)-one
(80D) 4-[3,5-bis(trifluoromethyl)benzyl]-6-(2-methylphenyl)-4,5-dihydropyrido[3,2-f][1,4]oxazepin-3(2H)-one
(81D) N-(4-{4-[3,5-bis(trifluoromethyl)benzyl]-3-oxo-2,3,4,5-tetrahydropyrido[3,2-f][1,4]oxazepin-6-yl}phenyl)acetamide
(82D) 7-[2-(benzyloxy)phenyl]-5-{[3,5-bis(trifluoromethyl)phenyl]sulfonyl}-3,4,5,6-tetrahydro-2H-pyrido[2,3-b][1,5]oxazocine
(83D) 2-(5-([3,5-bis(trifluoromethyl)phenyl]sulfonyl)-3,4,5,6-tetrahydro-2H-pyrido[2,3-b][1,5]oxazocine-7-yl)phenol
(84D) 5-{[3,5-bis(trifluoromethyl)phenyl]sulfonyl}-7-[2-(2-methoxyethoxy)phenyl]-3,4,5,6-tetrahydro-2H-pyrido[2,3-b][1,5]oxazocine
(85D) tert-butyl {2-[2-(5-([3,5-bis(trifluoromethyl)phenyl]sulfonyl}-3,4,5,6-tetrahydro-2H-pyrido[2,3-b][1,5]oxazocin-7-yl)phenoxy]ethyl}carbamate
(86D) {2-[2-(5-{[3,5-bis(trifluoromethyl)phenyl]sulfonyl}-3,4,5,6-tetrahydro-2H-pyrido[2,3-b][1,5]oxazocin-7-yl)phenoxy]ethyl}amine dihydrochloride
(87D) 4-[2-methoxy-5-(trifluoromethoxy)benzyl]-6-phenyl-4,5-dihydropyrido[3,2-f][1,4]oxazepin-3(2H)-one
(88D) 4-(2-methoxy-5-[5-(trifluoromethyl)-1H-tetrazol-1-yl]benzyl)-6-phenyl-4,5-dihydropyrido[3,2-f][1,4]oxazepin-3(2H)-one
(89D) 6-(2-fluorophenyl)-4-(pyridin-2-ylmethyl)-4,5-dihydropyrido[3,2-f][1,4]oxazepin-3(2H)-one
(90D) 4-[3,5-bis(trifluoromethyl)benzyl]-6-pyridin-3-yl-4,5-dihydropyrido[3,2-f][1,4]oxazepin-3(2H)-one
(91D) 4-[3,5-bis(trifluoromethyl)benzyl]-6-pyridin-2-yl-4,5-dihydropyrido[3,2-f][1,4]oxazepin-3(2H)-one
(92D) 4-[3,5-bis(trifluoromethyl)benzyl]-6-(1,3-thiazol-2-yl)-4,5-dihydropyrido[3,2-f][1,4]oxazepin-3(2H)-one
(93D) 2-{4-[3,5-bis(trifluoromethyl)benzyl]-3-oxo-2,3,4,5-tetrahydropyrido[3,2-f][1,4]oxazepin-6-yl}benzamide
(94D) 4-[3,5-bis(trifluoromethyl)benzyl]-6-piperidin-1-yl-4,5-dihydropyrido[3,2-f][1,4]oxazepin-3(2H)-one
(95D) 4-[3,5-bis(trifluoromethyl)benzyl]-6-morpholin-4-yl-4,5-dihydropyrido[3,2-f][1,4]oxazepin-3(2H)-one
(96D) 4-[3,5-bis(trifluoromethyl)benzyl]-6-pyrrolidin-1-yl-4,5-dihydropyrido[3,2-f][1,4]oxazepin-3(2H)-one
(97D) 6-azepan-1-yl-4-[3,5-bis(trifluoromethyl)benzyl]-4,5-dihydropyrido [3,2-f][1,4]oxazepin-3(2H)-one
(98D) 2-{4-[3,5-bis(trifluoromethyl)benzyl]-3-oxo-2,3,4,5-tetrahydropyrido[3,2-f][1,4]oxazepin-6-yl}benzaldehyde

Furthermore, the compounds described in the following Table 1 - Table 7 are also exemplified as compound (I).

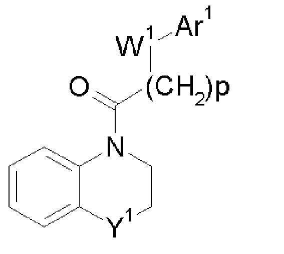

**Table 1**

| compound | p | W¹ | Y¹ | Ar¹ |
|---|---|---|---|---|
| 1E | 1 | 0 | CH₂ | 5-isoquinolyl |
| 2E | 3 | 0 | CH₂ | 2-methoxy-4-methylphenyl |
| 3E | 3 | 0 | CH₂ | 1-naphthyl |
| 4E | 3 | 0 | CH₂ | 2-benzyloxyphenyl |
| 5E | 3 | 0 | CH₂ | 3-biphenyl |
| 6E | 3 | 0 | CH₂ | 2-benzoylphenyl |
| 7E | 3 | 0 | CH₂ | 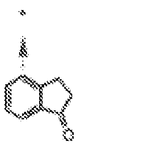 |
| 8E | 3 | 0 | CH₂ | 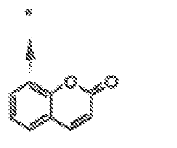 |
| 9E | 3 | 0 | CH₂ | 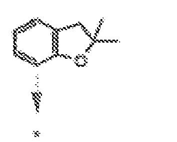 |
| 10E | 3 | 0 | CH₂ | 2-tert-butyl-5-methylphenyl |
| 11E | 3 | 0 | CH₂ | 5-isoquinolyl |

**Table 2**

| compound | p | W¹ | Y¹ | Ar¹ |
|---|---|---|---|---|
| 12E | 3 | 0 | CH₂ | 4-quinolyl |
| 13E | 3 | 0 | CH₂ | 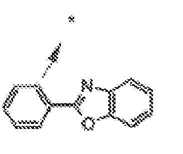 |
| 14E | 3 | 0 | CH₂ | 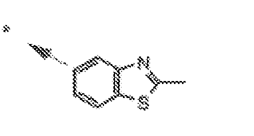 |
| 15E | 3 | 0 | CH₂ | 3,5-dimethylphenyl |
| 16E | 3 | 0 | CH₂ | 5,6,7,8-tetrahydro-2-naphthyl |
| 17E | 3 | 0 | CH₂ | 4-methoxy-1-naphthyl |
| 18E | 3 | 0 | CH₂ | 2-ethylphenyl |
| 19E | 3 | 0 | CH₂ | 4-chloro-1-naphthyl |
| 20E | 3 | 0 | CH₂ | 2-chlorophenyl |
| 21E | 3 | 0 | CH₂ | 3,5-dichlorophenyl |
| 22E | 3 | 0 | CH₂ | 2-bromo-4-fluorophenyl |
| 23E | 3 | 0 | CH₂ | 4-bromo-3-methylphenyl |
| 24E | 3 | 0 | CH₂ | 2,6-dichloro-4-fluorophenyl |
| 25E | 3 | 0 | CH₂ | 4-cyanophenyl |
| 26E | 3 | 0 | CH₂ | 2-trifluoromethylphenyl |
| 27E | 3 | 0 | CH₂ | 3,5-bis(trifluoromethyl)phenyl |
| 28E | 3 | 0 | CH₂ | 2,5-difluorophenyl |
| 29E | 3 | 0 | CH₂ | 4-cyano-2-methoxyphenyl |

**Table 3**

| compound | p | W¹ | Y¹ | Ar¹ |
|---|---|---|---|---|
| 30E | 3 | 0 | CH₂ | 1,3-benzodioxol-5-yl |
| 31E | 3 | 0 | CH₂ | 3,4,5-trimethoxyphenyl |
| 32E | 3 | 0 | CH₂ | 3-methoxyphenyl |
| 33E | 3 | 0 | CH₂ | 3-chlorophenyl |
| 34E | 4 | 0 | CH₂ | 2-methoxy-4-methylphenyl |
| 35E | 4 | 0 | CH₂ | 1-naphthyl |
| 36E | 4 | 0 | CH₂ | 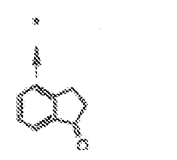 |
| 37E | 4 | 0 | CH₂ | 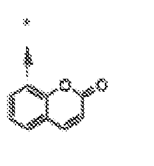 |
| 38E | 4 | 0 | CH₂ | 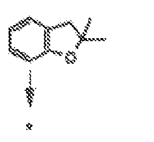 |
| 39E | 4 | 0 | CH₂ | 2-tert-butyl-5-methylphenyl |
| 40E | 4 | 0 | CH₂ | 5-isoquinolyl |
| 41E | 4 | 0 | CH₂ | 4-quinolyl |
| 42E | 4 | 0 | CH₂ | 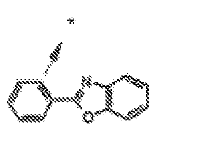 |

**[Table 4]**

| compound | p | W¹ | Y¹ | Ar¹ |
|---|---|---|---|---|
| 43E | 4 | 0 | CH₂ | 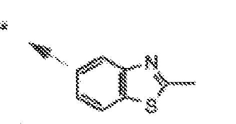 |
| 44E | 4 | 0 | CH₂ | 4,6-dimethyl-2-pyrimidinyl |
| 45E | 4 | 0 | CH₂ | 3,5-dimethylphenyl |
| 46E | 4 | 0 | CH₂ | 5,6,7,8-tetrahydro-2-naphthyl |
| 47E | 4 | 0 | CH₂ | 4-methoxy-1-naphthyl |
| 48E | 4 | 0 | CH₂ | 2-ethylphenyl |
| 49E | 4 | 0 | CH₂ | 4-chloro-1-naphthyl |
| 50E | 4 | 0 | CH₂ | 2-iodophenyl |
| 51E | 4 | 0 | CH₂ | 2-chlorophenyl |
| 52E | 4 | 0 | CH₂ | 3,5-dichlorophenyl |
| 53E | 4 | 0 | CH₂ | 2-bromo-4-fluorophenyl |
| 54E | 4 | 0 | CH₂ | 4-bromo-3-methylphenyl |
| 55E | 4 | 0 | CH₂ | 2,6-dichloro-4-fluorophenyl |
| 56E | 4 | 0 | CH₂ | 4-cyanophenyl |
| 57E | 4 | 0 | CH₂ | 2-trifluoromethylphenyl |
| 58E | 4 | 0 | CH₂ | 3,5-bis(trifluoromethyl)phenyl |
| 59E | 4 | 0 | CH₂ | 2,5-difluorophenyl |
| 60E | 4 | 0 | CH₂ | 3-methyl-4-(methylthio)phenyl |
| 61E | 4 | 0 | CH₂ | 4-cyano-2-methoxyphenyl |
| 62E | 4 | 0 | CH₂ | 3,4,5-trimethoxyphenyl |

**[Table 5]**

| compound | p | W¹ | Y¹ | Ar¹ |
|---|---|---|---|---|
| 63E | 4 | 0 | CH₂ | 3-chlorophenyl |
| 64E | 3 | 0 | CH₂ | 5-isoquinolinyl |
| 65E | 3 | 0 | 0 | 3,5-dimethylphenyl |
| 66E | 3 | 0 | 0 | 1-naphthyl |
| 67E | 3 | 0 | 0 | 3,5-bis(trifluoromethyl)phenyl |
| 68E | 3 | 0 | 0 | 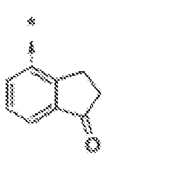 |
| 69E | 3 | 0 | 0 | 5-isoquinolinyl |
| 70E | 3 | 0 | 0 | 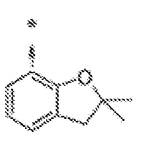 |
| 71E | 3 | 0 | 0 | 3,5-dichlorophenyl |
| 72E | 3 | 0 | 0 | 3,4,5-trimethoxyphenyl |
| 73E | 3 | 0 | S | 3,5-dimethylphenyl |
| 74E | 3 | 0 | S | 1-naphthyl |
| 75E | 3 | 0 | S | 3,5-bis(trifluoromethyl)phenyl |
| 76E | 3 | 0 | S | 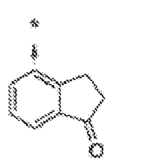 |
| 77E | 3 | 0 | S | 5-isoquinolinyl |

**[Table 6]**

| compound | p | W¹ | Y¹ | Ar¹ |
|---|---|---|---|---|
| 78E | 3 | 0 | S | 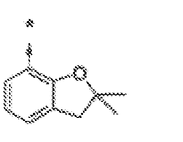 |
| 79E | 3 | 0 | S | 3,5-dichlorophenyl |
| 80E | 3 | 0 | S | 3,4,5-trimethoxyphenyl |
| 81E | 3 | S | 0 | 3,5-dimethylphenyl |
| 82E | 3 | S | 0 | 1-naphthyl |
| 83E | 3 | S | 0 | 3,5-bis(trifluoromethyl)phenyl |
| 84E | 3 | S | 0 | 7-(trifluoromethyl)-4-quinolinyl |
| 85E | 3 | S | S | 3,5-dimethylphenyl |
| 86E | 3 | S | S | 1-naphthyl |
| 87E | 3 | S | S | 3,5-bis(trifluoromethyl)phenyl |
| 88E | 3 | S | S | 3-fluorophenyl |
| 89E | 3 | S | S | 3-(trifluoromethyl)phenyl |
| 90E | 3 | S | S | 7-(trifluoromethyl)-4-quinolinyl |
| 91E | 3 | S | CH₂ | 2,4-dimethylphenyl |
| 92E | 3 | S | CH₂ | 4-methoxyphenyl |
| 93E | 3 | S | CH₂ | 2,4-difluorophenyl |
| 94E | 3 | S | CH₂ | 7-(trifluoromethyl)-4-quinolinyl |
| 95E | 3 | S | CH₂ | 3,4-dichlorophenyl |
| 96E | 3 | S | CH₂ | 4-methylphenyl |
| 97E | 3 | S | CH₂ | 3-bromophenyl |
| 98E | 3 | S | CH₂ | 3,5-dimethylphenyl |
| 99E | 3 | S | CH₂ | 1-naphthyl |

**[Table 7]**

| compound | p | W¹ | Y¹ | Ar¹ |
|---|---|---|---|---|
| 100E | 3 | S | CH₂ | 3,5-bis(trifluoromethyl)phenyl |
| 101E | 3 | S | CH₂ | 3-fluorophenyl |
| 102E | 3 | S | CH₂ | 3-trifluoromethylphenyl |
| 103E | 4 | S | CH₂ | 2,4-dimethylphenyl |
| 104E | 4 | S | CH₂ | 3,4-dichlorophenyl |
| 105E | 4 | S | CH₂ | 3-bromophenyl |
| 106E | 3 | SO₂ | CH₂ | 3,5-dimethylphenyl |
| 107E | 3 | SO₂ | CH₂ | 3,5-bis(trifluoromethyl)phenyl |
| 108E | 3 | SO₂ | 0 | 3,5-dimethylphenyl |
| 109E | 3 | SO₂ | 0 | 3,5-bis(trifluoromethyl)phenyl |

Moreover, the following compounds are also exemplified as compound (I).
(110E) 5-[4-(3,4-dihydroquinolin-1(2H)-yl)-4-oxobutoxy]-3,4-dihydronaphthalen-1(2H)-one
(111E) 5-[4-(2,3-dihydro-4H-1,4-benzoxazin-4-yl)-4-oxobutoxy]-3,4-dihydronaphthalen-1(2H)-one
(112E) methyl 3-[4-(3,4-dihydroquinolin-1(2H)-yl)-4-oxobutoxy]benzoate
(113E) 3-[4-(3,4-dihydroquinolin-1(2H)-yl)-4-oxobutoxy]benzoic acid
(114E) 4-[4-oxo-4-(2,3,4,5-tetrahydro-1H-1-benzoazepin-1-yl)butoxy]indan-1-one
(115E) 4-[4-(6-fluoro-3,4-dihydroquinolin-1(2H)-yl)-4-oxobutoxy]indan-1-one
(116E) 4-[4-(4-methyl-3,4-dihydroquinolin-1(2H)-yl)-4-oxobutoxy]indan-1-one
(117E) 4-[4-(2-methyl-3,4-dihydroquinolin-1(2H)-yl)-4-oxobutoxy]indan-1-one
(118E) 4-[4-(3,4-dihydroquinolin-1(2H)-yl)-4-oxobutoxy]indan-1-one
(119E) 4-[4-(3,4-dihydroquinolin-1(2H)-yl)-4-oxobutoxy]indan-1-ol
(120E) 4-[4-(3,4-dihydroquinolin-1(2H)-yl)-4-oxobutoxy]indan-1-one oxime
(121E) 4-[4-(3,4-dihydroquinolin-1(2H)-yl)-4-oxobutoxy]indan-1-one O-methyloxime
(122E) 4-[4-(3,4-dihydroquinolin-1(2H)-yl)-4-oxobutoxy]-2,2-dimethylindan-1-one
(123E) 1-{3-[4-(3,4-dihydroquinolin-1(2H)-yl)-4-oxobutoxy]phenyl}ethanone
(124E) N-{3-[4-(3,4-dihydroquinolin-1(2H)-yl)-4-oxobutoxy]phenyl}acetamide
(125E) 1-{4-[3-(pyrrolidin-1-ylcarbonyl)phenoxy]butanoyl}-1,2,3,4-tetrahydroquinoline
(126E) 3-[4-(3,4-dihydroquinolin-1(2H)-yl)-4-oxobutoxy]benzamide
(127E) 3-[4-(3,4-dihydroquinolin-1(2H)-yl)-4-oxobutoxy]-N,N-dimethylbenzamide
(128E) 4-{[(12)-4-(3,4-dihydroquinolin-1(2H)-yl)-4-oxobut-1-en-1-yl]oxy}indan-1-one
(129E) 5-[4-(3,4-dihydroquinolin-1(2H)-yl)-4-oxobutoxy]-1,2,3,4-tetrahydronaphthalen-1-ol
(130E) 4-[4-(3,4-dihydroquinolin-1(2H)-yl)-4-oxobutoxy]-1-methylindan-1-ol
(131E) 5-[4-(3,4-dihydroquinolin-1(2H)-yl)-4-oxobutoxy]-3,4-dihydronaphthalen-1(2H)-one oxime
(132E) 5-[4-(3,4-dihydroquinolin-1(2H)-yl)-4-oxobutoxy]-2,2-dimethyl-3,4-dihydronaphthalen-1(2H)-one
(133E) 1-{4-[(1-methoxy-2,3-dihydro-1H-inden-4-yl)oxy]butanoyl}-1,2,3,4-tetrahydroquinoline
(134E) 5-[4-(6-fluoro-3,4-dihydroquinolin-1(2H)-yl)-4-oxobutoxy]-3,4-dihydronaphthalen-1(2H)-one
(135E) 5-[4-(6-fluoro-3,4-dihydroquinolin-1(2H)-yl)-4-oxobutoxy]-1,2,3,4-tetrahydronaphthalen-1-ol
(136E) 1-[4-(3,4-dihydroquinolin-1(2H)-yl)-4-oxobutoxy]-6,7,8,9-tetrahydro-5H-benzo[7]annulen-5-one
(137E) 5-[4-(3,4-dihydroquinolin-1(2H)-yl)-4-oxobutoxy]quinoline
(138E) 5-[4-(3,4-dihydroquinolin-1(2H)-yl)-4-oxobutoxy]quinoline 1-oxide
(139E) 5-[4-(3,4-dihydroquinolin-1(2H)-yl)-4-oxobutoxy]-1,2,3,4-tetrahydronaphthalen-1-amine
(140E) 4-[4-(3,4-dihydroquinoline-1(2H)-yl)-4-oxobutoxy]indan-1-amine
(141E) 1-[(isoquinolin-5-yloxy)acetyl]-1,2,3,4-tetrahydroquinoline
(142E) 6-fluoro-1-[(isoquinolin-5-yloxy)acetyl]-1,2,3,4-tetrahydroquinoline
(143E) 6-chloro-1-[(isoquinolin-5-yloxy)acetyl]-1,2,3,4-tetrahydroquinoline
(144E) 1-{[(2-oxidoisoquinolin-5-yl)oxy]acetyl}-1,2,3,4-tetrahydroquinoline
(145E) methyl1-[(isoquinolin-5-yloxy)acetyl]-1,2,3,4-tetrahydroquinoline-6-carboxylate
(146E) 6-fluoro-1-([(2-oxidoisoquinolin-5-yl)oxy]acetyl}-1,2,3,4-tetrahydroquinoline
(147E) 1-[(isoquinolin-5-yloxy)acetyl]-1,2,3,4-tetrahydroquinoline-6-carboxylic acid
(148E) 5-[2-(6-fluoro-3,4-dihydroquinolin-1(2H)-yl)-2-oxoethoxy]isoquinoline-1-carbonitrile
(149E) 1-[(isoquinolin-5-yloxy)acetyl]-2-methyl-1,2,3,4-tetrahydroquinoline hydrochloride
(150E) 6-fluoro-1-[2-(isoquinolin-5-yloxy)propanoyl]-1,2,3,4-tetrahydroquinoline
(151E) 6-fluoro-1-[(isoquinolin-5-yloxy)acetyl]-2-methyl-1,2,3,4-tetrahydroquinoline
(152E) methyl1-[(isoquinolin-5-yloxy)acetyl]-1,2,3,4-tetrahydroquinoline-2-carboxylate
(153E) 1-[(isoquinolin-5-yloxy)acetyl]-1,2,3,4-tetrahydroquinoline-2-carboxylic acid
(154E) 7-fluoro-4-[(isoquinolin-5-yloxy)acetyl]-3-methyl-3,4-dihydroquinoxalin-2(1H)-one
(155E) 3-ethyl-7-fluoro-4-[(isoquinolin-5-yloxy)acetyl]-3,4-dihydroquinoxalin-2(1H)-one
(156E) 7-fluoro-3-isobutyl-4-[(isoquinolin-5-yloxy)acetyl]-3,4-dihydroquinoxalin-2(1H)-one
(157E) 3-benzyl-7-fluoro-4-[(isoquinolin-5-yloxy)acetyl]-3,4-dihydroquinoxalin-2(1H)-one (158E) 4-[(isoquinolin-5-yloxy)acetyl]-3-methyl-3,4-dihydroquinoxalin-2(1H)-one
(159E) 4-[(isoquinolin-5-yloxy)acetyl]-3,4-dihydroquinoxalin-2(1H)-one
(160E) 4-[2-(isoquinolin-5-yloxy)propanoyl]-3,4-dihydroquinoxalin-2(1H)-one
(161E) 4-[(isoquinolin-5-yloxy)acetyl]-3,3-dimethyl-3,4-dihydroquinoxalin-2(1H)-one
(162E) 7-fluoro-4-[(isoquinolin-5-yloxy)acetyl]-1,3-dimethyl-3,4-dihydroquinoxalin-2(1H)-one
(163E) [7-fluoro-4-[(isoquinolin-5-yloxy)acetyl]-3-methyl-2-oxo-3,4-dihydroquinoxalin-1(2H)-yl]acetic acid
(164E-1) (3S)-7-fluoro-4-[(isoquinolin-5-yloxy)acetyl]-3-methyl-3,4-dihydroquinoxalin-2(1H)-one
(164E-2) (3R)-7-fluoro-4-[(isoquinolin-5-yloxy)acetyl]-3-methyl-3,4-dihydroquinoxalin-2(1H)-one
(165E) 7-fluoro-4-[(isoquinolin-5-yloxy)acetyl]-3,3-dimethyl-3,4-dihydroquinoxalin-2(1H)-one
(166E) 7-fluoro-3,3-dimethyl-4-{[(1-methylisoquinolin-5-yl)oxy]acetyl}-3,4-dihydroquinoxalin-2(1H)-one
(167E) 7-fluoro-3,3-dimethyl-4-{[(3-methylisoquinolin-5-yl)oxy]acetyl}-3,4-dihydroquinoxalin-2(1H)-one
(168E) 4-{[(1,3-dimethylisoquinolin-5-yl)oxy]acetyl}-7-fluoro-3,3-dimethyl-3,4-dihydroquinoxalin-2(1H)-one
(169E) 7-fluoro-3,3-dimethyl-4-{[(2-methyl-1-oxo-1,2,3,4-tetrahydroisoquinolin-5-yl)oxy]acetyl}-3,4-dihydroquinoxalin-2(1H)-one
(170E) 7-fluoro-3,3-dimethyl-4-{[(2-methyl-1-oxo-1,2-dihydroisoquinolin-5-yl)oxy]acetyl}-3,4-dihydroquinoxalin-2(1H)-one
(171E) 7-fluoro-3,3-dimethyl-4-{[(1-oxo-1,2,3,4-tetrahydroisoquinolin-5-yl)oxy]acetyl}-3,4-dihydroquinoxalin-2(1H)-one
(172E) 7-fluoro-3,3-dimethyl-4-{[(1-oxo-1,2-dihydroisoquinolin-5-yl)oxy]acetyl}-3,4-dihydroquinoxalin-2(1H)-one
(173E) 7-fluoro-4-[(isoquinolin-5-yloxy)acetyl]-1,3,3-trimethyl-3,4-dihydroquinoxalin-2(1H)-one
(174E) 1-ethyl-7-fluoro-4-[(isoquinolin-5-yloxy)acetyl]-3,3-dimethyl-3,4-dihydroquinoxalin-2(1H)-one
(175E) 7-fluoro-4-[(isoquinolin-5-yloxy)acetyl]-3,3-dimethyl-1-propyl-3,4-dihydroquinoxalin-2(1H)-one
(176E) 1-butyl-7-fluoro-4-[(isoquinolin-5-yloxy)acetyl]-3,3-dimethyl-3,4-dihydroquinoxalin-2(1H)-one
(177E) tert-butyl [7-fluoro-4-[(isoquinolin-5-yloxy)acetyl]-3,3-dimethyl-2-oxo-3,4-dihydroquinoxalin-1(2H)-yl]acetate
(178E) [7-fluoro-4-[(isoquinolin-5-yloxy)acetyl]-3,3-dimethyl-2-oxo-3,4-dihydroquinoxalin-1(2H)-yl]acetic acid
(179E) tert-butyl 4-[7-fluoro-4-[(isoquinolin-5-yloxy)acetyl]-3,3-dimethyl-2-oxo-3,4-dihydroquinoxalin-1(2H)-yl]butanoate
(180E) 4-[7-fluoro-4-[(isoquinolin-5-yloxy)acetyl]-3,3-dimethyl-2-oxo-3,4-dihydroquinoxalin-1(2H)-yl]butanoic acid
(181E) 7-fluoro-1-(3-hydroxypropyl)-4-[(isoquinolin-5-yloxy)acetyl]-3,3-dimethyl-3,4-dihydroquinoxalin-2(1H)-one
(182E) 7-fluoro-1-(2-hydroxyethyl)-4-[(isoquinolin-5-yloxy)acetyl]-3,3-dimethyl-3,4-dihydroquinoxalin-2(1H)-one
(183E) methyl [7-fluoro-4-[(isoquinolin-5-yloxy)acetyl]-3,3-dimethyl-2-oxo-3,4-dihydroquinoxalin-1(2H)-yl]acetate
(184E) [7-fluoro-4-[(isoquinolin-5-yloxy)acetyl]-3,3-dimethyl-2-oxo-3,4-dihydroquinoxalin-1(2H)-yl]acetonitrile
(185E) 2-[7-fluoro-4-[(isoquinolin-5-yloxy)acetyl]-3,3-dimethyl-2-oxo-3,4-dihydroquinoxalin-1(2H)-yl]acetamide
(186E) 2-{3-[7-fluoro-4-[(isoquinolin-5-yloxy)acetyl]-3,3-dimethyl-2-oxo-3,4-dihydroquinoxalin-1(2H)-yl]propyl}-1H-isoindole-1,3(2H)-dione
(187E) tert-butyl {3-[7-fluoro-4-[(isoquinolin-5-yloxy)acetyl]-3,3-dimethyl-2-oxo-3,4-dihydroquinoxalin-1(2H)-yl]propyl}carbamate
(188E) 1-(3-aminopropyl)-7-fluoro-4-[(isoquinolin-5-yloxy)acetyl]-3,3-dimethyl-3,4-dihydroquinoxalin-2(1H)-one
(189E) 7-fluoro-1,3,3-trimethyl-4-{[(2-oxideisoquinolin-5-yl)oxy]acetyl}-3,4-dihydroquinoxalin-2(1H)-one
(190E) 4-{[(1-chloroisoquinolin-5-yl)oxy]acetyl}-7-fluoro-1,3,3-trimethyl-3,4-dihydroquinoxalin-2(1H)-one
(191E) 7-fluoro-3,3-dimethyl-4-{[(1-methyl-2-oxidoisoquinolin-5-yl)oxy]acetyl}-3,4-dihydroquinoxalin-2(1H)-one
(192E) methyl {5-[2-(6-fluoro-2,2-dimethyl-3-oxo-3,4-dihydroquinoxaline-1(2H)-yl)-2-oxoethoxy]isoquinolin-1-yl}acetate
(193E) 7-fluoro-4-({[1-(hydroxymethyl)isoquinolin-5-yl]oxy}acetyl)-3,3-dimethyl-3,4-dihydroquinoxalin-2(1H)-one
(194E) 4-{4-[3,5-bis(trifluoromethyl)phenoxy]butanoyl}-7-fluoro-3,4-dihydroquinoxalin-2(1H)-one
(195E) 4-{4-[3,5-bis(trifluoromethyl)phenoxy]butanoyl}-7-fluoro-3-methyl-3,4-dihydroquinoxalin-2(1H)-one
(196E) 7-fluoro-3-methyl-4-{4-[(1-oxo-2,3-dihydro-1H-inden-4-yl)oxy]butanoyl}-3,4-dihydroquinoxalin-2(1H)-one
(197E) 7-fluoro-4-{4-[(1-oxo-2,3-dihydro-1H-inden-4-yl)oxy]butanoyl}-3,4-dihydroquinoxalin-2(1H)-one
(198E) 7-fluoro-1-methyl-4-{4-[(1-oxo-2,3-dihydro-1H-inden-4-yl)oxy]butanoyl}-3,4-dihydroquinoxalin-2(1H)-one
(199E) 7-fluoro-1,3-dimethyl-4-{4-[(1-oxo-2,3-dihydro-1H-inden-4-yl)oxy]butanoyl}-3,4-dihydroquinoxalin-2(1H)-one
(200E) 4-{4-[3,5-bis(trifluoromethyl)phenoxy]butanoyl}-7-fluoro-1-methyl-3,4-dihydroquinoxalin-2(1H)-one
(201E) 4-[4-(6-chloro-3,4-dihydroquinolin-1(2H)-yl)-4-oxobutoxy]indan-1-one
(202E) 4-[4-(6-methyl-3,4-dihydroquinolin-1(2H)-yl)-4-oxobutoxy]indan-1-one

Of compounds (I), for example, preferable specific compounds are the following compounds.
4-[3,5-bis(trifluoromethyl)benzyl]-6-(2-fluorophenyl)-4,5-dihydropyrido[3,2-f][1,4]oxazepin-3(2H)-one (16D)
4-{1-[3,5-bis(trifluoromethyl)phenyl]ethyl}-6-(2-fluorophenyl)-4,5-dihydropyrido[3,2-f][1,4]oxazepin-3(2H)-one (18D)
7-fluoro-4-[(isoquinolin-5-yloxy)acetyl]-1,3,3-trimethyl-3,4-dihydroquinoxalin-2(1H)-one (173E)
4-{1-[3,5-bis(trifluoromethyl)phenyl]ethyl}-6-phenyl-2,3,4,5-tetrahydropyrido[3,2-f][1,4]oxazepine hydrochloride (33C)
4-[3,5-bis(trifluoromethyl)benzyl]-6-(2-hydroxyphenyl)-4,5-dihydropyrido[3,2-f][1,4]oxazepin-3(2H)-one (50D)

Examples of a compound having a TGR5 receptor agonistic activity or a particular fused ring compound (e.g., compounds (II), (III)) that can be contained as an active ingredient of the agent for the prophylaxis or treatment of irritable bowel syndrome of the present invention include the compounds described in WO2004-067008, W02004-043468 and the like, which can be produced according to the methods described in pamphalets. These compounds can be produced according to the method described in the pamphalets.
Compound (I) or a salt thereof, or compound (II) or a salt thereof can be produced according to a method known per se, for example, the method described in WO98/47882 or EP-A-733632. Particularly, compound (II') or a salt thereof can be produced based on the description of JP-A-2006-56881. Furthermore, compound (III) or a salt thereof can be produced based on the description of JP-A-2006-63064.
In the present specification, the above-mentioned compound that can be contained as an active ingredient in the agent for the prophylaxis or treatment of irritable bowel syndrome of the present invention is conveniently referred to as the compound of the present invention.

The compound of the present invention may form a salt. As the salt, a pharmacologically acceptable salt is preferable. For example, salt with inorganic base, salt with organic base, salt with inorganic acid, salt with organic acid, salt with basic or acidic amino acid and the like can be mentioned.

Preferable examples of the salt with inorganic base include alkali metal salts such as sodium salt, potassium salt, lithium salt and the like; alkaline earth metal salts such as calcium salt, magnesium salt and the like; aluminum salt, ammonium salt and the like.
Preferable examples of the salt with organic base include salts with trimethylamine, triethylamine, pyridine, picoline, ethanolamine, diethanolamine, triethanolamine, dicyclohexylamine, N,N'-dibenzylethylenediamine and the like.

Preferable examples of the salt with inorganic acid include salts with hydrochloric acid, hydrobromic acid, nitric acid, sulfuric acid, phosphoric acid and the like.
Preferable examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include salts with arginine, lysine, ornithine and the like.
Preferable examples of the salt with acidic amino acid include salts with aspartic acid, glutamic acid and the like.

When the compound of the present invention contains an optical isomer, a stereoisomer, a positional isomer or a rotational isomer, these are also encompassed in the compound of the present invention, and can be obtained as a single product according to a synthesis method and separation method known *per se.* For example, when the compound of the present invention has an optical isomer, an optical isomer resolved from this compound is also encompassed in the compound of the present invention.

The optical isomer can be produced by a method known *per se.* To be specific, an optically active synthetic intermediate is used, or the final racemate product is subjected to optical resolution according to a conventional method to give an optical isomer.
The method of optical resolution may be a method known *per se,* such as a fractional recrystallization method, a chiral column method, a diastereomer method and the like.

### 1) Fractional recrystallization method

A salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine and the like) is formed, which is separated by a fractional recrystallization method, and a free optical isomer is obtained by a neutralization step where desired.

### 2) Chiral column method

A racemate or a salt thereof is applied to a column for separation of an optical isomer (chiral column) to allow separation. In the case of a liquid chromatography, for example, a mixture of an optical isomer is applied to a chiral column such as ENANTIO-OVM (manufactured by Tosoh Corporation) or CHIRAL series (manufactured by Daicel Chemical Industries, Ltd.) and the like, and developed with water, various buffers (e.g., phosphate buffer) and organic solvents (e.g., ethanol, methanol, isopropanol, acetonitrile, trifluoroacetic acid, diethylamine and the like) solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A racemic mixture is prepared into a diastereomeric mixture by chemical reaction with an optically active reagent, which is prepared into a single substance by a typical separation means (e.g., fractional recrystallization, chromatography method and the like) and the like, and subjected to a chemical treatment such as hydrolysis reaction and the like to separate an optically active reagent moiety, whereby an optical isomer is obtained. For example, when the compound of the present invention contains hydroxy or primary or secondary amino in a molecule, the compound and an optically active organic acid (e.g., MTPA [α-methoxy-α-(trifluoromethyl)phenylacetic acid], (-)-menthoxyacetic acid and the like) and the like are subjected to condensation reaction to give an ester form diastereomer or amide form diastereomer, respectively. When the compound of the present invention has a carboxylic acid group, this compound and an optically active amine or an optically alcohol reagent are subjected to condensation reaction to give an amide form diastereomer or ester form diastereomer, respectively. The separated diastereomer is converted to an optical isomer of the original compound by acidic hydrolysis or basic hydrolysis reaction.

A prodrug of the compound of the present invention is a compound that converts to the present invention, for example, compound (I) due to the reaction by enzyme, gastric acid and the like under the physiological conditions in the body; that is, a compound that converts to compound (I) by enzymatic oxidation, reduction, hydrolysis and the like, and a compound that converts to compound (I) by hydrolysis and the like by gastric acid and the like. Examples of a prodrug of compound (I) include a compound wherein an amino group of compound (I) is acylated, alkylated or phosphorylated (e.g., a compound where an amino group of compound (I) is eicosanoylated, alanylated, pentylaminocarbonylated, (5-methyl-2-oxo-1,3-dioxolen-4-yl)methoxycarbonylated, tetrahydrofuranylated, tetrahydropyranylated, pyrrolidylmethylated, pivaloyloxymethylated, tert-butylated and the like); a compound wherein a hydroxy group of compound (I) is acylated, alkylated, phosphorylated or borated (e.g., a compound where a hydroxy group of compound (I) is acetylated, palmitoylated, propanoylated, pivaloylated, succinylated, fumarylated, alanylated, dimethylaminomethylcarbonylated, tetrahydropyranylated and the like); a compound wherein a carboxyl group of compound (I) is esterified or amidated (*e.g*., a compound where a carboxyl group of compound (I) is ethyl esterified, phenyl esterified, carboxymethyl esterified, dimethylaminomethyl esterified, pivaloyloxymethyl esterified, ethoxycarbonyloxyethyl esterified, phthalizyl esterified, (5-methyl-2-oxo-1,3-dioxolen-4-yl)methyl esterified, cyclohexyloxycarbonylethyl esterified, methylamidated and the like) and the like. These compounds can be produced from compound (I) by a method known *per se.*
A prodrug of the compound of the present invention may be a compound that converts to the compound of the present invention under physiological conditions as described in IYAKUHIN NO KAIHATSU, vol. 7, BUNSHI SEKKEI, 163-198, Hirokawa Shoten (1990).

In addition, the compound of the present invention may be labeled with an isotope (e.g., ³H, ¹⁴C, ³⁵S, ¹²⁵I and the like) or the like.
Furthermore, the compound of the present invention may be a non-solvate (e.g., anhydride), a solvate (e.g., hydrate), or a deuterium exchanged compound.

The compound of the present invention shows low toxicity (e.g., acute toxicity, chronic toxicity, genetic toxicity, reproductive toxicity, cardiotoxicity, drug interaction, carcinogenicity), and can be directly used safely as an agent for the prophylaxis or treatment of irritable bowel syndrome, or in the form of a pharmaceutical composition by mixing with a pharmacologically acceptable carrier and the like. Of the agents for the prophylaxis or treatment of irritable bowel syndrome, it can be used as a diarrhea type agent for the prophylaxis or treatment of irritable bowel syndrome.

Here, various organic or inorganic carriers conventionally used as materials for pharmaceutical preparations are used as the pharmacologically acceptable carrier, which are added as excipient, lubricant, binder, disintegrant for solid preparations; and solvent, solubilizing agents, suspending agent, isotonicity agent, buffer, soothing agent and the like for liquid preparations. Where necessary, additive for pharmaceutical preparations such as preservative, antioxidant, colorant, sweetening agent and the like can be used.

Preferable examples of the excipient include lactose, sucrose, D-mannitol, D-sorbitol, starch, pregelatinized starch, dextrin, crystalline cellulose, low-substituted hydroxypropyl cellulose, sodium carboxymethylcellulose, gum acacia, pullulan, light anhydrous silicic acid, synthetic aluminum silicate, magnesium aluminate metasilicate and the like.

Preferable examples of the lubricant include magnesium stearate, calcium stearate, talc, colloidal silica and the like.

Preferable examples of the binder include pregelatinized starch, saccharose, gelatin, gum acacia, methylcellulose, carboxymethylcellulose, sodium carboxymethylcellulose, crystalline cellulose, sucrose, D-mannitol, trehalose, dextrin, pullulan, hydroxypropyl cellulose, hydroxypropyl methylcellulose, polyvinylpyrrolidone and the like.

Preferable examples of the disintegrant include lactose, sucrose, starch, carboxymethylcellulose, calcium carboxymethylcellulose, sodium croscarmellose, sodium carboxymethyl starch, light anhydrous silicic acid, low-substituted hydroxypropyl cellulose and the like.

Preferable examples of the solvent include water for injection, physiological brine, Ringer's solution, alcohol, propylene glycol, polyethylene glycol, sesame oil, corn oil, olive oil, cottonseed oil and the like.

Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil; and the like.

Preferable examples of the isotonicity agent include sodium chloride, glycerol, D-mannitol, D-sorbitol, glucose and the like.

Preferable examples of the buffer include phosphate buffer, acetate buffer, carbonate buffer, citrate buffer and the like.

Preferable examples of the soothing agent include benzyl alcohol and the like. Preferable examples of the preservative include p-oxybenzoates, chlorobutanol, benzyl alcohol, phenethyl alcohol, dehydroacetic acid, sorbic acid and the like.

Preferable examples of the antioxidant include sulfite, ascorbate and the like.

Preferable examples of the colorant include water-soluble edible tar pigments (e.g., foodcolors such as Food Color Red Nos. 2 and 3, Food Color Yellow Nos. 4 and 5, Food Color Blue Nos. 1 and 2 and the like), water insoluble lake pigments (e.g., aluminum salt of the aforementioned water-soluble edible tar pigment and the like), natural pigments (e.g., β-carotene, chlorophil, ferric oxide red etc.) and the like.

Preferable examples of the sweetening agent include saccharin sodium, dipotassium glycyrrhizinate, aspartame, stevia and the like.

The above-mentioned pharmaceutical composition can be produced according to a method conventionally used in the field of pharmaceutical preparation, such as the method described in Japan Pharmacopoeia (e.g., 13th Ed.). The content of the compound of the present invention in the pharmaceutical composition is, for example, 0.1-100 wt% of the whole composition.

The dosage form of the pharmaceutical composition is, for example, an oral agent such as tablets (inclusive of sublingual tablets and orally disintegrable tablets), capsules (inclusive of soft capsules and micro capsules), powders, granules, troches, syrups, orally disintegrable film and the like; or a parenteral agent such as injections (e.g., subcutaneous injections, intravenous injections, intramuscular injections, intraperitoneal injections, drip infusions etc.), external agents (e.g., transdermal preparations, ointments etc.), suppositories (e.g., rectal suppositories, vaginal suppositories etc.), pellets, nasal preparations, pulmonary preparations (inhalations), ophthalmic preparations and the like. These agents may be controlled-release preparations such as rapid-release preparations and sustained-release preparations (e.g., sustained-release microcapsules).

The agent for the prophylaxis or treatment of irritable bowel syndrome of the present invention can be administered safely to mammals (e.g., human, mouse, rat, rabbit, guinea pig, hamster, dog, cat, bovine, horse, pig, monkey etc.).

While the dose of the agent for the prophylaxis or treatment of irritable bowel syndrome of the present invention varies depending on the administration subject, administration route, target disease and the like, for example, when the agent is orally administered to an adult (about 60 kg), it is about 0.1 to 100 mg, preferably about 1.0 to 50 mg, more preferably about 1.0 to 20 mg, based on the compound of the present invention, which is the active ingredient, per day. The dose may be given at once or in several portions. When the agent for the prophylaxis or treatment of irritable bowel syndrome of the present invention is parenterally (e.g., intravenous injection) administered to an adult (about 60 kg), the dose is about 0.01 to 30 mg, preferably about 0.1 to 20 mg, more preferably about 0.1 to 10 mg, based on the compound of the present invention, which is the active ingredient, per day. The dose may be given at once or in several portions.

The agent for the prophylaxis or treatment of irritable bowel syndrome of the present invention can be used in combination with medicaments such as therapeutic agents for diabetes, therapeutic agents for diabetic complications, therapeutic agents for hyperlipidemia, antihypertensive agents, antiobestic agents, diuretics, chemotherapeutic agents, immunotherapeutic agents, antiinflammatory drugs, antithrombotic agents, therapeutic agents for osteoporosis, vitamins, antidementia agents, therapeutic agents for incontinentia or pollakiuria, therapeutic agents for dysuria, medicaments confirmed to show a cachexia-improving effect in animal models or clinical use and the like.

As the aforementioned therapeutic agents for diabetes, insulin preparations (e.g., animal insulin preparations extracted from the pancreas of bovine and pig; human insulin preparations genetically synthesized using *Escherichia coli,* yeast; zinc insulin; protamine zinc insulin; fragment or derivative of insulin (e.g., INS-1 etc.), oral insulin preparation and the like), insulin sensitizers (e.g., Pioglitazone or a salt thereof (preferably hydrochloride), Rosiglitazone or a salt thereof (preferably maleate), troglitazone, Reglixane (JTT-501), Netoglitazone (MCC-555), GI-262570, FK-614, CS-011, compound described in WO99/58510 (e.g., (E)-4-[4-(5-methyl-2-phenyl-4-oxazolylmethoxy)benzyloxyimino]-4-phenylbutyric acid), compound described in WO01/38325, Tesaglitazar (AZ-242), BM-13-1258, LM-4156, MBX-102, LY-519818, MX-6054, LY-510929, Balaglitazone (NN-2344), T-131 or a salt thereof, THR-0921), α-glucosidase inhibitors (e.g., voglibose, acarbose, miglitol, emiglitate etc.), biguanides (e.g., phenformin, metformin, buformin or a salt thereof (e.g., hydrochlide, fumarate, succinate) etc.), insulin secretagogues [sulfonylurea (e.g., tolbutamide, glibenclamide, gliclazide, chlorpropamide, tolazamide, acetohexamide, glyclopyramide, glimepiride etc.), repaglinide, senaglinide, mitiglinide or calcium salt hydrate thereof, nateglinide, etc.], GLP-1 receptor agonists [e.g., GLP-1, GLP-1MR agent, NN-2211, AC-2993 (exendin-4), BIM-51077, Aib(8, 35)hGLP-1(7, 37)NH₂, CJC-1131 etc.], dipeptidyl peptidase IV inhibitor (e.g., NVP-DPP-278, PT-100, P32/98, P93/01, NVP-DPP-728, LAF237, TS-021 etc.), β3 agonist (e.g., CL-316243, SR-58611-A, UL-TG-307, AJ-9677, AZ40140 etc.), amylin agonists (e.g., pramlintide etc.), phosphotyrosine phosphatase inhibitors (e.g., sodium vanadate etc.), gluconeogenesis inhibitors (e.g., glycogen phosphorylase inhibitor, glucose-6-phosphatase inhibitor, glucagon antagonist etc.), SGLT (sodium-glucose cotransporter) inhibitors (e.g., T-1095 etc.), 11β-hydroxysteroid dehydrogenase inhibitors (e.g., BVT-3498 etc.), adiponectin or agonist thereof, IKK inhibitors (e.g., AS-2868 etc.), leptin resistance improving drugs, somatostatin receptor agonists (compound described in WO01/25228, WO03/42204, WO98/44921, WO98/45285, WO99/22735 etc.), glucokinase activators (e.g., Ro-28-1675) and the like can be mentioned.

Examples of the therapeutic agent for diabetic complications include aldose reductase inhibitors (e.g., Tolrestat, Epalrestat, Zenarestat, Zopolrestat, Fidarestat (SNK-860), AS-3201, Minalrestat (ARI-509), CT-112 etc.), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole etc.) and the like), protein kinase C (PKC) inhibitors (e.g., LY-333531 etc.), AGE inhibitors (e.g., ALT-945, pimagedine, pyratoxanthine, N-phenacylthiazolium bromide (ALT-766), EXO-226, ALT-711, Pyridorin, Pyridoxamine etc.), active oxygen scavengers (e.g., thioctic acid etc.), cerebral vasodilators (e.g., tiapride etc.), somatostatin receptor agonists (BIM23190), apoptosis signal regulating kinase-1 (ASK-1) inhibitors and the like.

Examples of the therapeutic agent of hyperlipidemia include HMG-CoA reductase inhibitors (e.g., pravastatin, simvastatin, lovastatin, atorvastatin, fluvastatin, pitavastatin, rosuvastatin and salts thereof (e.g., sodium salt etc.) etc.), squalene synthase inhibitors (e.g., compound described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid etc.), fibrate compounds (e.g., bezafibrate, clofibrate, simfibrate, clinofibrate etc.), antioxidant (e.g., lipoic acid, probucol) and the like.

Examples of the antihypertensive agent include angiotensin converting enzyme inhibitors (e.g., captopril, enalapril, delapril etc.), angiotensin II antagonists (e.g., losartan, candesartan cilexetil, eprosartan, valsartan, telmisartan, irbesartan, olmesartan medoxomil, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid etc.), calcium antagonist (e.g., manidipine, nifedipine, amlodipine, efonidipine, nicardipine etc.), Clonidine and the like.

Examples of the antiobestic agent include antiobestic agents acting on the central nervous system (e.g., Dexfenfluramine, fenfluramine, phentermine, Sibutramine, amfepramone, dexamphetamine, Mazindol, phenylpropanolamine, clobenzorex; MCH receptor antagonists (e.g., SB-568849; SNAP-7941; compound described in WO01/82925 and WO01/87834 etc.); neuropeptide Y antagonists (e.g., CP-422935 etc.); cannabinoid receptor antagonists (e.g., SR-141716, SR-147778 etc.); ghrelin antagonist; 11β-hydroxysteroid dehydrogenase inhibitors (e.g., BVT-3498 etc.) and the like), pancreatic lipase inhibitors (e.g., orlistat, ATL-962 etc.), β3 agonists (e.g., CL-316243, SR-58611-A, UL-TG-307, AJ-9677, AZ40140 etc.), peptidic anorexiants (e.g., leptin, CNTF (Ciliary Neurotropic Factor) etc.), cholecystokinin agonists (e.g., lintitript, FPL-15849 etc.), anorexigenic agent (e.g., P-57 etc.) and the like.

Examples of the diuretic include xanthine derivatives (e.g., sodium salicylate and theobromine, calcium salicylate and theobromine etc.), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide etc.), antialdosterone preparations (e.g., spironolactone, triamterene etc.), carbonate dehydratase inhibitors (e.g., acetazolamide etc.), chlorobenzenesulfonamide preparations (e.g., chlortalidone, mefruside, indapamide etc.), azosemide, isosorbide, etacrynic acid, piretanide, bumetanide, furosemide and the like.

Examples of the chemotherapeutic agent include alkylation agents (e.g., cyclophosphamide, ifosfamide etc.), metabolic antagonists (e.g., methotrexate, 5-fluorouracil and derivatives thereof etc.), anti-cancer antibiotics (e.g., mitomycin, adriamycin etc.), plant-derived anti-cancer agents (e.g., vincristin, vindesine, taxol etc.), cisplatin, carboplatin, etoposide and the like. Among them, furtulon and neofurtulon, which are 5-fluorouracil derivatives, and the like are preferable.
Examples of the immunotherapeutic agent include microorganism or bacterial components (e.g., muramyl dipeptide derivative, picibanil etc.), polysaccharides having immunity potentiating activity (e.g., lentinan, sizofiran, krestin etc.), cytokines obtained by genetic engineering techniques (e.g., interferon, interleukin (IL) etc.), colony stimulating factors (e.g., granulocyte colony stimulating factor, erythropoietin etc.) and the like, with preference given to interleukins such as IL-1, IL-2 and IL-12.

Examples of the anti-inflammatory drug include steroid (e.g., dexamethasone etc.), sodium hyaluronate, cyclooxygenase inhibitors (e.g., aspirin, acetaminophen, indomethacin, ketoprofen, loxoprofen, meloxicam, ampiroxicam, celecoxib, rofecoxib etc.).

Examples of the antithrombotic agent include heparin (e.g., heparin sodium, heparin calcium, dalteparin sodium etc.), warfarin (e.g., warfarin potassium etc.), anti-thrombin drugs (e.g., aragatroban etc.), thrombolytic agents (e.g., urokinase, tisokinase, alteplase, nateplase, monteplase, pamiteplase etc.), platelet aggregation inhibitors (e.g., ticlopidine hydrochloride, cilostazol, ethyl icosapentate, beraprost sodium, sarpogrelate hydrochloride etc.) and the like.

Examples of the therapeutic agent of osteoporosis include alfacalcidol, calcitriol, elcatonin, calcitonin salmon, estriol, ipriflavone, pamidronate disodium, alendronate sodium hydrate, incadronate disodium and the like.

As the vitamin, for example, vitamin B1, vitamin B12 and the like can be mentioned.

Examples of the antidementia agent include tacrine, donepezil, rivastigmine, galanthamine and the like.
Examples of the therapeutic agent for incontinentia or pollakiuria include flavoxate hydrochloride, oxybutynin hydrochloride, propiverine hydrochloride and the like.

Examples of the therapeutic agent for dysuria include acetylcholine esterase inhibitors (e.g., distigmine) and the like.

Examples of the medicaments confirmed to show a cachexia-improving effect in animal models or clinical use include progesterone derivatives (e.g., megestrol acetate) (Journal of Clinical Oncology, vol. 12, pages 213-225, 1994), metoclopramide pharmaceuticals (ibid), tetrahydrocannabinol pharmaceuticals (ibid), fat metabolism ameliorating agent (e.g., eicosapentaenoic acid and the like) (British Journal of Cancer, vol. 68, pages 314-318, 1993), growth hormone, IGF-1, antibodies to TNF-α, LIF, IL-6 and oncostatin M, which are cachexia inducers, and the like.

Moreover, glycosylation inhibitors (e.g., ALT-711), nerve regeneration stimulating agents (e.g., Y-128, VX853, prosaptide), drugs acting on the central nervous system (e.g., desipramine, amitriptyine, imipramine), antidepressants (e.g., lamotrigine), antiarrhythmics (e.g., mexiletine), acetylcholine receptor ligands (e.g., ABT-594), endothelin receptor antagonists (e.g., morphine), monoamine uptake inhibitors (e.g., tramadol), indoleamine uptake inhibitors (e.g., fluoxetine, paroxetine), GABA receptor agonists (e.g., gabapentin), GABA uptake inhibitors (e.g., tiagabine) α2 receptor agonists (e.g., clonidine), topical analgesics (e.g., capsaicin), antianxiety drugs (e.g., benzothiazepine), phosphodiesterase inhibitors (e.g., sildenafil), dopamine receptor agonists (e.g., apomorphine), anticholinergic agents, protein kinase C inhibitors (e.g., LY-333531), α1 receptor blockers (e.g., tamsulosin), muscle relaxants (e.g., baclofen etc.), potassium channel openers (e.g., nicorandil), calcium channel blockers (e.g., nifedipine), prophylactic or therapeutic drugs for Alzheimer's disease (e.g., donepezil, rivastigmine, galanthamine), therapeutic drugs for Parkinson's disease (e.g., L-DOPA), NK2 receptor antagonists, therapeutic drugs for HIV infection (e.g., saquinavir, zidovudine, lamivudine, nevirapine), therapeutic drugs for chronic obstructive pulmonary diseases (e.g., salmeterol, thiotropium bromide, cilomilast), C1C-2 channel openers (intestinal fluid secretion promoting agent) (e.g., lubiprostone) and the like (hereinafter to be also referred to as a concomitant drug) can also be used in combination with the agent for the prophylaxis or treatment of irritable bowel syndrome of the present invention.

The above-mentioned concomitant drug may be a combination of two or more kinds thereof at an appropriate ratio.

By combining the compound of the present invention and the concomitant drug, a superior effect such as,
(1) the dose of the agent for the prophylaxis or treatment of irritable bowel syndrome of the present invention and/or the concomitant drug can be reduced as compared to single administration of the agent for the prophylaxis or treatment of irritable bowel syndrome of the present invention or the concomitant drug,
(2) a synergistic effect can be afforded by a combined use of the agent for the prophylaxis or treatment of irritable bowel syndrome of the present invention and the concomitant drug, and the like, can be achieved.
For the use of the agent for the prophylaxis or treatment of irritable bowel syndrome of the present invention and the concomitant drug in combination, the administration time of the agent for the prophylaxis or treatment of irritable bowel syndrome of the present invention and the concomitant drug is not restricted, and the agent for the prophylaxis or treatment of irritable bowel syndrome of the present invention and the concomitant drug can be administered to an administration subject simultaneously, or may be administered at staggered times. The dosage of the concomitant drug may be determined according to the dose clinically used, and can be appropriately selected depending on an administration subject, administration route, disease, combination and the like.

The administration mode of the agent for the prophylaxis or treatment of irritable bowel syndrome of the present invention and the concomitant drug is not particularly restricted, as long as the agent for the prophylaxis or treatment of irritable bowel syndrome of the present invention and the concomitant drug are combined in administration. Examples of such administration mode include the following methods: (1) The agent for the prophylaxis or treatment of irritable bowel syndrome of the present invention and the concomitant drug are simultaneously formulated to give a single preparation which is administered. (2) The agent for the prophylaxis or treatment of irritable bowel syndrome of the present invention and the concomitant drug are separately formulated to give two kinds of preparations which are administered simultaneously by the same administration route. (3) The agent for the prophylaxis or treatment of irritable bowel syndrome of the present invention and the concomitant drug are separately formulated to give two kinds of preparations which are administered by the same administration route at staggered times. (4) The agent for the prophylaxis or treatment of irritable bowel syndrome of the present invention and the concomitant drug are separately formulated to give two kinds of preparations which are administered simultaneously by the different administration routes. (5) The agent for the prophylaxis or treatment of irritable bowel syndrome of the present invention and the concomitant drug are separately formulated to give two kinds of preparations which are administered by the different administration routes at staggered times (e.g., the agent for the prophylaxis or treatment of irritable bowel syndrome of the present invention and the concomitant drug are administered in this order, or in the reverse order), and the like. Examples

The present invention is explained in more detail in the following by referring to Examples. However, the present invention is not limited by the Examples and may be changed as long as the scope of the present invention is not deviated.

### Explanation of stress-induced bowel movement model

Various clinical reports have clarified that stress is deeply involved in the pathology of IBS (1, 2). In addition, the intestine motility of IBS patients has been reported to be enhanced when an emotional stress is loaded (3, 4), and it is considered that such enhanced motility caused by the stress is involved in the abnormal bowel movement of IBS (5). As a therapeutic drug for IBS, Alosetron which is a 5-HT3 receptor antagonist is placed in the market, and 5-HT3 antagonists including Alosetron have been shown to suppress stress-induced bowel movement in various animals (6-9). For this experiment, therefore, a restraint stress-induced bowel movement model mouse was used.

1. Whitehead WE et al. Psychologic considerations in the irritable bowel syndrome. Gastroenterol Clin North Ame. (1991) 20:249-267
2. Whitehead WE et al. Is rectal pain sensitivity a biological marker for irritable bowel syndrome: Psychological influences on pain perception. Gastroenterology. (1998) 115:1263-71
3. Fukudo S et al. Colonic motility, autonomic function, and gastrointestinal hormones under psychological stress on irritable bowel syndrome. Tohoku Exp Med. (1987) 15:373-85
4. Fukudo S et al. Brain-gut response to stress and cholinergic stimulation in irritable bowel syndrome. A preliminary study. J Clin Gastroenterol. (1993) 17: 133-41
5. Posserud I et al. Functional findings in irritable bowel syndrome. World J Gastroenterol. (2006) 12:2830-8
6. Okano S et al. Role of tachykinin NK1 receptors on novelty stress-induced defecation in Mongolian gerbils. Gastroenterology. (2006) 130(4, Suppl. 2): Abst S1947
7. Okano S et al. Novelty stress increases fecal pellet output in mongolian gerbils: Effects of several drugs. J Pharmacol Sci. (2005) 98: 411-8
8. Ohta M et al. Novel 5-hydroxytryptamine (5-HT3) receptor antagonists. III. Pharmacological evaluations and molecular modeling studies of optically active 4,5,6,7-tetrahydro-1H-benzimidazole derivatives. Chem Pharm Bull (Tokyo) (1996) 44:1707-16
9. Tamaoki S et al. Pharmacological properties of 3-amino-5,6,7,8-tetrahydro-2-[4-[4-(quinolin-2-yl)piperazin-1-yl]butyl]quinazolin-4(3H)-one (TZB-30878), a novel therapeutic agent for diarrhea-predominant irritable bowel syndrome (IBS) and its effects on an experimental IBS model. J Pharmacol Exp Ther (2007) 322:1315-23

### Example 1

### Consideration of reactivity of TGR5 agonist against mouse TGR5

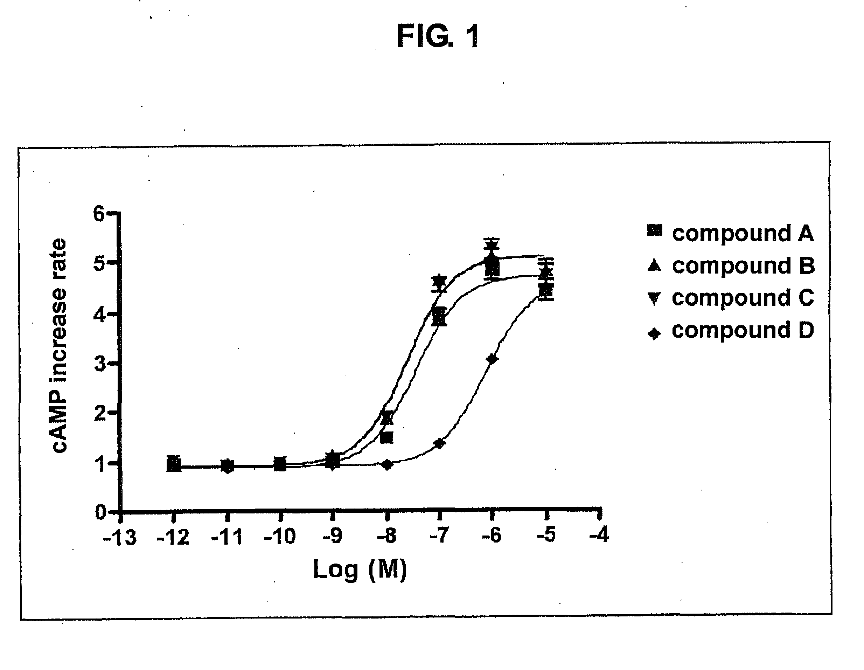

DNA fragment encoding mouse TGR5 was cloned according to the method described in W02004/043467, and the obtained DNA fragment was introduced into pAKKO-111H (plasmid vector same as pAKKO-111H described in Biochem Biophys Acta, Hinuma S et al., 1219, 251-259, 1994) to construct an expression vector. CHO cell line expressing mouse TGR5 (CHO-mTGR5), which was prepared by a method known per se and using the mouse TGR5 expression vector, was suspended in an assay medium (Hanks' Balanced Salt Solution (Invitrogen) added with 0.2 mM isobutylmethylxanthine and 0.1% bovine albumin) at 1×10⁷ cells/ml and stood at room temperature for 30 min. Then, a compound diluted with the assay medium to each concentration was added, and the mixture was stood still at room temperature for 30 min, and the cAMP amount was measured according to the protocol of ALPHA SCREEN cAMP ASSAY KIT (Perkin Elmer). As a result, cAMP production from CHO-mTGR5 was induced by the stimulation with each compound (Fig. 1). The EC50 value was calculated by using GraphPad PRISM (GraphPad software) from the amount of cAMP produced by each compound.

As the compounds to be used for the Example, the following were used.

### (1) Compound A (4-[3,5-bis(trifluoromethyl)benzyl]-6-(2-fluorophenyl)-4,5-dihydropyrido[3,2-f][1,4]oxazepin-3(2H)-one)

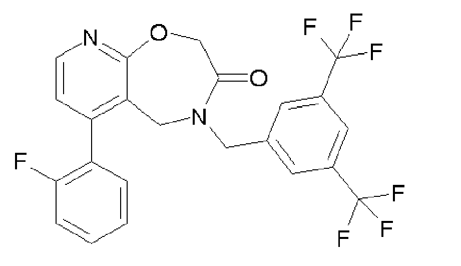

Compound A is disclosed in JP-A-2006-056881 (Example 16), and can be produced according to the method described therein.

### (2) Compound B (4-{1-[3,5-bis(trifluoromethyl)phenyl]ethyl}-6-(2-fluorophenyl)-4,5-dihydropyrido[3,2-f][1,4]oxazepin-3(2H)-one)

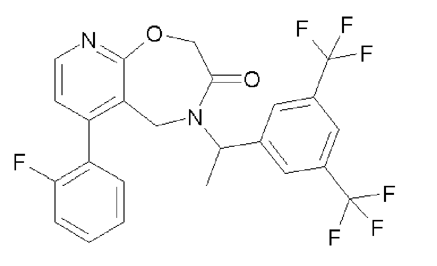

Compound B is disclosed in JP-A-2006-056881 (Example 18), and can be produced according to the method described therein.

### (3) Compound C (7-fluoro-4-[(isoquinolin-5-yloxy)acetyl]-1,3,3-trimethyl-3,4-dihydroquinoxalin-2(1H)-one)

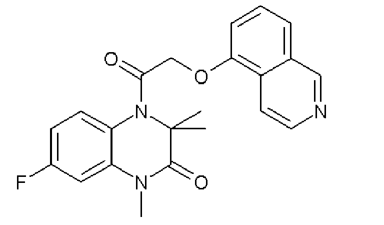

Compound C is disclosed in JP-A-2006-63064 (Example 173), and can be produced according to the method described therein.

### (4) Compound D (4-{1-[3,5-bis(trifluoromethyl)phenyl]ethyl}-6-phenyl-2,3,4,5-tetrahydropyrido[3,2-f][1,4]oxazepine hydrochloride)

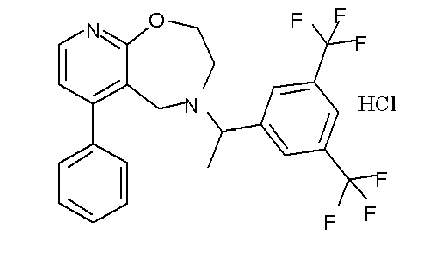

Compound D is disclosed in W02004-067008 (Example 33C), and can be produced according to the method described therein.

### (5) Compound E (4-[3,5-bis(trifluoromethyl)benzyl]-6-(2-hydroxyphenyl)-4,5-dihydropyrido[3,2-f][1,4]oxazepin-3(2H)-one)

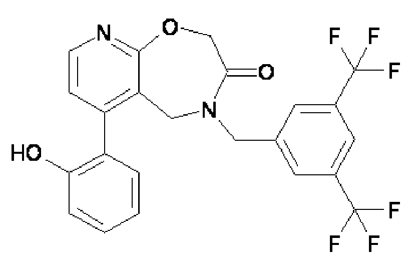

Compound E is disclosed in JP-A-2006-056881 (Example 50), and can be produced according to the method described therein.

As a result, the EC50 values of compound A, compound B, compound C, compound D and compound E were 36 nM, 25 nM, 25 nM, 730 nM, and N.D. (not determined), respectively.

### Example 2

### Consideration of reactivity of TGR5 agonist against rat TGR5

CHO cell line expressing rat TGR5 (CHO-rTGR5) was prepared in the same manner as in Example 1 and the EC50 value was calculated in the same manner as in Example 1.
The compounds subjected to this Example were similar to those in Example 1.
As a result, the EC50 values of compound A, compound B, compound C, compound D and compound E were 4.7 nM, 2.4 nM, 9.9 nM, 120 nM and 320 nM, respectively.

### Example 3

### Consideration of reactivity of TGR5 agonist against human TGR5

CHO cell line expressing human TGR5 (CHO-hTGR5) was prepared in the same manner as in Example 1 and the EC50 value was calculated in the same manner as in Example 1.
The compounds subjected to this Example were similar to those in Example 1. As a result, the EC50 values of compound A, compound B, compound C, compound D and compound E were 0.23 nM, 0.62 nM, 2.1 nM, 1.6 nM, and 240 nM, respectively. The results of Examples 1 to 3 are shown in the following Table.

**[Table 8]**

| compound | structural formula | EC50 (M) | | |
|---|---|---|---|---|
| | | Human | Rat | Mouse |
| A | 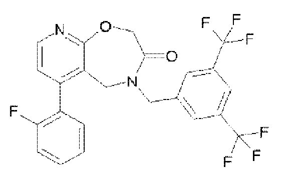 | 2.3E-10 | 4.7E-09 | 3.6E-08 |
| B | 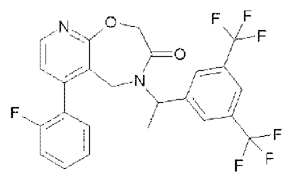 | 6.2E-10 | 2.4E-09 | 2.5E-08 |
| C | 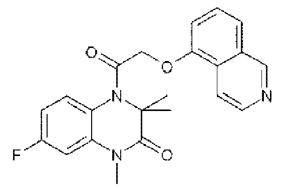 | 2.1E-09 | 9.9E-09 | 2.5E-08 |
| D | 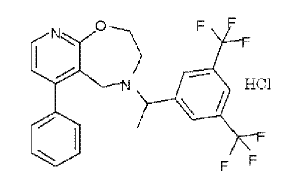 | 1.6E-09 | 1.2E-07 | 7.3E-07 |
| E | 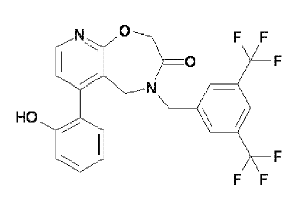 | 2.4E-07 | 3.2E-07 | N.D. |

| | | | | |
|---|---|---|---|---|
| N.D.: Not Determined | | | | |

### Example 4

### Effect of TGR5 agonist on mouse restraint stress-induced bowel movement

Male C57BL/6 mice were used under non-fasting conditions. On the day of the test, the body weight was measured, and the mice were individually bred for 2 hr. Thereafter, the mice were placed in a mouse restraint stress cage, and the number of excreted feces in 2 hr was counted. The normal group was individually bred for 2 hr, and subsequently was bred under the same conditions, the number of excreted feces in 2 hr was counted. Each drug (same as in Example 1) was suspended in 0.5% methylcellulose, and intraperitoneally administered at a dose of 10 ml/kg 10 min before stress loading. As a result, compound A, compound B, compound C and compound D all suppressed a restraint stress-induced bowel movement in a dose-dependent manner (Fig. 2). A statistically significant suppressive action on the restraint stress-induced bowel movement was shown by compound A (Fig. 2-1) and compound B (Fig. 2-2) at a dose of 30 mg/kg, by compound C (Fig. 2-3) at doses of 10 and 30 mg/kg, and by compound D (Fig. 2-4) at doses of 3, 10 and 30 mg/kg.

### Example 5

### Effect of TGR5 agonist on rat exo-vivo ileum peristalsis

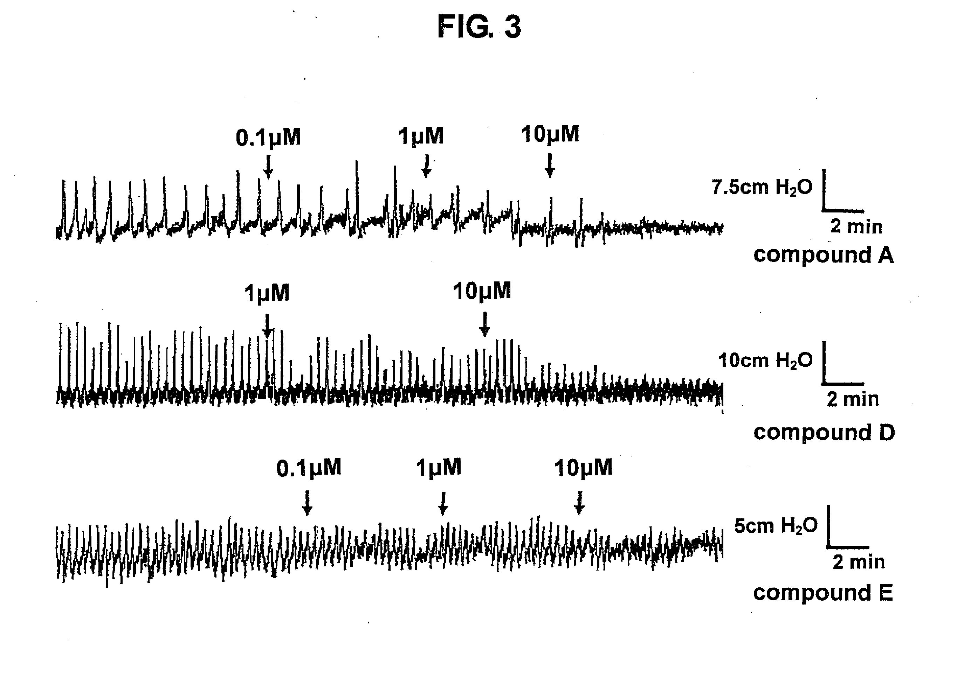

Male SD rats were used under non-fasting conditions. On the day of the test, 4-5 cm of the ileum was isolated, and the oral side end and aboral side end were subjected to cannulation. 95% oxygen/5% carbon dioxide was passed therethrough and placed horizontally in a bath filled with a Krebs solution warmed to 37°C. A tube outflux on the aboral side was set at about 4 cm higher than the bath, 95% oxygen/5% carbon dioxide was passed from the tube on the oral side and perfused with a Krebs solution warmed to 37°C at a flow rate of 10 cc/hr to induce peristalsis motility. Changes of the intraluminal pressure of the intestine due to the peristalsis motility of the ileum were recorded by a transducer from the tube on the oral side. A compound at various concentrations was added to the Krebs solution surrounding the ileum. When a compound at various concentrations was added, the Krebs solution was exchanged immediately before the addition. As a result, compound A, compound D and compound E all suppressed the peristalsis motility of the ileum in a dose-dependent manner (Fig. 3).

### Formulation Example 1

| | |
|---|---|
| 1) (7-fluoro-4-[(isoquinolin-5-yloxy)acetyl]-1,3,3-trimethyl-3,4-dihydroquinoxalin-2(1H)-one) | 30 mg |
| 2) finely divided powder cellulose | 10 mg |
| 3) lactose | 19 mg |
| 4) magnesium stearate | 1 mg |
| | Total 60 mg |

The above-mentioned 1), 2), 3) and 4) are mixed and filled in a gelatin capsule.

### Formulation Example 2

| | |
|---|---|
| 1) (7-fluoro-4-[(isoquinolin-5-yloxy)acetyl]-1,3,3-trimethyl-3,4-dihydroquinoxalin-2(1H)-one) | 30 g |
| 2) lactose | 50 g |
| 3) cornstarch | 15 g |
| 4) calcium carboxymethylcellulose | 44 g |
| 5) magnesium stearate | 1 g |
| 1000 tablets | Total 140 g |

The total amount of the above-mentioned 1), 2) and 3) and 30 g of 4) are kneaded with water, vacuum dried and sieved. The sieved powder is mixed with 14 g of 4) and 1 g of 5), and the mixture is punched by a tableting machine. In this way, 1000 tablets containing 30 mg of (7-fluoro-4-[(isoquinolin-5-yloxy)acetyl]-1,3,3-trimethyl-3,4-dihydroquinoxalin-2(1H)-one) per tablet are obtained.

### Formulation Example 3

| | |
|---|---|
| 1) 4-[3,5-bis(trifluoromethyl)benzyl]-6-(2-fluorophenyl)-4,5-dihydropyrido[3,2-f][1,4]oxazepin-3(2H)-one | 30 mg |
| 2) finely divided powder cellulose | 10 mg |
| 3) lactose | 19 mg |
| 4) magnesium stearate | 1 mg |
| | Total 60 mg |

The above-mentioned 1), 2), 3) and 4) are mixed and filled in a gelatin capsule. In this way, a capsule containing 30 mg of 4-[3,5-bis(trifluoromethyl)benzyl]-6-(2-fluorophenyl)-4,5-dihydropyrido[3,2-f][1,4]oxazepin-3(2H)-one is obtained.

### Formulation Example 4

| | |
|---|---|
| 1) 4-[3,5-bis(trifluoromethyl)benzyl]-6-(2-fluorophenyl)-4,5-dihydropyrido[3,2-f][1,4]oxazepin-3(2H)-one | 30 g |
| 2) lactose | 50 g |
| 3) cornstarch | 15 g |
| 4) calcium carboxymethylcellulose | 44 g |
| 5) magnesium stearate | 1 g |
| 1000 tablets | Total 140 g |

The total amount of the above-mentioned 1), 2) and 3) and 30 g of 4) are kneaded with water, vacuum dried and sieved. The obtained sieved powder is mixed with 14 g of 4) and 1 g of 5), and the mixture is punched by a tableting machine. In this way, 1000 tablets containing 30 mg of 4-[3,5-bis(trifluoromethyl)benzyl]-6-(2-fluorophenyl)-4,5-dihydropyrido[3,2-f][1,4]oxazepin-3(2H)-one per tablet are obtained.

### Formulation Example 5

| | |
|---|---|
| 1) (4-{1-[3,5-bis(trifluoromethyl)phenyl]ethyl}-6-phenyl-2,3,4,5-tetrahydropyrido[3,2-f][1,4]oxazepine hydrochloride) | 30 mg |
| 2) finely divided powder cellulose | 10 mg |
| 3) lactose | 19 mg |
| 4) magnesium stearate | 1 mg |
| | Total 60 mg |

The above-mentioned 1), 2), 3) and 4) are mixed and filled in a gelatin capsule. In this way, a capsule containing 30 mg of (4-{1-[3,5-bis(trifluoromethyl)phenyl]ethyl}-6-phenyl-2,3,4,5-tetrahydropyrido[3,2-f][1,4]oxazepine hydrochloride) is obtained.

### Formulation Example 6

| | |
|---|---|
| 1) (4-{1-[3,5-bis(trifluoromethyl)phenyl]ethyl}-6-phenyl-2,3,4,5-tetrahydropyrido[3,2-f][1,4]oxazepine hydrochloride) | 30 g |
| 2) lactose | 50 g |
| 3) cornstarch | 15 g |
| 4) calcium carboxymethylcellulose | 44 g |
| 5) magnesium stearate | 1 g |
| 1000 tablets | Total 140 g |

The total amount of the above-mentioned 1), 2) and 3) and 30 g of 4) are kneaded with water, vacuum dried and sieved. The obtained sieved powder is mixed with 14 g of 4) and 1 g of 5),
and the mixture is punched by a tableting machine. In this way, 1000 tablets containing 30 mg of (4-{1-[3,5-bis(trifluoromethyl)phenyl]ethyl}-6-phenyl-2,3,4,5-tetrahydropyrido[3,2-f][1,4]oxazepine hydrochloride) per tablet are obtained.

### Industrial Applicability

The present invention can provide a medicament having an action to suppress a stress-induced bowel movement, and capable of improving abdominal symptoms of irritable bowel syndrome(IBS), particularly, diarrhea type irritable bowel syndrome (IBS), in which stress is considered to be deeply involved in the pathology.
This application is based on patent application No. 2008-204843 filed in Japan, the contents of which are encompassed in full herein.

## Claims

1. An agent for the prophylaxis or treatment of irritable bowel syndrome, comprising a compound having a TGR5 receptor agonistic activity.

2. The agent according to claim 1, wherein the compound having a TGR5 receptor agonistic activity is a fused ring compound represented by the formula 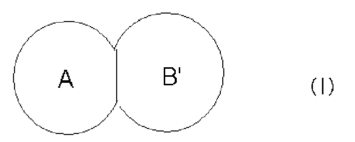 wherein ring A is an optionally substituted aromatic ring, and ring B' is a 5- to 9-membered ring having one or more substituents, or a salt thereof.

3. An agent for the prophylaxis or treatment of irritable bowel syndrome, comprising a fused ring compound represented by the formula 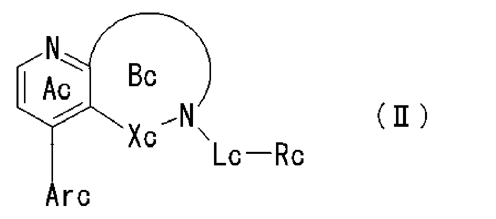 wherein ring Ac is a pyridine ring optionally further having substituent(s) other than -Arc,
ring Bc is a nitrogen-containing 6- to 9-membered ring optionally further having substituent(s) other than -Lc-Rc,
Xc is an optionally substituted methylene group,
Arc is an optionally substituted aromatic group,
Rc is an optionally substituted cyclic group,
Lc is an optionally substituted C₁₋₃ alkylene group, -CONH-, -SO₂NH- or-SO₂-, and
Xc group is not a methylene group substituted by an oxo group,
or a salt thereof.

4. The agent according to claim 3, wherein the fused ring compound is represented by the formula 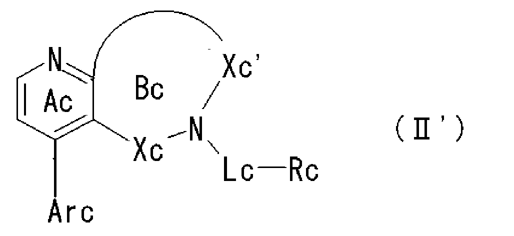 wherein Xc' is an optionally substituted methylene group, and other symbols are each as defined in claim 3.

5. The agent according to claim 3, wherein the fused ring compound is represented by the formula 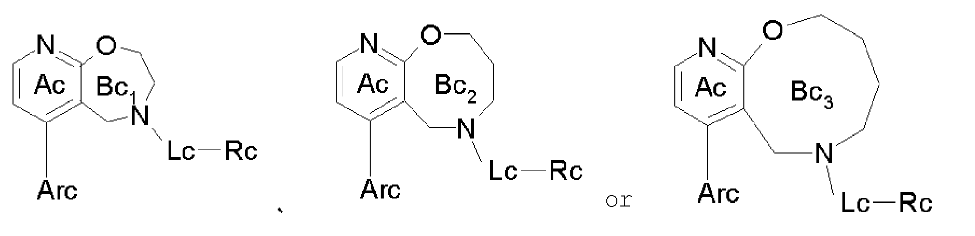 wherein ring Bc₁, ring Bc₂ and ring Bc₃ each optionally further have substituent(s) other than Lc-Rc, and other symbols are each as defined in claims 3.

6. The agent according to claim 3 or 4, wherein Xc is a methylene group.

7. The agent according to any one of claims 3 to 5, wherein the cyclic group for Rc is a phenyl group.

8. The agent according to any one of claims 3 to 5, wherein Rc is a 3,5-bis(trifluoromethyl)phenyl group.

9. The agent according to any one of claims 3 to 5, wherein Lc is a C₁₋₃ alkylene group or -SO₂-, which is optionally substituted by a C₁₋₃ alkyl group or an oxo group.

10. The agent according to any one of claims 3 to 5, wherein Arc is an optionally substituted phenyl group.

11. The agent according to any one of claims 3 to 5, wherein Arc is a phenyl group optionally having substituent(s) at the ortho-position relative to the bond with ring Ac.

12. The agent according to claim 3, wherein the fused ring compound is represented by the formula 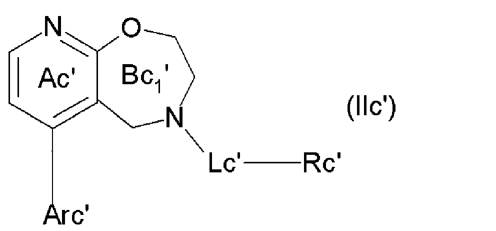 wherein ring Ac' is a pyridine ring optionally further having substituent(s) other than -Arc',
ring Bc₁' optionally further has substituent(s) other than -Lc'-Rc', Lc' is a C₁₋₃ alkylene group optionally substituted by a C₁₋₃ alkyl group or an oxo group,
Rc' is an optionally substituted phenyl group, and
Arc' is a phenyl group optionally having substituent(s) at the ortho-position relative to the bond with ring Ac'.

13. An agent for the prophylaxis or treatment of irritable bowel syndrome, comprising a fused ring compound represented by the formula 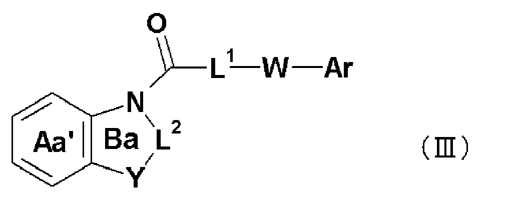 wherein ring Aa' is an optionally substituted benzene ring,
ring Ba is a 6- to 8-membered nonaromatic nitrogen-containing heterocycle,
W is -O- or -S(O)nₐ- (nₐ is an integer of 0 to 2),
Y is a bond, -O-, -NR- (R is a hydrogen atom or an optionally substituted C₁₋₆ alkyl group) or -S(O)n_{b}- (n_{b} is an integer of 0 to 2),
L¹ is (1) a chained C₁₋₅ alkylene group optionally substituted by an optionally halogenated C₁₋₆ alkyl group or (2) an optionally substituted chained C₂₋₅ alkenylene group,
L² is (1) a chained C₂₋₅ alkylene group optionally substituted by substituent(s) selected from a fluorine atom, an optionally halogenated C₁₋₆ alkyl group, an optionally substituted C₇₋₁₁ aralkyl group, an optionally halogenated C₁₋₆ alkoxy group, a hydroxy group and an oxo group or (2) an optionally substituted chained C₂₋₅ alkenylene group, and
Ar is an optionally substituted phenyl group or an optionally substituted bicyclic benzene fused ring group,
or a salt thereof.

14. The agent according to claim 13, wherein W is -O-.

15. The agent according to claim 13, wherein L¹ is a chained C₁₋₅ alkylene group.

16. The agent according to claim 13, wherein ring Ba is a 6-membered nonaromatic nitrogen-containing heterocycle.

17. The agent according to claim 13, wherein ring Aa' is a benzene ring optionally substituted by 1 to 3 substituents selected from a halogen atom, an optionally halogenated C₁₋₆ alkyl group, a carboxyl group and a C₁₋₆ alkoxy-carbonyl group.

18. The agent according to claim 13, wherein Ar is a phenyl group, an indanyl group, a tetrahydronaphthyl group or a 5-isoquinolinyl group each optionally substituted by 1 to 3 substituents selected from a halogen atom, an optionally halogenated C₁₋₆ alkyl group, a heterocyclic group, an optionally halogenated C₁₋₆ alkoxy group, a C₇₋₁₄ aralkyloxy group, an optionally halogenated C₁₋₆ alkylthio group, a hydroxy group, a mercapto group, a cyano group, a carboxyl group, a formyl group, an optionally halogenated C₁₋₆ alkyl-carbonyl group,
a C₆₋₁₄ aryl-carbonyl group, a heterocyclic group-carbonyl group, a C₁₋₆ alkoxy-carbonyl group, a mono- or di-C₁₋₆ alkylamino group, a formylamino group, an optionally halogenated C₁₋₆ alkyl-carbonylamino group, a carbamoyl group, a mono- or di-C₁₋₆ alkyl-carbamoyl group, a C₆₋₁₄ aryl group, an oxo group, a hydroxyimino group and a C₁₋₆ alkoxyimino group.

19. The agent according to claim 13, wherein ring Aa' is a benzene ring optionally substituted by a halogen atom,
ring Ba is a 6-membered nonaromatic nitrogen-containing heterocycle, W is -O-, Y is -NR- (R is a hydrogen atom or an optionally substituted C₁₋₆ alkyl group),
L¹ is a methylene group optionally substituted by an optionally halogenated C₁₋₆ alkyl group,
L² is a chained C₂₋₅ alkylene group optionally substituted by substituent(s) selected from a C₁₋₆ alkyl group and an oxo group, and
Ar is an optionally substituted bicyclic benzene fused ring group.

20. A method for the prophylaxis or treatment of irritable bowel syndrome, comprising administering an effective amount of a compound having a TGR5 receptor agonistic activity to a mammal.

21. A method for the prophylaxis or treatment of irritable bowel syndrome, comprising administering an effective amount of the fused ring compound according to claim 3 or 13 to a mammal.

22. Use of a compound having a TGR5 receptor agonistic activity for the production of an agent for the prophylaxis or treatment of irritable bowel syndrome.

23. Use of the fused ring compound according to claim 3 or 13 for the production of an agent for the prophylaxis or treatment of irritable bowel syndrome.
